# EUROPEAN PATENT APPLICATION

(11) **EP 4 458 290 A1**
(43) Date of publication of application: **06.11.2024**
(21) Application number: 22914821.8
(22) Date of filing: 27.12.2022
(51) Int. Cl.: A61B 17/34, A61M 25/00

(54) **CATHETER ASSEMBLY, TREATMENT SYSTEM AND METHOD FOR TRANSCATHETER TREATMENT SYSTEM**

(30) Priority: 30.12.2021 CN 202111660519; 31.12.2021 CN 202111679034; 31.12.2021 CN 202111679305
(71) Applicant: Hangzhou Deke Medtech Co., Ltd., Hangzhou, Zhejiang 310052 (CN)
(72) Inventor: SHAO, Nan, Hangzhou Zhejiang 310052 (CN); LI, Yang, Hangzhou Zhejiang 310052 (CN); ZHUANG, Zhenping, Hangzhou Zhejiang 310052 (CN); ZHU, Peng, Hangzhou Zhejiang 310052 (CN); HU, Fanbo, Hangzhou Zhejiang 310052 (CN); ZENG, Jianfeng, Hangzhou Zhejiang 310052 (CN); GAN, Yijie, Hangzhou Zhejiang 310052 (CN)
(74) Representative: Zaboliene, Reda
(86) International application number: PCT/CN2022/142503
(87) International publication number: WO 2023/125569

(57) **Abstract**

The present disclosure discloses a guide tube assembly for transcatheter treatment system, a treatment system and a method. The guide tube assembly for transcatheter treatment system has opposing distal and proximal ends. The distal section of the guide tube assembly has a curved extension including a first curved section, which is configured to have a bend angle by pre-shaping and/or bending, and a second curved section, which is located at a distal end of the first curved section and configured to have a bend angle by bending. The first curved section and the second curved section are located in different planes and provided by one or two tubes respectively which are movably inserted one in the other. The guide tube assembly is more suitable for interventional operations in complex physiological structures.

## Description

### TECHNICAL FIELD

The present disclosure relates to the technical field of medical devices, and in particular to a guide tube assembly for a transcatheter treatment system, a treatment system and a method.

### DESCRIPTION OF THE PRIOR ART

Heart failure (HF) refers to heart circulatory disturbances caused by congestion in the venous system and insufficient perfusion in the arterial system because venous returning blood cannot be fully discharged from the heart due to cardiac systolic dysfunction and diastolic dysfunction. A main manifestation of heart failure worsening and pathological remodeling is the changes in left ventricular morphology and structure, such as ventricular enlargement, hypertrophy, and spherical dilation, which further aggravate the patient's clinical symptoms.

With the development of medical technology, heart failure treatment methods such as injecting therapeutic agents for myocardial repair, such as alginate hydrogel, into the ventricular wall have emerged. Extensive clinical investigations have shown that the alginate hydrogel injected into the ventricular wall can change the geometric structure of the myocardium and the myocardial tension, improving cardiac function, and delay or reverse the progression of heart failure in patients with left ventricular enlargement, significantly improving cardiac function.

In the field of interventional therapy of structural heart diseases, conventional interventional paths include: puncture via the femoral vein, through the inferior vena cava, right atrium to the right ventricle, or through the inferior vena cava, right atrium, left atrium to the left ventricle; or puncture via the femoral artery, through the aortic arch to the left ventricle. For the interventional path through the aortic arch, due to the special arc-shaped stereoscopic morphology of the aortic arch, the guiding device is required to include multiple bendable sections, i.e., multiple sections hinged in sequence and cooperated with each other, which will increase the structural complexity and reduce the stability. Further, the hinge structure, when being displaced laterally, requires large avoidance spaces, and the avoidance spaces will be gradually reduced after the lateral displacement, increasing the bending difficulty. In addition, because the vascular tissue is flexible with strong compliance, the tissue may extend into the avoidance area, causing irreversible damage to the vascular tissue and increasing the operation risk of the treatment device.

### SUMMARY OF THE DISCLOSURE

The present disclosure provides a guide tube assembly for a transcatheter treatment system. The guide tube assembly has opposite distal and proximal ends. The distal section of the guide tube assembly has a curved extension including:
a first curved section which is configured to have a bend angle by pre-shaping and/or bending; and
a second curved section which is located at a distal end of the first curved section and configured to have a bend angle by bending;
wherein the first curved section and the second curved section are located in different planes and provided by one or two tubes respectively which are movably inserted one in the other.

Optionally, the first curved section is provided by a hollow flexible catheter, and the second curved section is provided by a bending sheath.

The flexible catheter includes a main section, a trans-aortic arch section and a distal section that are connected and communicated in sequence from a proximal end to a distal end, the distal section at least includes a transition segment and a straight segment, wherein the trans-aortic arch section and the transition segment constitute the first curved section.

The bending sheath is bendable in a single direction which is different from a bend direction of the distal section of the flexible catheter, and a bendable section of the bending sheath constitutes the second curved section.

Optionally, the trans-aortic arch section is pre-shaped into a plurality of arc segments distributed in different planes, and the transition segment of the distal section is arc-shaped and two ends of the transition segment are respectively tangent to the straight segment and the trans-aortic arch section.

Optionally, angles between a central axis of a projection of the straight segment on a first plane, a second plane, and a third plane, respectively, and a central axis of a projection of the main section are α, β, and γ respectively, wherein the first plane is a vertical plane, the second plane is perpendicular plane perpendicular to the vertical plane, and the third plane is a horizontal plane perpendicular to both the vertical plane and the perpendicular plane, where 35° ≤ α ≤ 85°, 25° ≤ β ≤ 80°, 20° ≤ γ ≤ 75°.

Optionally, the trans-aortic arch section has a curved structure simulating an aortic arch, and includes a first arc segment, a second arc segment, a third arc segment and a fourth arc segment connected in sequence and tangent to each other, and the first arc segment, the second arc segment, the third arc segment and the fourth arc segment are distributed in different planes, wherein the first arc segment is connected and communicated with the main section, and the fourth arc segment is connected and communicated with the distal section.

Optionally, the first arc segment has an angle of a1, and a radius of an arc bend of the first arc segment is R1; the second arc segment has an angle of a2, and a radius of an arc bend of the second arc segment is R2; the third arc segment has an angle of a3, and a radius of an arc bend of the third arc segment is R3; the fourth arc segment has an angle of a4, and a radius of an arc bend of the fourth arc segment is R4.

Among them, 4.5° ≤ a1 ≤ 30°, 90 mm ≤ R1 ≤ 450 mm; 25° ≤ a2 ≤ 80°, 10 mm < R2 ≤ 60 mm; 20° ≤ a3 ≤ 70°, 25 mm ≤ R3 ≤ 50 mm; 20° ≤ a4 ≤ 110 °, 20 mm ≤ R4 ≤ 50 mm.

Optionally, the transition segment has an angle of a5, and a radius of an arc bend of the transition segment is R5, wherein 15° ≤ a5 ≤ 70°, 25 mm ≤ R5 ≤ 45 mm.

Optionally, an angle between a central axis of a projection of the transition segment on the first plane and the central axis of the main section is δ, where the first plane is the vertical plane, 0 < δ ≤ 45°.

Optionally, the straight segment has a length of l, where 0 < I < 80 mm.

Optionally, the transition segment is configured to be bent by a pull assembly, and the pull assembly includes a pull wire, a guiding passageway and an anchor ring, wherein the guiding passageway is defined in a wall of the flexible catheter for the pull wire to pass through, the anchor ring is fixedly connected to the transition segment of the distal section, and the pull wire is connected to the anchor ring.

Optionally, the anchor ring is adjacent to a connection of the transition segment and the straight segment.

The anchor ring also serves as an imaging mark, and a distal end of the straight segment is also configured with an imaging mark.

Optionally, a start point of the guiding passageway at the distal section is A, point B is deflected by 90° relative to the point A in a circumferential direction of the flexible catheter, and a transitional guiding section is formed between point A and point B, wherein the transitional guiding section extends along a part or an entire of the trans-aortic arch section, or a part or an entire the main section.

Optionally, the transitional guiding section has a length of L, where 0 mm < L ≤ 250 mm.

The present disclosure further provides a guide tube assembly for a transcatheter treatment system. The guide tube assembly has opposite distal and proximal ends. The distal section of the guide tube assembly includes:
a first curved section, which is configured to have a bend angle by bending;
an extension section, which has an adjustable length; and
a second curved section, which is configured to have a bend angle by bending;
wherein the first curved section, the extension section and the second curved section are arranged in sequence from the proximal end to the distal end.

Optionally, the extension section extends in a straight line.

Optionally, the first curved section and the second curved section are located in different planes.

Optionally, the first curved section itself is a non-planar section.

Optionally, the length of the extension section is in a range of 0 to 120 mm.

Optionally, the first curved section and the second curved section are respectively provided by different tubes.

The first curved section is provided by a first tube.

The second curved section is provided by a second tube slidably inserted in the first tube.

The extension section includes a straight segment of a most distal section of the first tube, and a straightly extending segment of a section of the second tube which is extendable out of the first tube, which is adjacent to the straight segment.

Optionally, the straight segment of the most distal section of the first tube has a length in a range of 0 to 80 mm.

Optionally, the straightly extending segment of the section of the second tube which is extendable out of the first tube, which is adjacent to the straight segment, has a length in a range of 0 to 80 mm.

Optionally, the first tube and the second tube have different bend directions.

Optionally, the first tube and the second tube are rotatably matched and at least rotatable to have different bend directions.

Optionally, the first tube and the second tube have perpendicular bend directions.

Optionally, the first tube and the second tube each have a force application point for bending and pulling, and the force application points of the first tube and the second tube are offset from each other in a circumferential direction, thereby obtaining different bend directions.

Optionally, the second curved section has a length in a range of 20 to 80 mm, and the first curved section has a length in a range of 160 to 300 mm.

Optionally, the first curved section and the second curved section are respectively configured to be bent entirely or partially.

Optionally, bent portions of the first curved section and the second curved section are respectively adjacent to distal ends of corresponding tubes.

Optionally, the first curved section is pre-shaped and has a bendable transition segment adjacent to a distal section of the first curved section.

Optionally, a force application point for bending of the guide tube assembly is configured with an imaging mark.

Optionally, distal ends of the first curved section and the second curved section respectively serve as force application points for bending.

Optionally, the guide tube assembly includes a first tube and a second tube slidably inserted in the first tube, wherein the first curved section is provided by the first tube, the second curved section is provided by the second tube, distal sections of the tubes are each configured with at least two imaging marks, and the imaging marks on a same tube are axially spaced from each other.

Optional, each tube is configured with two imaging marks, and a distance between the two imaging marks is in a range of 20 to 120 mm.

Optionally, the first tube is configured with a first imaging mark and a second imaging mark, and the second imaging mark is located at a distal side of the first imaging mark.

The first imaging mark is located at a force application point for bending of the first tube, corresponding to a connection of the first curved section and the extension section; and the second imaging mark is located at a most distal end of the first tube.

The second tube is configured with a third imaging mark and a fourth imaging mark, and the fourth imaging mark is located at a distal side of the third imaging mark.

The third imaging mark and the fourth imaging mark correspond to two ends of the second curved section, and the fourth imaging mark is located at a force application point for bending of the second tube.

Optionally, a distance between a distal end of the second curved section and a distal end of the second tube is in a range of 0 to 20 mm.

Optionally, when the second tube extends out of the distal end of the first tube, a distance between the first imaging mark and the fourth imaging mark is adjustable, and the distance between the first imaging mark and the fourth imaging mark is in a range of 40 to 200 mm.

The present disclosure further provides a transcatheter treatment system, including:
a guide tube assembly as mentioned above;
an inner sheath movably inserted in the guide tube assembly; and
a treatment assembly movably installed in the inner sheath and being extendable out of a distal end of the inner sheath.

Optionally, the transcatheter treatment system further includes:
handles respectively arranged at proximal ends of the treatment assembly, the inner sheath and the guide tube assembly; and
an auxiliary support including a guide rod and a plurality of clamping members that are movable along the guide rod and positionable on the guide rod, wherein the handles are respectively installed on corresponding clamping members and relative positions of the handles are adjusted by movement of the clamping members.

Optionally, the guide tube assembly includes a first tube and a second tube slidably inserted into the first tube, and proximal ends of the first tube and the second tube are respectively configured with handles corresponding to respective clamping members on the auxiliary support.

The first tube and the second tube are respectively configured with imaging marks, and relative positions of the imaging marks on the first tube and the second tube are adjusted through the respective clamping members on the auxiliary support.

Optionally, the treatment assembly is an ablation assembly including an ablation needle; or the treatment assembly is an injection assembly for supplying hydrogel.

Optionally, the hydrogel has a storage modulus in a range of 2880 to 4250 Pa and a loss modulus in a range of 200 to 800 Pa when supplied in the injection assembly.

Optionally, the treatment assembly is an injection assembly, the injection assembly includes a needle body, a needle connector and a needle connection tube, the needle connector connects the needle body and needle connection tube, and the needle connection tube includes an inner tube and an outer tube, wherein an interior of the inner tube is a first channel, a gap is defined between the inner tube and the outer tube and serves as a second channel, and a third channel is defined between a catheter and the injection assembly.

The distal end of the inner sheath is provided with a sleeve for abutting a target tissue region, the sleeve includes a main body, the main body defines a fourth channel passing through the main body in an axial direction, and a side wall of the main body defines at least one fifth channel which is communicated with the fourth channel.

Optionally, the main body includes a distal section, and the distal section is provided with an abutment surface for abutting a myocardial tissue; and the sleeve further includes at least one sliding member, at least a part of the sliding member protrudes from the abutment surface, and the sliding member is configured to selectively open or close the fifth channel.

Optionally, the sliding member has a first position for opening the fifth channel and a second position for closing the fifth channel, and the sleeve further includes at least one spring element acting on the sliding member to move the sliding member toward the second position.

Optionally, the sleeve further includes at least one sixth channel communicated with the fifth channel, and the sliding member and the spring element are arranged in the sixth channel.

Optionally, a distal end of the sixth channel defines an opening on the abutment surface, and a proximal end of the sixth channel is closed or provided with a blocking member, wherein a distal end of the sliding member protrudes from the abutment surface through the opening, and the sliding member is configured to selectively open or close the fifth channel according to a position of the sliding member in the sixth channel.

Optionally, a proximal end of the sliding member is connected to the spring element, and a proximal end of the spring element abuts a proximal end of the sixth channel.

Optionally, the sliding member defines a through hole, and when the spring element is in a first state, the sliding member is in the first position, and the through hole communicates with the fifth channel.

When the spring element is in a second state, the sliding member is in the second position, and the sliding member blocks the fifth channel.

Optionally, an outer wall of the main body has a balance opening communicated to a proximal end of the sixth channel.

Optionally, the main body includes a proximal section, an outer diameter of the proximal section gradually increases from a proximal end to a distal end, and a radial dimension of a part of the fourth channel corresponding to the proximal section is a constant value or gradually decreases.

Optionally, the main body further includes a middle section extending from the proximal section to the distal section, the middle section defines the fifth channel, and a radial dimension of a part of the middle section adjacent to the proximal section is greater than a radial dimension of a part of the middle section adjacent to the distal section.

Optionally, a radial dimension of the distal section is a constant value, or a radial dimension of a part of the distal section adjacent to the middle section is greater than a radial dimension of a part of the distal section away from the middle section.

Optionally, at least one electrode is provided on a side wall of the main body, or the main body is made of metal.

Optionally, an operating handle is connected to proximal ends of the inner sheath and the injection assembly, and the operating handle includes a housing and a driving assembly; wherein the driving assembly includes a driving knob that is rotatably connected to the housing and a driving rod that is movably inserted into the housing and threaded to the driving knob.

The proximal end of the inner sheath is fixedly connected to the housing.

The proximal end of the injection assembly is fixedly connected to the driving rod.

The driving rod is configured to move axially by rotation of the driving knob relative to the housing so as to drive the injection assembly to move and thus drive the needle body to penetrate into the target tissue region.

Optionally, a damping structure is provided between the driving knob and the housing.

Optionally, the damping structure includes a damping ring and a rib in an interference fit with the damping ring, and one of the damping ring and the rib is provided on the housing, and the other is provided on the driving knob.

Optionally, a part of the driving knob extends into an interior of the housing and is provided with a circumferential groove, and the damping ring is at least partially received in the circumferential groove; and the rib protrudes inward on an inner wall of the housing, and the rib press the damping ring.

Optionally, the operating handle further includes an injection interface seat located outside the housing, a proximal end of the driving rod is fixedly connected to the injection interface seat, and a proximal end of the injection assembly is fixedly connected to the injection interface seat.

Optionally, the driving rod defines a connection through-hole extending in an axial direction, a first protection tube is arranged in the connection through-hole and extends out of the connection through-hole, and the injection assembly is inserted into the first protection tube and the connection through-hole.

Optionally, the operating handle further includes a hemostatic valve assembly fixedly installed in the housing, and a distal end of the hemostatic valve assembly is fixedly connected to the proximal end of the inner sheath, wherein the hemostatic valve assembly includes a sealing gasket, a second protection tube passes through the sealing gasket, the second protection tube is connected to the sealing gasket in a tight seal, and the injection assembly passes through the second protection tube.

Optionally, the transcatheter treatment system further includes a feedback mechanism, the feedback mechanism including:
a middle piece fixedly connected to the housing of the operating handle, an outer wall of the middle piece being provided with a first abutment portion;
a sleeving tube movably arranged around the middle piece, an inner wall of the sleeving tube is provided with a second abutment portion, wherein in an initial state, a proximal end of the sleeving tube contacts a distal end of the housing, and the second abutment portion is located on a proximal side of the first abutment portion; and
a spring element, one end of the spring element abuts the first abutment portion, and an other end of the spring element abuts the second abutment portion.

When the distal end of the inner sheath abuts the target tissue region, the middle piece is forced to press the spring element and the middle piece moves proximally relative to the sleeving tube, generating a gap between the proximal end of the sleeving tube and the distal end of the housing.

Optionally, the guide tube assembly includes a first tube and a second tube slidably inserted in the first tube, the first curved section is provided by the first tube, and the second curved section is provided by the second tube.

The first tube is a bendable guiding sheath, and a proximal end of the guiding sheath is connected with a guide handle for controlling the guiding sheath.

The second tube is a bendable sheath, and a proximal end of the bendable sheath is connected with a bending handle for controlling the bendable sheath.

The transcatheter treatment system further includes an adjusting device, and the adjusting device includes:
a connecting piece, fixedly connected to a proximal end of the bending handle;
a guide tube, inserted into the connecting piece and rotatably connected to the connecting piece, the inner sheath being movably inserted into the guide tube;
a middle piece, movably arranged around the guide tube and fixedly connected to the housing; and
an adjusting piece, movably provided on the middle piece and configured to directly or indirectly lock or unlock the guide tube in a radial direction of the middle piece to prevent or allow axial movement of the middle piece relative to the guide tube, thereby preventing or allowing axial movement of the operating handle relative to the bending handle.

The present disclosure further provides a transcatheter treatment system having opposite distal and proximal ends, the transcatheter treatment system including:
a guide tube assembly, including a first tube and a second tube slidably inserted in the first tube, the first tube having a first, bendable curved section, and the second tube having a second, bendable curved section;
a guide handle, a proximal end of the first tube being connected to the guide handle;
a bending handle, a proximal end of the second tube being connected to the bending handle;
an inner sheath, movably inserted into the guide tube assembly;
a treatment assembly, movably installed in the inner sheath and being extendable out of a distal end of the inner sheath; and
an operating handle, proximal ends of the inner sheath and the treatment assembly being both connected to the operating handle.

The guide handle, the bending handle and the operating handle are arranged in sequence from a distal end to a proximal end, and distances therebetween are adjustable.

Optionally, the guide handle, the bending handle and the operating handle have a synchronous motion state after at least two of them are locked with each other, and an independent motion state after unlocking.

Optionally, the transcatheter treatment system further includes an auxiliary support including a guide rod and a plurality of clamping members that are movable along the guide rod and positionable on the guide rod, and wherein at least one of the guide handle, the bending handle and the operating handle is installed on a corresponding clamping member, and a position of the at least one of the guide handle, the bending handle and the operating handle is adjusted by movement of the corresponding clamping member.

Optionally, the transcatheter treatment system is further provided with a locking mechanism for maintaining the synchronous motion state after locking or for unlocking and switching to the independent motion state.

The locking mechanism acts between the clamping member and the guide rod, or between two adjacent ones of the guide handle, the bending handle and the operating handle.

Optionally, in the transcatheter treatment system, a driving wheel is configured for the operating handle, and the driving wheel is configured to drive the operating handle through a transmission mechanism to change a distance from the bending handle.

The present disclosure further provides a transcatheter treatment method, including:
providing the transcatheter treatment system as mentioned above;
delivering a distal part of the transcatheter treatment system into a left ventricle via an aortic arch; and
determining a current treatment location on a endocardium of the left ventricle and performing operations.

The technical solution of the present disclosure can at least simplify the guidance operation, reduce the pressure to human blood vessels, and improve the reliability of the device.

### BRIEF DESCRIPTION OF DRAWINGS

FIG. 1 is a schematic perspective view of an assembled interventional injection system according to an embodiment of the present disclosure;
FIG. 2 is a perspective view of an assembled interventional injection device of the interventional injection system shown in FIG. 1;
FIG. 3 is a schematic view of the interventional injection device shown in FIG. 2 from another perspective;
FIG. 4 is a schematic exploded view of the interventional injection device shown in FIG. 2;
FIG. 5 is a partial axial cross-sectional view of the interventional injection device shown in FIG. 2;
FIG. 6 is a side view of a driving rod, a first protection tube, and an injection interface seat of the interventional injection device which are assembled together;
FIG. 7a is a side view of the driving rod and the first protection tube assembled together;
FIG. 7b is an axial cross-sectional view of the driving rod and the first protection tube assembled together;
FIG. 8 is a partially enlarged view of the proximal section of an operating handle of the interventional injection device shown in FIG. 5;
FIG. 9a is a schematic view of a damping ring in the operating handle of the interventional injection device;
FIG. 9b is a cross-sectional view of the damping ring shown in FIG. 9a;
FIG. 10a is a side view of an injection member and the injection interface seat of the interventional injection device which are assembled together;
FIG. 10b is an axial cross-sectional view of the injection member and the injection interface seat of the interventional injection device which are assembled together;
FIG. 11 is an axial cross-sectional view of a hemostatic valve assembly in the operating handle of the interventional injection device;
FIG. 12a is a top view of a sealing gasket in the hemostatic valve assembly;
FIG. 12b is a cross-sectional view of the sealing gasket;
FIG. 13 is a schematic structural view of the distal end of a sheath of the interventional injection device;
FIG. 14a is a schematic view of an adjusting device of the interventional injection system;
FIG. 14b is an axial cross-sectional view of the adjusting device shown in FIG. 14a;
FIG. 15a is a perspective exploded view of an adjustment knob and an adjusting piece of the adjusting device;
FIG. 15b is an axial cross-sectional view of the adjustment knob and the adjusting piece of the adjusting device which are assembled together;
FIGS. 16 to 18 are schematic views showing the process of injecting a myocardial repair substance into the myocardial tissue using the interventional injection system;
FIG. 19 is an axial cross-sectional view of an interventional injection system according to another embodiment of the present disclosure;
FIG. 20 is a cross-sectional view of an adjusting device and an operating handle when a head at the distal end of a sheath in the interventional injection system shown in FIG. 19 does not abut against the target tissue region;
FIG. 21 is a cross-sectional view of the adjusting device and the operating handle when the head at the distal end of the sheath in the interventional injection system shown in FIG. 19 abuts against the target tissue region;
FIG. 22 is a schematic view of the heart;
FIG. 23 is a schematic structural view of a sleeve according to an embodiment of the present disclosure;
FIG. 24 is a cross-sectional view of the sleeve in FIG. 23;
FIG. 25 is a schematic structural view of a sleeve according to another embodiment of the present disclosure;
FIG. 26 is a cross-sectional view of the sleeve in FIG. 25;
FIG. 27 is a view of the sleeve in FIG. 25 in one working state;
FIG. 28 is a view of the sleeve in FIG. 25 in another working state;
FIG. 29 is a schematic structural view of a catheter and a sleeve of an injection device according to an embodiment of the present disclosure;
FIG. 30 is a schematic structural view of an injection device according to an embodiment of the present disclosure;
FIG. 31 is an exploded view of an injection assembly shown in FIG. 30;
FIG. 32 is a schematic structural view of the catheter shown in FIG. 29;
FIG. 33 is a schematic view of the exposed part of the conductive wire in the catheter in FIG. 32;
FIG. 34 is a schematic view of a variant of the catheter in FIG. 29;
FIG. 35 is a schematic structural view of an injection system according to an embodiment of the present disclosure;
FIG. 36 is a schematic structural view of the handle shown in FIG. 35;
FIGS. 37 to 45 are schematic views showing the process of cardiac tissue wall injection using the injection system of the present disclosure;
FIG. 46 is a schematic structural view of an ablation assembly of an ablation device according to an embodiment of the present disclosure;
FIG. 47 is a schematic structural view of an ablation system according to an embodiment of the present disclosure;
FIG. 48 is a schematic view of an overall adaptive guiding device according to an embodiment of the present disclosure;
FIG. 49 is a schematic partial view of a flexible catheter according to an embodiment of the present disclosure;
FIG. 50 is a schematic partial front view of the flexible catheter according to an embodiment of the present disclosure;
FIG. 51 is a schematic partial side view of the flexible catheter according to an embodiment of the present disclosure;
FIG. 52 is a schematic partial bottom view of the flexible catheter according to an embodiment of the present disclosure;
FIGS. 53a to 53b are schematic views showing imaging marks of the guide tube assembly according to an embodiment of the present disclosure;
FIG. 54a is a schematic view showing the flexible catheter according to an embodiment of the present disclosure crossing the aortic arch of the heart;
FIGS. 54b to 54c are schematic views showing the flexible catheter according to an embodiment of the present disclosure in the heart;
FIG. 55 is a schematic view showing the assembly of the flexible catheter and the pull assembly according to an embodiment of the present disclosure;
FIG. 56 is a cross-sectional view of section C-C in FIG. 55;
FIG. 57 is an enlarged view of part M in FIG. 56;
FIG. 58 is a cross-sectional view of section D-D in FIG. 55;
FIG. 59 is a schematic view showing the bend direction of the flexible catheter according to an embodiment of the present disclosure;
FIG. 60 is a schematic view of the flexible catheter according to an embodiment of the present disclosure when used in the heart and being bent;
FIG. 61 is a schematic view of an overall transcatheter endocardium injection system according to an embodiment of the present disclosure;
FIG. 62 is an enlarged view of part N in FIG. 61;
FIG. 63 is an exploded view of a transcatheter endocardium injection system according to an embodiment of the present disclosure;
FIG. 64 is an exploded view of a transcatheter endocardium injection system according to another embodiment of the present disclosure;
FIG. 65 is a schematic view showing a needle of a transcatheter endocardium injection system according to an embodiment of the present disclosure penetrated into the free wall;
FIG. 66 is a schematic view showing the bend direction of a bending sheath and the flexible catheter of the adaptive guiding device of the transcatheter endocardium injection system according to an embodiment of the present disclosure;
FIG. 67 is a schematic view showing a first state in which the transcatheter endocardium injection system according to an embodiment of the present disclosure is delivered into the left atrium of the heart;
FIG. 68 is an enlarged view of part O in FIG. 66;
FIG. 69 is a schematic view showing a second state in which the transcatheter endocardium injection system according to an embodiment of the present disclosure is delivered into the left atrium of the heart;
FIG. 70 is a further exemplary structural view of a transcatheter endocardium injection system according to an embodiment of the present disclosure;
FIGS. 71 to 75 are schematic views showing the process of using the transcatheter treatment system according to an embodiment of the present disclosure; and
FIG. 76 is a schematic view of a transcatheter treatment system according to an embodiment of the present disclosure using a myocardial ablation device as a treatment device.

### DESCRIPTION OF EMBODIMENTS

In order to make the objects, technical solutions and advantages of the embodiments of the disclosure more apparent, the technical solutions according to the embodiments of the disclosure will be described below in detail with reference to the drawings in the embodiments of the disclosure.

It should be understood that terms such as "comprises" and "can comprise" used in this specification specify the presence of disclosed features, operations, or elements, and do not preclude one or more additional features, operations, or elements. In the present disclosure, terms such as "includes" and/or "has" specify the presence of specific characteristics, numbers, operations, elements, components, or combinations thereof, but do not preclude the presence or addition of one or more other characteristics, numbers, operations, elements, components, or combinations thereof.

Furthermore, in the present disclosure, the phrase "and/or" includes any one or more of the listed elements. For example, the phrase "A and/or B" includes "A", "B", or "A and B".

In the present disclosure, expressions including ordinal numbers such as "first" and "second" can be used to modify various elements. However, these elements are not limited by such expressions. For example, such expressions do not limit the order and/or importance of the elements. Such expressions are only intended to distinguish one element from other elements. Similarly, a first element could be termed a second element, and a second element could be termed a first element, without departing from the scope of the present disclosure.

When a component "connects" or is "connected" to another component, it will be understood that the component can directly connect or be directly connected to another component, or connect or be connected to another component through an intermediate component. However, when a component "directly connects" or is "directly connected" to another component, it should be understood that no intermediate component is present between them.

In the field of interventional medical device technology, the proximal end refers to the end close to the operator, and the distal end refers to the end far away from the operator. The axial direction refers to the direction parallel to the line connecting the distal center and the proximal center of the medical device, the radial direction refers to the direction perpendicular to the axial direction, and the circumferential direction refers to the direction surrounding the axial direction.

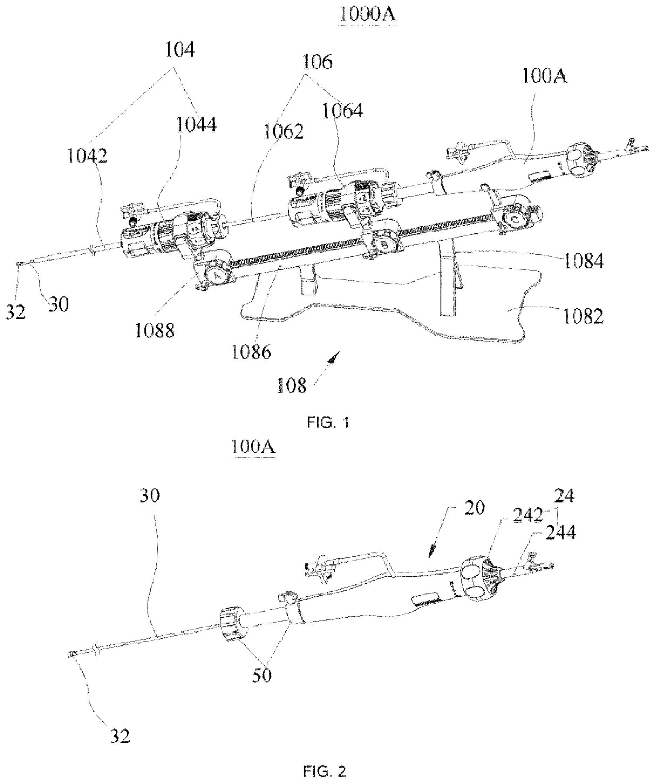

Referring to FIG. 1, an embodiment of the present disclosure provides an interventional injection system 1000A, which can inject a first injectable substance into a target tissue region through different interventional paths to repair or treat the target tissue region.

In this embodiment, the target tissue region is myocardial tissue. The interventional injection system 1000A can be introduced into the heart through a catheter and inject the first injectable substance into the myocardial tissue of a heart failure patient, to repair or treat the heart failure. Preferably, the first injectable substance is, but not limited to, an alginate hydrogel composed of sodium alginate system, calcium alginate system, and sodium chloride solution. The myocardial tissue can be but is not limited to atrial wall, ventricular wall, or interventricular septum, or the like.

It can be understood that the interventional injection system 1000A is also suitable for injection treatment for other target tissue regions.

The interventional injection system 1000A includes an interventional injection device 100A, a guiding device 104, a bending device 106 and an auxiliary support 108. The guiding device 104, the bending device 106, and the interventional injection device 100A are all supported by the auxiliary support 108. The guiding device 104 includes a bendable guiding sheath 1042 and a guide handle 1044 for controlling the guiding sheath 1042. The bending device 106 includes a bendable sheath 1062 and a bending handle 1064 for controlling the bendable sheath 1062. The bendable sheath 1062 is movably inserted into the guide handle 1044 and the guiding sheath 1042. The interventional injection device 100A is movably inserted into the bending device 106 and is configured to penetrate into the target tissue region for injection.

During surgery, the guiding sheath 1042 first enters the patient's body to establish a path for the bendable sheath 1062 and the interventional injection device 100A. The bendable sheath 1062 has good bending and steering performance. By adjusting the bendable sheath 1062, the interventional injection device 100A passing through the bendable sheath 1062 can be adjusted to approach the target tissue region and to a suitable injection site.

The auxiliary support 108 includes a bottom plate 1082, a support post 1084, a guide rod 1086 and three clamping members 1088. The support post 1084 is vertically fixed on the bottom plate 1082. The guide rod 1086 is fixed to an end of the support post 1084 away from the bottom plate 1082. The guide rod 1086 is inclined relative to the bottom plate 1082. The three clamping members 1088 are respectively movable along the guide rod 1086 and positionable on the guide rod 1086. The guide handle 1044, the bending handle 1064 and the interventional injection device 100A each have a clamping position where the corresponding clamping member 1088 can clamp and fix the corresponding component, thereby ensuring the positional stability of the corresponding component on the guide rod 1086. For example, after the guide handle 1044 and the bending handle 1064 are adjusted and fixed on the guide rod 1086 through the corresponding clamping members 1088, the relative positions of the guide handle 1044 and the bending handle 1064 are fixed and maintained. The present disclosure does not limit the structure of the auxiliary support 108.

Referring to FIGS. 1 to 5, the interventional injection device 100A includes an operating handle 20, a sheath 30 and an injection member 40. The operating handle 20 includes a housing 22 and a driving assembly 24. The driving assembly 24 includes a driving knob 242 that is rotatably connected to the housing 22 and a driving rod 244 that is movably inserted into the housing 22 and is threaded to the driving knob 242. The proximal end of the sheath 30 is fixedly connected to the housing 22. The sheath 30 is movably inserted into the bending handle 1064 and the bendable sheath 1062 of the bending device 106. The injection member 40 is movably inserted into the sheath 30.

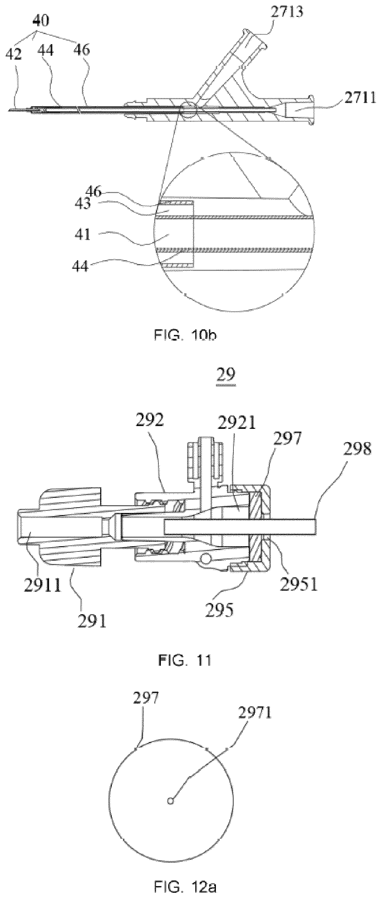

The injection member 40 includes a first channel 41 (as shown in FIG. 10b) and an injection needle 42 connected to the distal end of the first channel 41 in a tight seal. The proximal end of the injection member 40 is fixedly connected to the driving rod 244. The driving rod 244 is configured to be driven by the rotation of the driving knob 242 relative to the housing 22 to move axially, so as to drive the injection member 40 to move, so that the injection needle 42 can penetrate into the target tissue region.

When the injection needle 42 is required to move from the proximal end to the distal end (to advance the needle), for example, when the injection needle 42 is required to penetrate into the target tissue region, the driving knob 242 is operated to rotate in the first rotation direction (for example, clockwise), and thus the driving rod 244 is driven to move axially in a straight line from the proximal end to the distal end.

When the injection needle 42 is required to move from the distal end to the proximal end (to withdraw the needle), for example, when the injection needle 42 is required to move out from the target tissue region, the driving knob 242 is operated to rotate in a second rotation direction opposite to the first rotation direction (for example, counterclockwise), and thus the driving rod 244 is driven to move axially in a straight line from the distal end to the proximal end.

Traditional interventional injection devices usually control the injection needle to penetrate based on ejection or push-pull techniques. The ejection technique has extremely high requirements on the coaxiality of a plurality of tubes that form a nested tubular assembly. When the coaxiality requirements are not met, the injection needle, when being ejected, is prone to collide with the sheath, damaging and blunting the tip of the injection needle in severe cases, thereby affecting the accuracy of the surgery. The push-pull technique has poor controllability, and it is difficult to control the forward and backward speed of the needle through the handle when advancing and withdrawing the needle.

In the interventional injection device 100A according to the present disclosure, the driving rod 244 and the driving knob 242 are threaded to each other, to convert a rotational motion into a linear motion for advancing or withdrawing the injection needle 42. During operation, the advance and withdrawal speed of the needle can be controlled more accurately, effectively and stably. Further, the threaded connection has a certain self-locking property, so that backout of the injection needle 42 can be effectively prevented when the driving rod 244 drives the distal injection needle 42 to penetrate into the target tissue region, and the stability of the needle advancing process is further improved, improving the accuracy and efficiency of surgery.

In this embodiment, the housing 22 includes two mutually snap-fastenable first housings 2200, 2210. The first housings 2200, 2210 have a clamping position 2220 where the corresponding clamping member 1088 can clamp and engage with the first housings 2200, 2210.

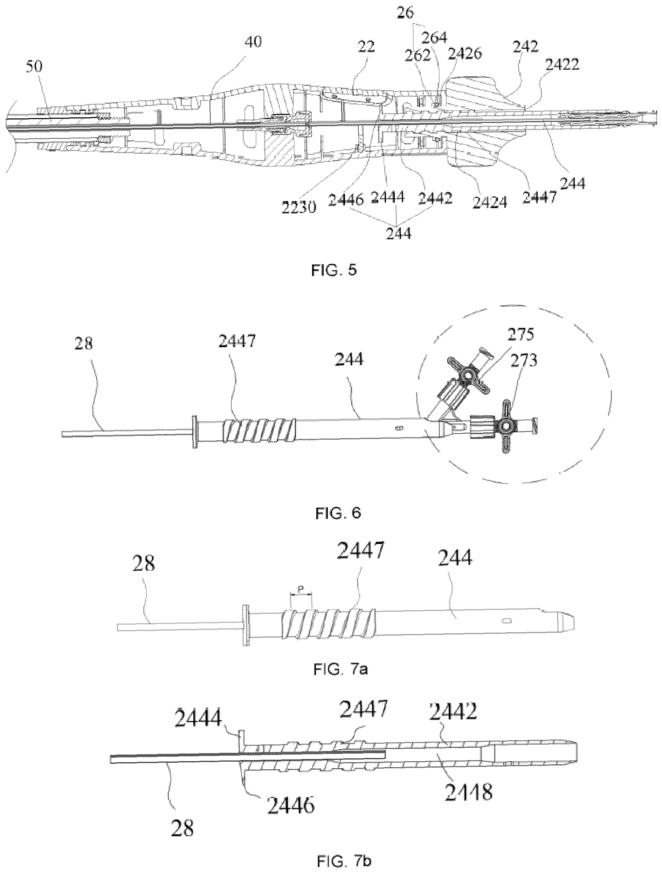

The driving knob 242 is rotatably connected to the proximal end of the housing 22 and is partially exposed outside the housing 22. The driving knob 242 can only rotate relative to the housing 22 but cannot move axially. The driving knob 242 defines an axially extending threaded hole 2422 for the driving rod 244 to pass through. The wall of the threaded hole 2422 is provided with a female thread 2424 (as shown in FIG. 5) for threaded connection with the driving rod 244.

Referring to FIG. 5, FIG. 6, FIG. 7a and FIG. 7b, the driving rod 244 includes a rod body 2442, a limiting portion 2444 and an indicator portion 2446. The outer wall of the rod body 2442 is provided with a male thread 2447. The rod body 2442 is inserted into the threaded hole 2422. The female thread 2424 of the driving knob 242 is threaded to the male thread 2447 of the rod body 2442. The male thread 2447 of the rod body 2442 can be a rectangular thread, a trapezoidal thread, a sawtooth thread, or the like. In this embodiment, the male thread 2447 of the rod body 2442 is preferably a trapezoidal thread. The trapezoidal thread has high root strength. The female thread 2424 of the threaded hole 2422 and the male thread 2447 of the rod body 2442 fit with each other through conical surfaces, with good centering performance, and are not susceptible to loosening, meeting usage needs better.

In this embodiment, when the driving knob 242 is rotated in the first rotation direction, the injection member 40 connected to the driving rod 244 moves distally. In an application scenario, before operating the driving knob 242, the safe distance between the injection needle 42 and the most distal end of the sheath 30 is about 7.5 mm, and the penetration depth of the injection needle 42 into the target tissue region is about 4.5 mm. The pitch P of the trapezoidal thread is approximately 8 millimeters (mm), and the thread is a single-start thread. One complete rotation of the driving knob 242 enables the driving rod 244 to drive the injection needle 42 to move a distance of L = P * 1 = 8 mm. Then, one and a half rotations of the driving knob 242 in the first rotation direction can cause the injection needle 42 to penetrate into the target tissue region at a suitable depth.

In other embodiments of the present disclosure, the safe distance between the injection needle 42 and the most distal end of the sheath 30 is not limited, the range of the thread pitch is not limited, and the moving distance of the injection needle 42 driven by the driving rod 244 through one complete rotation of the driving knob 242 is not limited.

The limiting portion 2444 radially protrudes from the outer wall of the rod body 2442. A blocking part 2230 (as shown in FIG. 5) is provided on the inner wall of the housing 22. The blocking part 2230 functions to block the limiting portion 2444 to limit the axial moving distance of the driving rod 244. When the driving rod 244 moves distally until the limiting portion 2444 is blocked by the blocking part 2230, it means that the driving rod 244 has reached a preset limit position and cannot continue moving distally, thereby controlling the injection depth of the injection needle 42 within a reasonable range.

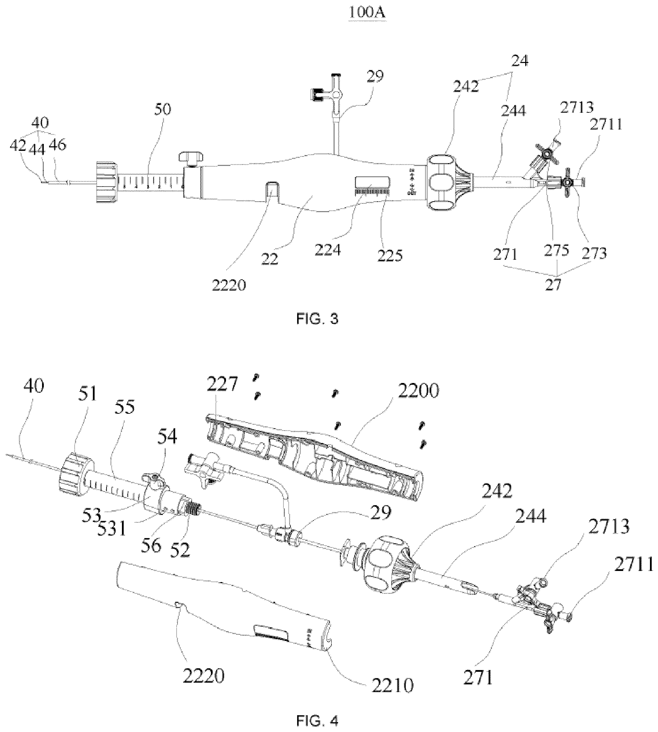

The indicator portion 2446 (shown in FIGS. 5 and 7b) radially protrudes from the outer wall of the rod body 2442, and the housing 22 is provided with a viewing window 224 (shown in FIG. 3) to show the indicator portion 2446. The housing 22 is further provided with a scale 225 (as shown in FIG. 3) for indicating the position of the indicator portion 2446. The viewing window 224 can be a transparent area or a hollow area. When the driving rod 244 moves relative to the housing 22, the position of the indicator portion 2446 can be determined through the viewing window 224 and the scale 225, so that the operator can determine the movement distance of the distal injection needle 42. In this way, the injection needle 42 can be prevented from penetrating too deeply into the target tissue region, otherwise the surgery may fail. For example, in the case where the target tissue region is the ventricular wall, if the injection needle 42 penetrates into the myocardial tissue too deeply, it may puncture the ventricular wall.

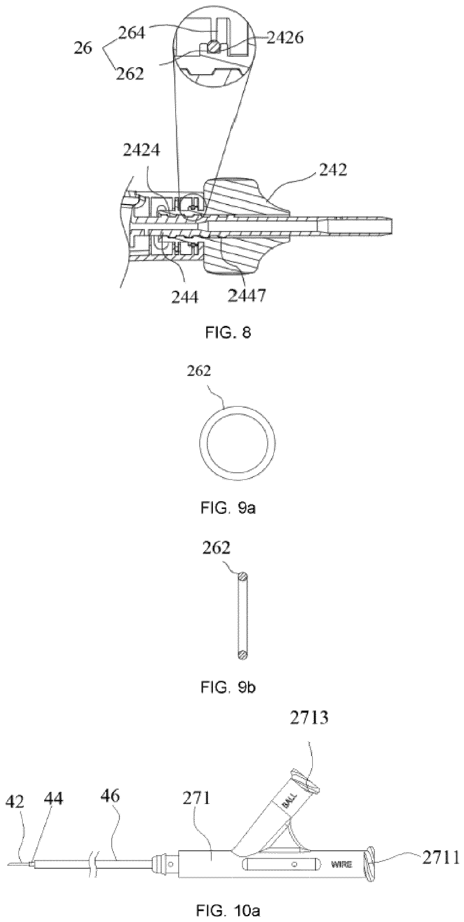

Referring to FIGS. 5 and 8, the operating handle 20 further includes a damping structure 26 located between the housing 22 and the driving knob 242, which functions to cooperate with the housing 22 to produce a damping effect during the rotation of the driving knob 242, to improve the handling feel of the driving knob 242 and make the rotation speed of the driving knob 242 more precise, thereby improving the uniformity and accuracy of the advance or withdrawal speed of the injection needle 42. The damping structure 26 includes a damping ring 262 and a rib 264 in an interference fit with the damping ring 262. In this embodiment, the rib 264 has a complete annular structure that is provided on the housing 22 and protrudes toward the interior of the housing. The driving knob 242 defines a circumferential groove 2426, and the circumferential groove 2426 is located in the housing 22. In other words, part of the driving knob 242 extends into the interior of the housing 22 and defines the circumferential groove 2426. The damping ring 262 is at least partially received in the circumferential groove 2426, facilitating the assembly of the damping ring 262 on the driving knob 242.

In this embodiment, the damping ring 262 is preferably but not limited to silicone, having good tensile strengths and tear resistances. The present disclosure does not limit the material of the damping ring 262, and for example, the material of the damping ring 262 can be rubber, provided that the damping ring 262 can provide a damping effect.

The damping effect of the damping structure 26 is related to the cross-sectional shape of the damping ring 262 and the interference amount between the damping ring 262 and the rib 264. The radial cross-sectional shape of the damping ring 262 can be rectangular, circular, trapezoidal or other special shapes. In this embodiment, the damping ring 262 is an O-ring (see FIG. 9a), and the radial cross section of the damping ring 262 is circular (see FIG. 9b), facilitating the manufacture. In addition, the damping ring 262 with a circular radial cross section can be assembled conveniently, without considering the installation direction. Depending on the magnitude of the damping, the interference amount on one side can be set in the range of 0.1 mm to 0.5 mm. In this embodiment, the interference amount on one side is preferably 0.25 mm, to ensure that the operator has more comfortable and good damping feel when rotating the driving knob 242, and thus to ensure an appropriate withdrawal speed of the needle. In other embodiments of the present disclosure, the range of the interference amount is not limited.

In other embodiments of the present disclosure, one of the damping ring 262 and the rib 264 is provided on the housing 22, and the other is provided on the driving knob 242. In other embodiments of the present disclosure, the rib can be consisted of a plurality of arc-shaped sections which are spaced from each other.

Referring to FIGS. 3, 4, 6, 10a and 10b, the operating handle 20 further includes an injection interface seat 27 located outside the housing 22. In this embodiment, the injection interface seat 27 is generally Y-shaped. The injection interface seat 27 is fixedly connected to the proximal end of the driving rod 244 and the proximal end of the injection member 40 is fixedly connected to the injection interface seat 27. The injection interface seat 27 includes a seat body 271, a first valve 273 and a second valve 275. In other embodiments of the present disclosure, the shape of the injection interface seat 27 is not limited.

The seat body 271 is fixedly connected to the proximal end of the rod body 2442. The seat body 271 and the rod body 2442 can be formed in one piece or separate pieces. In this embodiment, the seat body 271 and the driving rod 244 are formed in separate pieces, so that the structural complexity of the components can be reduced, facilitating the processing and manufacturing of the components, and the injection interface seat 27 and the driving rod 244 can be separately modularized and be conveniently assembled together. The injection interface seat 27 and the rod body 2442 can be connected by snapping, ultrasonic welding, glue bonding, etc. In this embodiment, the seat body 271 and the rod body 2442 are bonded with medical glue, providing better sealing effect.

The injection member 40 further includes a second channel 43 that is isolated from the first channel 41. The seat body 271 includes a first branch interface 2711 and a second branch interface 2713 that are isolated from each other. The first branch interface 2711 and the second branch interface 2713 are both connected to the injection member 40. The first branch interface 2711 and the second branch interface 2713 can be connected to different syringes. The first branch interface 2711 is selectively communicated to the first channel 41 and is used to provide a first injectable substance to the injection member 40. The second branch interface 2713 is selectively communicate to the second channel 43 and is used to provide a second injectable substance to the injection member 40. In this embodiment, the first injectable substance can be a myocardial repair substance, and the second injectable substance can be a contrast medium or other drugs. In other embodiments of the present disclosure, the functions and ingredients of the first injectable substance and the second injectable substance are not limited.

The first valve 273 is provided on the seat body 271 and is used to control the opening or closing of the first branch interface 2711. The second valve 275 is provided on the seat body 271 and is used to control the opening or closing of the second branch interface 2713. By operating the first valve 273, the first branch interface 2711 can be communicated to its corresponding syringe in one-way or be closed. By operating the second valve 275, the second branch interface 2713 can be communicated to its corresponding syringe in one-way or be closed.

Referring to FIG. 6, FIG. 7a and FIG. 7b again, the rod body 2442 defines a connection through-hole 2448 extending in the axial direction. The operating handle 20 further includes a first protection tube 28 inserted into the connection through-hole 2448 for the injection member 40 to pass through. The material of the first protection tube 28 includes but is not limited to stainless steel.

Referring to FIG. 4 and FIG. 11, the operating handle 20 further includes a hemostatic valve assembly 29 fixedly accommodated in the housing 22 for preventing blood leakage. The distal end of the hemostatic valve assembly 29 is fixedly connected to the proximal end of the sheath 30. In this embodiment, the hemostatic valve assembly 29 and the driving assembly 24 are coaxially installed in the housing 22 from distal end to proximal end. In other embodiments of the present disclosure, the hemostatic valve assembly 29 and the driving assembly 24 may not be coaxially installed in the housing 22.

The hemostatic valve assembly 29 includes a first joint 291, a second joint 292, a gasket cap 295, a sealing gasket 297 and a second protection tube 298.

The first joint 291 is fixedly accommodated in the housing 22. The first joint 291 defines a first installation hole 2911 extending in the axial direction for fixing the proximal end of the sheath 30. The first joint 291 can be, but is not limited to, a Luer fitting.

The second joint 292 is fixed to the housing 22 and partially accommodated in the housing 22. The second joint 292 is fixedly connected to the proximal end of the first joint 291. The second joint 292 defines a second installation hole 2921 extending in the axial direction for the second protection tube 298 to pass through. The second joint 292 can be, but is not limited to, a T-shaped joint.

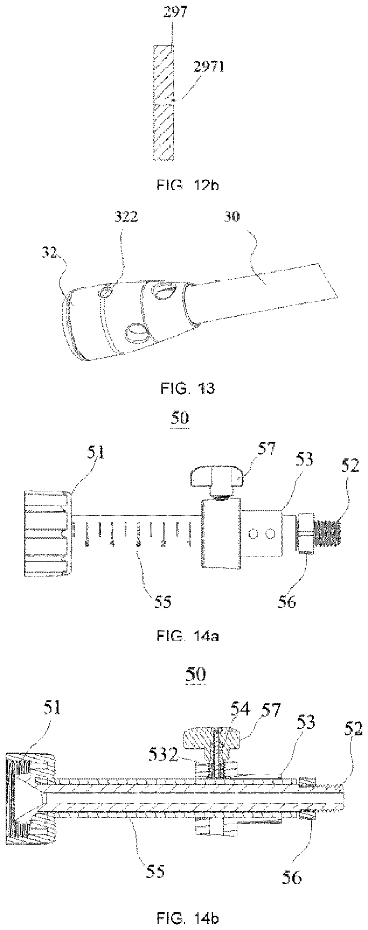

The gasket cap 295 is fixed to the proximal end of the second joint 292. The sealing gasket 297 is received in the gasket cap 295. The gasket cap 295 defines a third installation hole 2951. The sealing gasket 297 is clamped between the proximal end surface of the second joint 292 and the inner wall of the gasket cap 295. Referring to FIG. 12a and FIG. 12b, the sealing gasket 297 defines a through hole 2971 for the second protection tube 298 to pass through, which is connected with the second protection tube 298 in a tight seal.

The second protection tube 298 passes through the third installation hole 2951, the through hole 2971, and the second installation hole 2921. In this embodiment, the second protection tube 298 and the sealing gasket 297 have an interference fit. The material of the second protection tube 298 includes but is not limited to stainless steel. Since the sealing gasket 297 needs to directly contact the blood, the material of the sealing gasket 297 is preferably silicone. The hardness of silicone is in the range of 20 A to 40 A. In this embodiment, the hardness of silicone is preferably 30 A. The sealing gasket 297 with this hardness has a certain compressive strength and fits closely with the second protection tube 298 to prevent blood leakage. The through hole 2971 of the sealing gasket can be a square hole, a rectangular hole, a polygonal hole, a conical hole or a round straight hole, etc. The cross section of the second protection tube 298 fits with the through hole 2971 to improve the closeness between the sealing gasket 297 and the second protection tube 298. For example, if the cross section of the second protection tube 298 is circular, then the shape of the through hole 2971 of the sealing gasket 297 is also circular.

As the injection member 40 would be introduced into the human body, the remaining part of the injection member 40 except the injection needle 42 should be flexible so that the injection member 40 can conform to the curved human body lumen. The hard first protection tube 28 can protect the proximal end of the injection member 40 and prevent the proximal end of the injection member 40 from being bent. The injection member 40 passes through the hard second protection tube 298 to prevent the injection member 40 from being pressed by the sealing gasket 297 and to prevent the injection member 40 from being bent due to too much resistance from the sealing gasket 297 when the injection member 40 passes through the sealing gasket 297, allowing the injectable substance to flow smoothly to the distal end during injection.

Referring to FIG. 3, FIG. 11 and FIG. 13, the proximal end of the sheath 30 is fixed to the first installation hole 2911 of the first joint 291 and inserted into the housing 22. The distal end of the sheath 30 is exposed out from the distal end of the housing 22. Referring to FIG. 3 and FIG. 13, the distal end of the sheath 30 is provided with a head 32 for contacting the target tissue region. The head 32 defines an injection orifice 322. The injection orifice 322 is communicated with the second channel 43 and is used for injecting the second injectable substance. The second injectable substance in the second channel 43 can reach the target tissue region (for example, in the cardiac cavity) through the injection orifice 322 for imaging or drug release. Preferably, the axial direction of the injection orifice 322 is perpendicular to the axial direction of the sheath 30, and a plurality of injection orifices 322 are defined at intervals in the circumferential direction of the head 32.

The operating handle 20 functions to adjust the position of the flexible injection member 40 and the position of the head 32 against the target tissue region (the inner wall of the ventricle), provide connection interfaces for the injectable substances, and advance the needle for penetrating.

After the bendable sheath 1062 is adjusted to an appropriate position, the operating handle 20 can be pushed distally to make the head 32 closely contact the target tissue region (for example, the endocardium), and the injection needle 42 can be advanced by the driving assembly 24 of the operating handle 20 to penetrate into the target tissue region to perform injection.

Referring to FIG. 3, FIG. 10a and FIG. 10b, the injection member 40 further includes an inner tube 44 and an outer tube 46 surrounding the inner tube 44. The distal end of the inner tube 44 is connected to the injection needle 42 in a tight seal. The lumen of the inner tube 44 forms the first channel 41. The inner tube 44 is fixedly inserted into the seat body 271 and communicates with the first branch interface 2711.

The outer tube 46 is fixedly inserted into the seat body 271 and communicates with the second branch interface 2713. The outer tube 46 surrounds the inner tube 44, and the second channel 43 is defined between the inner wall of the outer tube 46 and the outer wall of the inner tube 44. The injection needle 42 is exposed outside the distal end of the outer tube 46, without affecting the penetrating of the injection needle 42.

In order to adapt to the curved interventional path, the inner tube 44 and the outer tube 46 are preferably flexible tubes. The inner tube 44 and the outer tube 46 both pass through the first protection tube 28 and the second protection tube 298. The first protection tube 28 and the second protection tube 298 can protect the flexible inner and outer tubes 44 and 46, reducing the risk of the inner tube 44 and the outer tube 46 being damaged by bending.

Since the seat body 271 and the driving rod 244 are fixedly connected together, after the inner tube 44 and the outer tube 46 are fixedly connected to the seat body 271, the injection member 40 and the driving rod 244 are connected in one piece. Therefore, the axial movement of the driving rod 244 can synchronously push the distal injection needle 42 to penetrate the target tissue region, for example, to penetrate the endocardium or the ventricular wall.

In other embodiments of the present disclosure, the injection member 40 can be a dual-lumen tube or a multi-lumen tube including the first channel 41 and the second channel 43.

The fluid input via the first branch interface 2711 is transported to the distal end of the injection needle 42 via the first channel 41 in the inner tube 44; and the fluid input via the second branch interface 2713 is transported to the distal end of the injection needle 42 through the second channel 43 between the outer tube 46 and the inner tube 44. That is, both the fluid input via the first branch interface 2711 and the fluid input via the second branch interface 2713 are output from the distal end of the injection needle 42, and there are two fluid channels in the injection member 40.

Further, the injection member 40 is movably inserted into the sheath 30, and a third channel 45 is formed between the inner wall of the sheath 30 and the outer wall of the outer tube 46. The proximal end of the third channel 45 communicates with the hemostatic valve assembly 29, and the distal end communicates with needle outlet of the head 32 (located on the distal end surface of the head 32) and the injection orifices 322 on the peripheral wall.

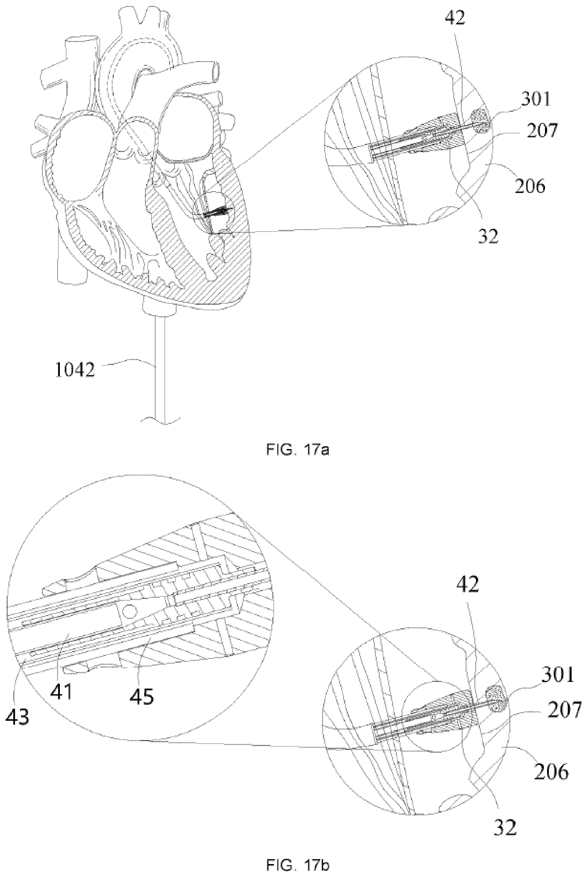

In summary, three fluid channels (the first channel 41, the second channel 43, and the third channel 45) are formed by the sheath 30 and the injection member 40 and different fluids can be transported therethrough as needed (see FIG. 17b).

Referring to FIGS. 3, 4, 14a and 14b, the interventional injection device 100A further includes an adjusting device 50 connected between the housing 22 and the bending handle 1064 for preventing or allowing the axial movement of the operating handle 20 relative to the bending handle 1064. The adjusting device 50 includes a connecting piece 51, a guide tube 52, a middle piece 53 and an adjusting piece 54.

The connecting piece 51 is fixedly connected to the proximal end of the bending handle 1064 and is used to connect the adjusting device 50 and the bending handle 1064 together.

The guide tube 52 is inserted into the connecting piece 51. The sheath 30 is movably inserted into the guide tube 52. The guide tube 52 is used to guide the sheath 30 into the bending handle 1064.

The middle piece 53 is movably arranged around the guide tube 52 and is fixedly connected to the housing 22. In this embodiment, an installation hole 531 is defined on the proximal end of the middle piece 53. A protruding installation post 227 is provided on the side wall of the inner cavity of the housing 22. The installation post 227 is engaged in the installation hole 531, thereby fixing the middle piece 53 to the housing 22. In other embodiments of the present disclosure, the fixed connection between the middle piece 53 and the housing 22 is not limited. For example, the middle piece 53 and the housing 22 can be fixed by gluing.

The adjusting piece 54 is movably provided on the middle piece 53 and is used to directly or indirectly lock or unlock the guide tube 52 in the radial direction of the middle piece 53 to prevent or allow the axial movement of the middle piece 53 relative to the guide tube 52, thereby preventing or allowing the axial movement of the operating handle 20 relative to the bending handle 1064, in order to adjust the length of the distal end of the sheath 30 extending beyond the distal end of the bendable sheath 1062.

In this embodiment, when the bottom of the adjusting piece 54 passes through the middle piece 53 and abuts against the guide tube 52, the guide tube 52 cannot move relative to the middle piece 53, and thus the operating handle 20 cannot move relative to the bending handle 1064, thereby fixing the position of the distal head 32 of the sheath 30 relative to the distal end of the bendable sheath 1062.

In this embodiment, when the adjusting piece 54 unlocks the guide tube 52, there is a gap between the adjusting piece 54 and the guide tube 52, the guide tube 52 can move relative to the middle piece 53, and thus the operating handle 20 can move axially relative to the bending handle 1064, so that the length of the distal head 32 of the sheath 30 extending beyond the distal end of the bending handle 1064 can be adjusted. When the adjusting piece 54 unlocks the guide tube 52, if the operating handle 20 is rotated, the operating handle 20 can drive the sheath 30 and the injection member 40 to rotate synchronously.

In this embodiment, the middle piece 53 defines an adjustment hole 532 extending in the radial direction. The adjustment hole 532 is a threaded hole. The adjusting piece 54 is inserted into the adjustment hole 532 and threaded with the adjustment hole 532 so that it can move in the adjustment hole 532.

The adjusting device 50 further includes a scale tube 55 and a positioning piece 56. The scale tube 55 is arranged around the guide tube 52. The scale tube 55 is located between the middle piece 53 and the guide tube 52. The positioning piece 56 is connected to the proximal end of the guide tube 52 so that the scale tube 55 is limited between the connecting piece 51 and the positioning piece 56 in the axial direction. In this embodiment, the proximal end of the guide tube 52 is threaded with the positioning piece 56. The scale tube 55 has scales in the axial direction to show the movement distance of the operating handle 20.

It can be understood that since the scale tube 55 is added outside the guide tube 52, the adjusting piece 54 indirectly abuts against or releases the guide tube 52 when the bottom of the adjusting piece 54 passes through the middle piece 53 to abut against or releases the scale tube 55.

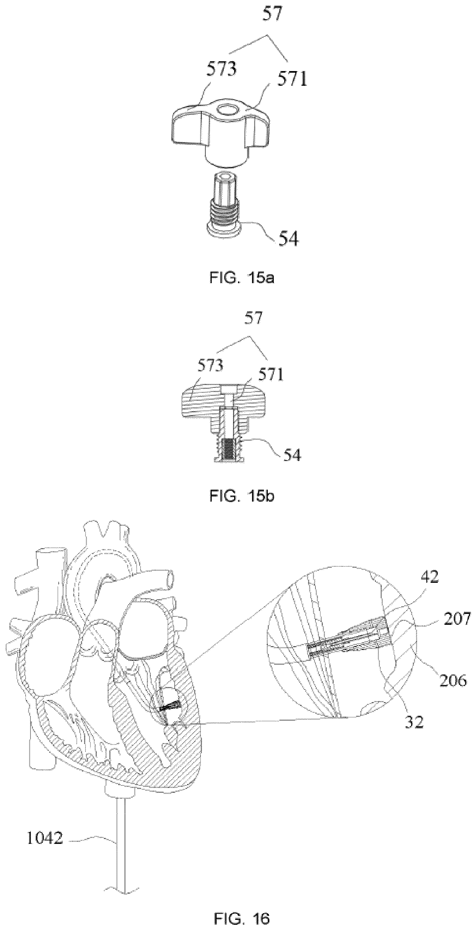

Referring to FIG. 15a and FIG. 15b, the adjusting device 50 further includes an adjustment knob 57 connected to the adjusting piece 54 wherein the adjustment knob 57 and the adjusting piece 54 cannot rotate relative to each other, and is used to drive the adjusting piece 54 to move in the radial direction of the middle piece 53. The adjustment knob 57 includes a base 571 and an adjustment indicator 573 provided on the outer wall of the base 571. The base 571 is arranged around the adjusting piece 54 and they cannot rotate relative to each other. In this embodiment, at least one pair of mating planes is provided between the base 571 and the adjusting piece 54 to prevent relative rotation therebetween. For example, the base 571 is provided with an octagonal hole, the top of the adjusting piece 54 has an octagonal cross-section, and the top of the adjusting piece 54 is engaged with the octagonal hole so that the adjusting piece 54 and the base 571 cannot rotate relative to each other.

The adjustment indicator 573 protrudes from the outer peripheral wall of the base 571 in the radial direction of the base 571, and the extension length thereof is different from that of the other part of the adjustment knob 57. In this embodiment, the adjustment indicator 573 has a longer extension length. When the adjustment indicator 573 points to a predetermined direction or when the adjustment indicator 573 rotates a predetermined angle, for example, 90°, it can be determined that the adjusting piece 54 has abutted against the scale tube 55.

When the adjustment knob 57 is loosened, there is a gap between the bottom of the adjusting piece 54 and the scale tube 55. Now, the operating handle 20 and the scale tube 55 can be operated to move along the scale tube 55 to be relatively close to or away from the bending handle 1064, thereby adjusting the distance of the distal head 32 of the sheath 30 relative to the distal end of the bendable sheath 1062. When the adjustment knob 57 is tightened, the bottom of the adjusting piece 54 abuts against the scale tube 55, and the operating handle 20 cannot move relative to the bending handle 1064, thereby fixing the position of the distal head 32 of the sheath 30 relative to the distal end of the bendable sheath 1062.

Taking the left ventricular wall as the target tissue region for example, the interventional injection system 1000A can inject the myocardial repair substance into the myocardial tissue of the left ventricular wall via a suitable interventional path, preferably via the femoral artery-aortic arch-aorta-left ventricular path. Taking this interventional path as an example, in surgery, the femoral artery is first punctured, the guiding sheath 1042 first establishes a passage to the left ventricle, then the bendable sheath 1062 is introduced into the left ventricle along the interior lumen of the guiding sheath 1042, and thereafter the bendable sheath 1062 is adjusted to a suitable injection location, thereby complete the establishment of the injection path. Then the operating handle 20 is operated as follows.

In step 1, the adjustment knob 57 is loosened, the entire operating handle 20 is slowly pushed forward to push the sheath 30 and the flexible injection member 40 forward, and under the guidance of DSA and ultrasound images, by determining the position of the head 32 of the sheath 30, it is determined whether the sheath 30 has contacted the surface of the left ventricular wall 206, for example, the endocardium 207 (which can be regarded as the surface of the target tissue region) in the left ventricle, as shown in FIG. 16.

In step 2, after determining that the head 32 has contacted the endocardium 207, the driving knob 242 is rotated in the first rotation direction (for example, clockwise) to make the injection needle 42 penetrate the endocardium 207 into the left ventricular wall 206. When the driving rod 244 reaches the preset limit position as mentioned above, the driving knob 242 cannot continue rotating, and thus the injection needle 42 cannot advance further, thereby limiting the injection depth within a safe range, as shown in FIGS. 17a and 17b.

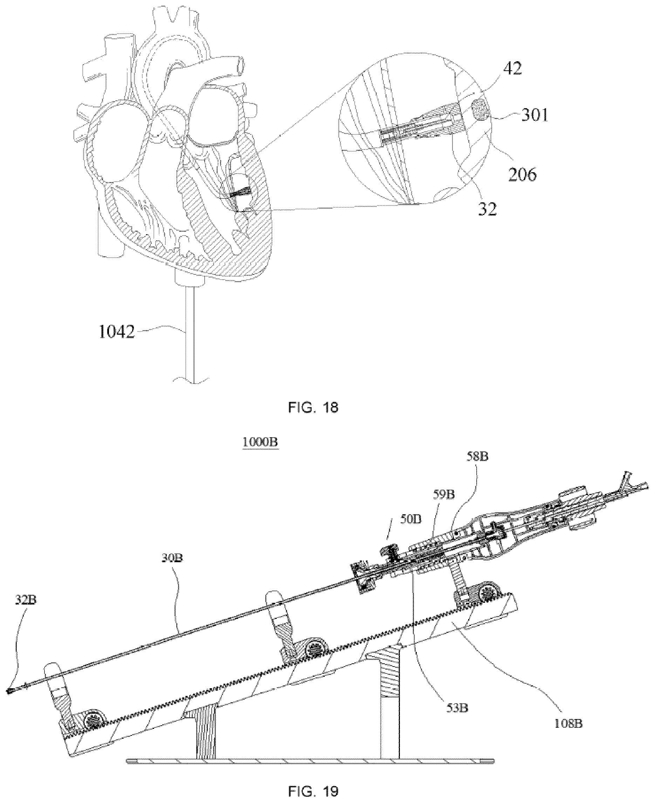

In step 3, after determining that the injection needle 42 has penetrated into the target injection site, the syringe can be used to inject the required first injectable substance 301 via the injection interface seat 27 into the left ventricular wall 206 through the first branch interface 2711 and the first channel 41. After the injection is finished, the injection needle 42 is withdrawn until it is separated from the left ventricular wall 206, as shown in FIG. 18. Also, a second injectable substance such as a contrast medium or other drugs can be injected through the second branch interface 2713, the second channel 43 and the injection orifices 322 on the distal head 32 of the sheath 30 into the left ventricle.

The above steps are repeated at a plurality of desired injection locations, and then the whole device is withdrawn, thereby completing the surgery.

The target tissue region can also be other myocardial tissues of the heart, such as the interventricular septum, the ventricular wall of the right ventricle, etc. Depending on different repair sites, corresponding interventional paths can be selected. For example, when the target tissue region is the ventricular wall of the right ventricle, then the interventional path can be the femoral vein-inferior vena cava-right atrium-right ventricular path.

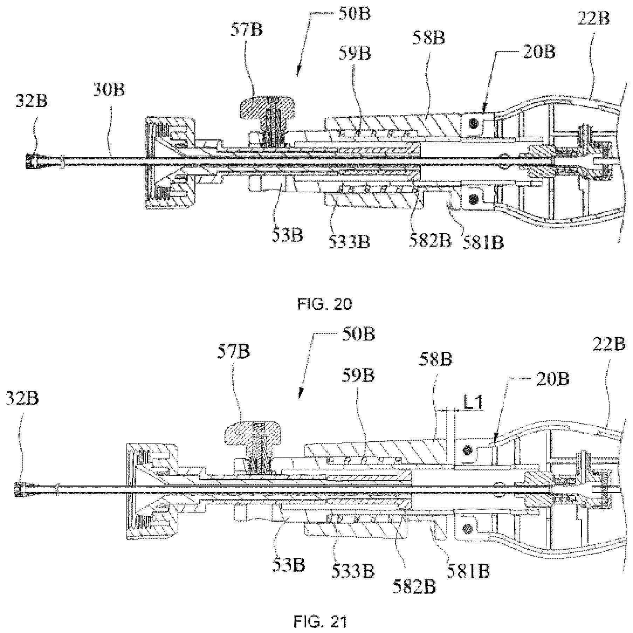

In other embodiments of the present disclosure, referring to FIG. 19, FIG. 20 and FIG. 21, in the interventional injection system 1000B, the adjusting device 50B further includes a sleeving tube 58B and a spring element 59B, to provide feedback to the operator when the head 32B of the sheath 30B contacts the target tissue region. That is, a feedback mechanism is provided, for prompting that the head 32B of the sheath 30B has contacted the target tissue region. This allows the operator to determine whether the head 32 has contacted the target tissue region without ultrasound images, simplifying the surgical process and improving the surgical efficiency.

The sleeving tube 58B is fixedly connected to the auxiliary support 108B. The sleeving tube 58B has a clamping position 581 B where the corresponding clamping member on the auxiliary support 108B can clamp and fix the sleeving tube 58B, thereby fixedly connecting the sleeving tube 58B on the auxiliary support 108B. In other embodiments of the present disclosure, the sleeving tube 58B and the auxiliary support 108B are not necessarily connected by clamping. The sleeving tube 58B is movably arranged around the middle piece 53B. The outer wall of the middle piece 53B is provided with a first abutment portion 533B, and the inner wall of the sleeving tube 58B is provided with a second abutment portion 582B. The first abutment portion 533B includes, but is not limited to, a radial step on the middle piece 53B, and the second abutment portion 582B includes, but is not limited to, a radial step on the inner wall of the sleeving tube 58B.

The spring element 59B is arranged around the middle piece 53B and received in the sleeving tube 58B. In the initial state, the proximal end of the sleeving tube 58B abuts the distal end of the housing 22B. The spring element 59B is disposed between the middle piece 53B and the sleeving tube 58B. One end of the spring element 59B abuts the first abutment portion 533B, and the other end of the spring element 59B abuts the second abutment portion 582B.

When the distal head 32B of the sheath 30B contacts the target tissue region, if further force is applied to push the sleeving tube 58B distally, the spring element 59B will be pressed and deformed by the sleeving tube 58B and the middle piece 53B, and the head 32B, the middle piece 53B and the housing 22B are relatively fixed, while only the sleeving tube 58B moves distally. That is, the middle piece 53B moves proximally relative to the sleeving tube 58B, and a gap is formed between the proximal end of the sleeving tube 58B and the distal end of the housing 22B. Specifically, after adjusting the bendable sheath to an appropriate position, it is necessary to loosen the adjustment knob 57B and push the entire operating handle 20B to move forward (this pushing step is completed by directly applying force on the sleeving tube 58B through the auxiliary support 108B), so that the head 32B of the sheath 30B extends out of the distal end of the bendable sheath. When the sheath 30B moves until the head 32B contacts the target tissue region (for example, the ventricular wall), the head 32B cannot continue advancing forward, and the target tissue region exert a backward reaction force to the head 32B, which force is transmitted to the operating handle 20B and the middle piece 53B fixedly connected to the operating handle 20B through the sheath 30B. If the operator continues pushing the sleeving tube 58B forward, the reaction force from the head 32B will cause the first abutment portion 533B and the second abutment portion 582B to move toward each other, that is, the spring element 59B will be compressed by the force, and the middle piece 53B and the operating handle 20B will move proximally relative to the sleeving tube 58B fixed by the clamping member, thereby separating the sleeving tube 58B from the distal end of the housing 22B of the operating handle 20B with a gap L1 formed therebetween, prompting that the head 32B has reached the target tissue region and a further needle advance operation can be performed.

In the interventional injection system of the present disclosure, the distal head has a multi-channel structure (a sliding member for auxiliary indication can be used), combined with the feedback mechanism on the operating handle, the process and effect of the head contacting tissue can be determined timely and accurately, and the fluid type can be switched timely as needed. In a specific case of multi-point injection, the orientation and position of the injection needle can be changed quickly by the rotation of the entire guide handle and the entire bending handle as well as the bendable characteristic, avoiding or alleviating repeated axial adjustment of the tubes, greatly shortening the injection time and improving the surgical efficiency.

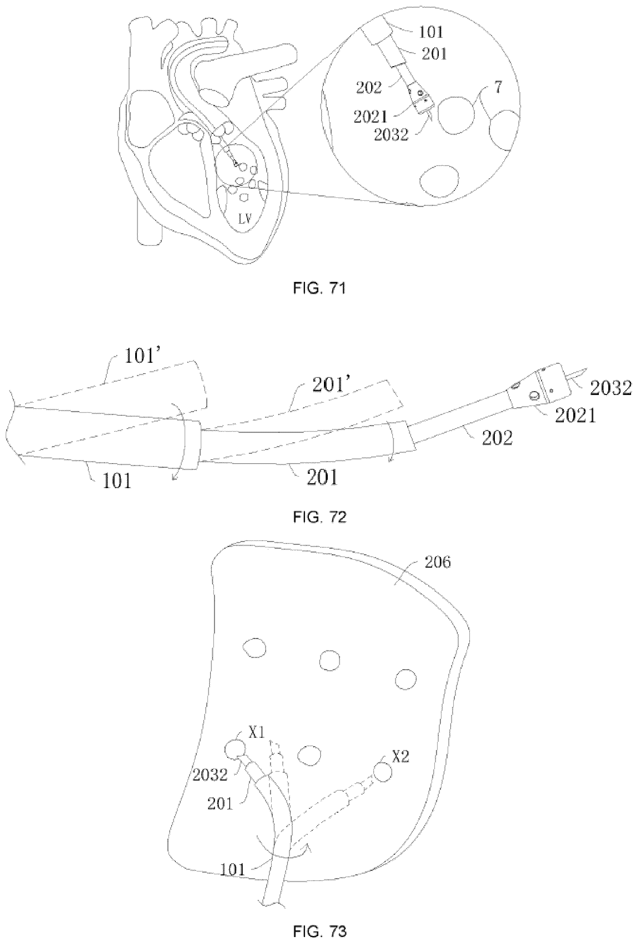

Another embodiment referring to FIGS. 73 to 75 below illustrates an operation method of the interventional injection system taking injection locations in a multiple-row-and-multiple-column arrangement as an example, i.e., a position adjustment method of the injection needle for continuous injections. The arrangement of injection locations in the figures is only an illustration, and can be adjusted in practice according to the area and location of the lesion.

In the treatment of some heart diseases, such as myocardial obstruction, drugs need to be injected into the endocardium. During specific treatment, the guiding sheath for providing an interventional passage is introduced into the left ventricle through puncture via the femoral artery. The imaging system performs three-dimensional mapping of the heart tissue and obtains relevant images. The operator can judge whether the needle outlet contacts the target puncture point based on the images and determine whether to advance the needle. When the needle is advanced, the injection needle punctures the endocardium and injects drugs into the myocardial tissue.

However, due to the anatomical configuration of the heart tissue, there may be errors in the images generated by the imaging system. The operator cannot quickly judge from the images whether the needle outlet is in contact with the puncture point. Therefore, the operator needs to spend time to observe the images carefully before advancing the needle to determine whether to advance the needle, resulting in a time-consuming surgery.

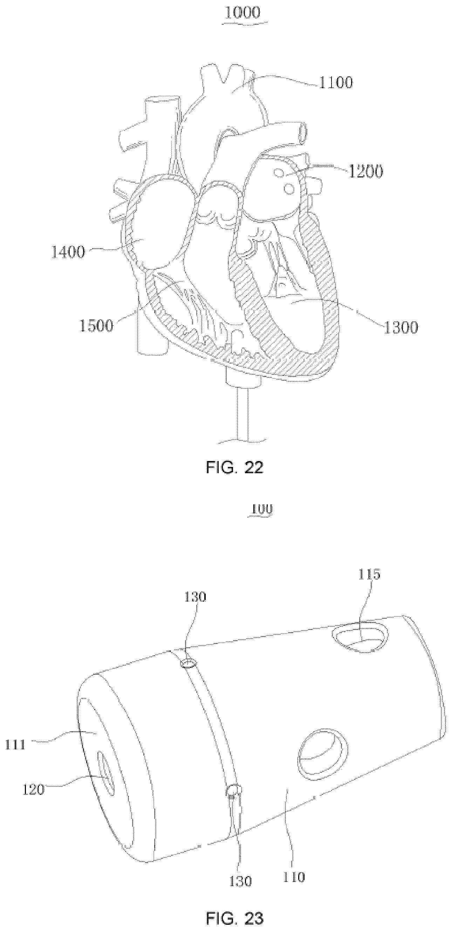

FIG. 22 is a schematic view of a heart 1000, which mainly includes an aortic arch 1100, a left atrium 1200, a left ventricle 1300, a right atrium 1400 and a right ventricle 1500.

Referring to FIGS. 23 to 24, an embodiment of the present disclosure discloses a sleeve 100, which can be used with an interventional medical device to treat various diseases of the heart 1000, for example, to inject hydrogel for treatment of myocardial obstruction, or for ablation in hypertrophic cardiomyopathy or the like. With the three-dimensional mapping system, it is possible to determine whether the sleeve 100 is in contact with the tissue wall by injecting fluid into the sleeve 100 in this embodiment and based on the fluid outflow, and accordingly determine whether to advance the needle.

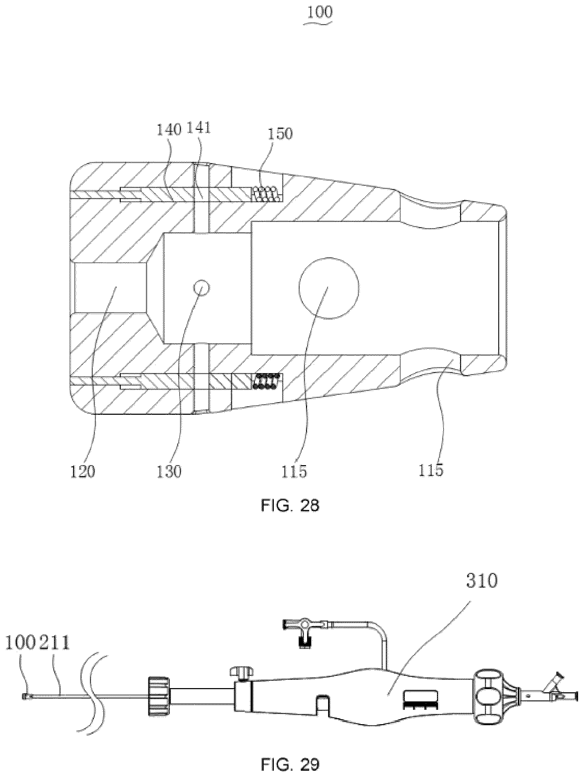

Referring to FIGS. 29 to 31, in use, the sleeve 100 in this embodiment is connected to the catheter 211, and introduced into the left ventricle 1300 through the catheter 211 via the aortic arch 1100. When the needle can be advanced for treatment, the needle body 221 is pushed through the catheter 211 and the sleeve 100 to puncture the endocardium to treat the myocardial tissue.

Referring to the above embodiment (for example, as shown in FIG. 13), the sleeve 100 in this embodiment can be considered a further development of the head 32, the catheter 211 connected to the sleeve 100 is equivalent to the sheath 30 connected to the head 32, and the fifth channel 130 is equivalent to the injection orifice 322.

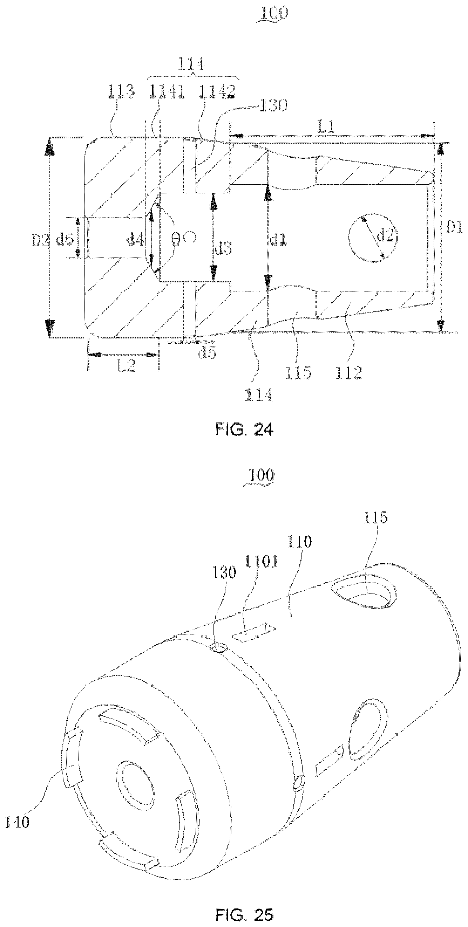

Referring to FIGS. 23 and 24, a sleeve 100 according to a first embodiment of the present disclosure includes a main body 110. The main body 110 defines a fourth channel 120 that passes through the main body 110 in the axial direction, the side wall of the main body 110 defines at least one fifth channel 130, and the fourth channel 120 is communicated with the at least one fifth channel 130.

Specifically, based on the above arrangement, after injecting fluid into the proximal end of the sleeve 100, fluid can flow out through both the fourth channel 120 and the fifth channel 130. When the distal end of the fourth channel 120 is blocked, fluid can only flow out through the fifth channel 130. Therefore, when the distal end surface of the main body 110 abuts against the tissue wall of the myocardial tissue, the distal end of the fourth channel 120 is blocked, and fluid can only flow out through the fifth channel 130. Therefore, when the sleeve 100 is arranged around the end of the injection assembly or the ablation assembly, it can be determined if the distal surface of the main body 110 is in close contact, or in partial contact, or not in contact with the tissue wall of the myocardium tissue by infusing fluid into the proximal end of the sleeve 100, depending on whether fluid flows out of the fourth channel 120. If it is determined that the distal surface of the main body 110 is in close contact with the tissue wall of the myocardium tissue, the needle can be advanced for treatment. Otherwise, the distal surface of the sleeve 100 needs to be adjusted.

The sleeve 100 in this embodiment can be made of metal or non-metal material. The metal material can be, for example, 304 stainless steel, 316 stainless steel, nickel-titanium alloy, etc., and the non-metal material can be, for example, polycarbonate (PC). In a specific embodiment of the present disclosure, the sleeve 100 is preferably made of 316 stainless steel.

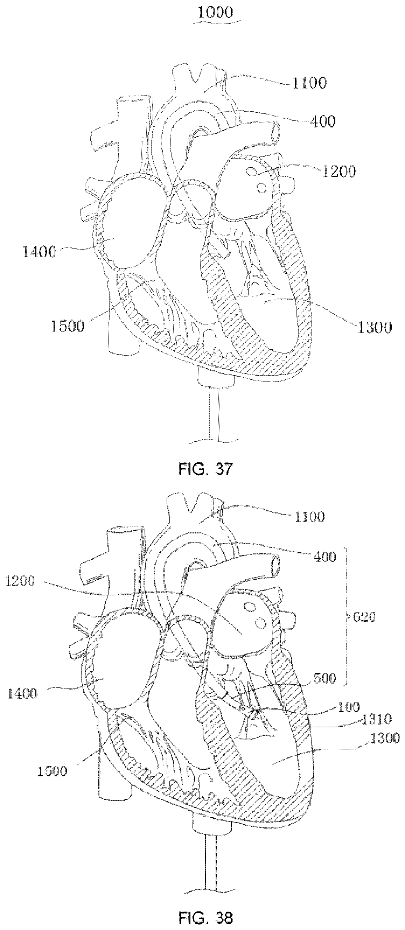

Referring to FIGS. 23 and 38, in this embodiment, the distal section of the main body 110 has an abutment surface 111 for abutting against the tissue wall 1310 of the myocardial tissue. Specifically, in order to ensure that the abutment surface 111 can closely contact the tissue wall 1310 of the myocardial tissue, the abutment surface 111 in this embodiment is a smooth plane. After the smooth abutment surface 111 abuts against the tissue wall 1310, it can be adjusted to closely contact the tissue wall 1310 of the myocardial tissue under the action of external force, and damage to the tissue wall can also be avoided.

Referring to FIG. 24, in some embodiments, the main body 110 includes a proximal section 112, a middle section 114, and a distal section 113 connected in sequence. The parts of the fourth channel 120 corresponding to the proximal section 112, the middle section 114, and the distal section 113 have different radial dimensions, so that fluid can be converged from the position with a large radial dimension to the position with a small radial dimension, to facilitate outflow through the fourth channel 120 or the fifth channel 130.

Referring to FIGS. 23 and 29, in this embodiment, the proximal section 112 is used to connect to the catheter 211. When connecting the proximal section 112 and the catheter 211, the catheter 211 can be arranged around the proximal section 112, so that the outer wall of the proximal section 112 is connected to the inner wall of the catheter 211, or the proximal section 112 can be arranged around the catheter 211 so that the outer wall of the catheter 211 is connected to the inner wall of the proximal section 112.

Referring to FIG. 24, in the case where the proximal section 112 is arranged around the catheter 211 and the outer wall of the catheter 211 is connected to the inner wall of the proximal section 112, the outer diameter D1 of the proximal section 112 gradually increases from the proximal end to the distal end and the radial dimension d1 of the part of the fourth channel 120 corresponding to the proximal section 112 is a constant value or gradually decreases. In the case where the proximal section 112 surrounds the distal end of the catheter 211, by gradually increasing the outer diameter D1 of the proximal section 112 from the proximal end to the distal end, the height of the transition step between the sleeve 100 with the gradually decreasing outer diameter and the catheters 211 is reduced, so the sleeve 100 can transition to the catheters 211 as smooth as possible, preventing blood flow from impacting the step surface to form thrombus during the surgery, and reducing damage to the human body.

In other embodiments, the outer diameter D1 of the proximal section 112 can be constant, so that the outer diameter of the entire sleeve 100 is consistent, facilitating the manufacture of the sleeve, without additional setting of the outer diameter of this section, reducing manufacture difficulty. In this case, when connecting the sleeve 100 with the catheter 211, the catheter 211 can be arranged around the proximal section 112.

In the case where the radial dimension d1 of the part of the fourth channel 120 corresponding to the proximal section 112 is a constant value, the proximal section 112 can be conveniently connected to the catheter 211 when arranging the proximal section 112 around the outer wall of the distal end of the catheter 211 which has a constant outer diameter. In the case where the radial dimension d1 of the part of the fourth channel 120 corresponding to the proximal section 112 gradually decreases, the proximal section 112 and the catheter 211 have an interference fit when arranging the proximal section 112 around the outer wall of the distal end of the catheter 211 for connection, increasing the connection strength between the two.

Specifically, in this embodiment, the catheter 211 is connected with the proximal section 112 corresponding to a constant radial dimension d1. In this case, an assembly clearance t for filling glue is reserved between the side wall of the part of the fourth channel 120 corresponding to the proximal section 112 and the outer wall of the catheter 211, so that the proximal section 112 and the outer wall of the catheter 211 can be bonded together. The assembly clearance t ranges from 0.1 mm to 0.2 mm, and the radial dimension d1 ranges from 2.0 mm to 3.0 mm.

In some embodiments, the assembly clearance t = 0.125 mm and the radial dimension d1 = 2.65 mm, to adapt to the size of the catheter 211, facilitating the assembly of the catheter 211, and allowing an appropriate amount of glue to be filled.

In the case where the side wall of the fourth channel 120 and the outer wall of the catheter 211 are bonded by glue, in order to facilitate the connection between the two, a glue injection hole 115 is opened in the proximal section 112 in this embodiment, so that glue can be injected into the clearance between the sleeve 100 and the catheter 211 through the glue injection hole 115. The quantity of the glue injection hole 115 defined on the proximal section 112 can be one or more, and the glue injection hole 115 can be a circular hole, a square hole, or other polygonal holes.

Specifically, a plurality of glue injection holes 115 are opened in the side wall of the proximal section 112 in this embodiment, so that glue can be injected from the plurality of glue injection holes 115, improving the assembly efficiency of the sleeve 100 and the catheter 211. Further, through the plurality of glue injection holes 115, glue can flow into a more uniform distribution, ensuring the connection strength. In this embodiment, four glue injection holes 115 are provided, which are arranged in two pairs opposite to each other in the proximal section 112, and the two pairs of glue injection holes 115 are offset from each other, so that the glue can flow throughout the assembly clearance t, to improve the tightness of the connection between the sleeve 100 and the catheter 211.

Specifically, the glue injection hole 115 in this embodiment is a circular hole, to facilitate processing. The diameter d2 of the glue injection hole 115 is 1.2 mm, facilitating the injection of glue. The length L1 of the proximal section 112 ranges from 3.0 mm to 5.0 mm, ensuring a sufficient connection length with the catheter 211, thereby ensuring the stability of the connection. Preferably, the length L1 of the proximal section 112 in this embodiment is 5.0 mm.

The transition section between the proximal section 112 to the distal section 113 is the middle section 114. The middle section 114 is provided with a fifth channel 130. The corresponding radial dimension d3 of the part of the middle section 114 close to the proximal section 112 is larger than the corresponding radial dimension d4 of the part of the middle section 114 close to the distal section 113. The proximal section 112 is configured to connect with the catheter 211, and fluid can flow through the interior lumen of the catheter 211 and into the sleeve 100. When fluid flows from the part of the fourth channel 120 corresponding to the proximal section 112 into the part of the fourth channel 120 corresponding to the distal section 113, the channel with a reduced inner diameter can converge the fluid.

Specifically, the middle section 114 in this embodiment includes two parts corresponding to the fourth channel 120: a first part 1141 close to the proximal section 112 and a second part 1142 close to the distal section. The radial dimension of the fourth channel 120 corresponding to the first part 1141 is d3, the radial dimension of the fourth channel 120 corresponding to the second part 1142 is d4, and the radial dimension d3 is larger than the radial dimension d4, so that the fluid flowing from the proximal section 112 to the distal section 113 can be converged.

In some embodiments, radial dimension d1 > radial dimension d3 > radial dimension d4, so that the size of the part of the fourth channel 120 corresponding to the middle section changes from large to small, and fluid gradually flows toward the part of the fourth channel 120 with a smaller radial dimension and flows out from the fourth channel 120 or the fifth channel 130 after being converged.

The radial dimension d3 in this embodiment ranges from 1.8 mm to 2.5 mm. After assembly with the catheter 211, the radial dimension d3 > the inner diameter of the catheter 211. In other embodiments, after assembly with the catheter 211, the radial dimension d2 can be less than or equal to the inner diameter of the catheter 211. In a specific embodiment of the disclosure, d3 = 2.2 mm.

The radial dimension d4 in this embodiment gradually decreases toward the distal section 113, so that the side wall of the part of the fourth channel 120 corresponding to the second part 1142 is tapered, so as to converge the fluid flowing out from the catheter 211 into the part of the fourth channel 120 corresponding to the distal section 113 through the second part 1142.

The cross-sectional shape of the fifth channel 130 in this embodiment can be circular, square or other geometric shapes. In this embodiment, the cross-sectional shape of the fifth channel 130 is circular, facilitating processing and manufacture. The radial dimension d5 of the fifth channel 130 ranges from 0.1 mm to 0.5 mm. In one embodiment of the present disclosure, d5 = 0.3 mm, ensuring that fluid can flow out of the fifth channel 130 smoothly.

In this embodiment, the radial dimension d6 corresponding to the distal section 113 is a constant value, or the radial dimension corresponding to the part of the distal section 113 close to the middle section 114 is larger than the radial dimension corresponding to the part of the distal section 113 away from the middle section 114 so as to converge the fluid. In the case where the radial dimension corresponding to the part of the distal section 113 close to the middle section 114 is larger than the radial dimension corresponding to the part of the distal section 113 away from the middle section 114, the corresponding radial dimension d6 of the distal section 113 can be graded, that is, the part of the fourth channel 120 corresponding to the distal section 113 can include multiple sections. It should be noted that the radial dimension d6 of the part of the fourth channel 120 corresponding to the distal section 113 should be large enough to ensure the free movement of the needle body when the needle is pushed out from the part of the fourth channel 120 corresponding to the distal section 113.

In some embodiments, the radial dimension d6 corresponding to the distal section 113 is a constant value, facilitating the processing and manufacture of the sleeve 100. The radial dimension d6 of the fourth channel 120 corresponding to the distal section 113 in this embodiment is larger than the outer diameter of the needle body, and the radial dimension d6 ranges from 0.5 mm to 1.5 mm, and more preferably d6 = 1.0 mm.

The corresponding outer diameter of the distal section 113 is D2. The larger the outer diameter D2, the larger the area of the abutment surface 111 and the larger the contact area with the target tissue wall 1310, and thus the more stable the abutment will be. However, it should be noted that as the outer diameter D2 increases, the flexibility decreases, making the operation inconvenient. The outer diameter D2 in this embodiment ranges from 3.0 mm to 6.0 mm. In a specific embodiment of the present disclosure, the outer diameter D2 = 5.0 mm.

In some embodiments, the cone angle of the part of the fourth channel 120 corresponding to the second part 1142 of the middle section 114 is θ, and the sum of the lengths of the distal section 113 and the second part 1142 of the sleeve 100 is L2. The cone angle θ is inversely proportional to the length L2. The larger θ is, the shorter L2 is. The smaller θ is, the longer L2 is. Further, the length L2 is inversely proportional to the flexibility of the catheter 211. The longer L2 is, the lower the flexibility of the catheter 211 is. The shorter L2 is, the higher the flexibility of the catheter 211 is. Accordingly, the θ in this embodiment ranges from 90° to 140°, and L2 ranges from 1.5 mm to 2.0 mm. In a specific embodiment of the present disclosure, θ = 120°, and L2 = 1.85 mm.

Referring to FIGS. 25 to 28, in some embodiments, another sleeve 100 is provided. The differences between the sleeve 100 in this embodiment and the sleeve 100 in the above embodiment are as follow.

The sleeve 100 in this embodiment further includes at least one sliding member 140, at least one spring element 150, and at least one sixth channel 160.

The sixth channel 160 is communicated to the fifth channel 130, and the distal surface of the main body 110 is the abutment surface 111. The distal end of the sixth channel 160 has an opening 161 on the abutment surface 111, and the proximal end of the sixth channel 160 is closed or provided with a blocking member. The sliding member 140 and the spring element 150 are arranged in the sixth channel 160. The distal end of the sliding member 140 protrudes from the abutment surface 111 through the opening 161, and the proximal end of the sliding member 140 is connected to the spring element 150. The proximal end of the spring element 150 abuts the proximal end of the sixth channel 160. Since the sleeve 100 is provided with the sliding member 140, the spring element 150 and the sixth channel 160, the fifth channel 130 can be closed or opened by the sliding member 140. After fluid is injected into the proximal end of the sleeve 100, if the abutment surface 111 of the sleeve 100 is in partial contact or not in contact with the tissue wall, the fourth channel 120 is opened but the fifth channel 130 is not opened, and fluid will only flow out from the fourth channel 120; if the abutment surface 111 of the sleeve 100 is in close contact with the tissue wall, the fourth channel 120 is not opened and the fifth channel 130 is opened, and fluid will only flow out from the fifth channel 130.

The outer wall of the main body 110 has a balance opening 1101 communicated with the proximal side of the sixth channel 160. When the sliding member 140 moves, the build-up fluid pressure on the proximal side of the sixth channel 160 can be relieved through the balance opening, to prevent the movement of the sliding member 140 from being resisted, maintaining pressure balance. In order to facilitate the assembly of the spring element 150, the main body 110 can be formed by separate pieces that are mutually snap-fastenable in the axial or radial direction.

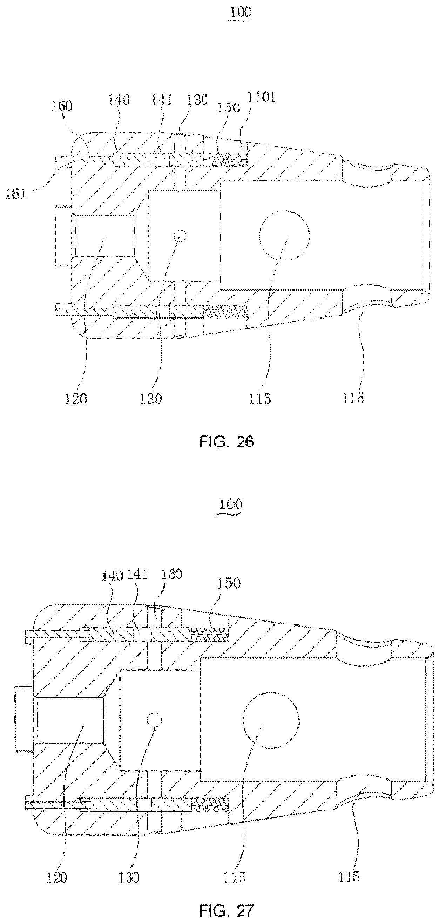

Specifically, referring to FIG. 28, the sliding member 140 defines a through hole 141. When the spring element 150 is in a first state, the sliding member 140 is in a first position, and the through hole 141 is communicated with the fifth channel 130. Referring to FIG. 26, when the spring element 150 is in a second state, the sliding member 140 is in a second position, and the sliding member 140 blocks the fifth channel 130. It can be understood that, in this embodiment, when the distal end of the sliding member 140 protrudes from the abutment surface 111 through the opening 161, the spring element 150 is in the second state, that is, in the natural state, the sliding member 140 blocks the fifth channel 130, fluid can only flow out from the fourth channel 120, and thus it can be determined that the abutment surface 111 of the sleeve 100 is not in contact with the tissue wall 1310. When the sliding member 140 compresses the spring element 150 so that the spring element 150 is in the first state, the sliding member 140 is in the first position, and the through hole 141 is communicated with the fifth channel 130, fluid can only flow out from the fifth channel 130 and the through hole 141, and thus it can be determined that the abutment surface 111 of the sleeve 100 is in close contact with the tissue wall 1310.

In addition, compared with the sleeve 100 mentioned above, in use of the sleeve 100 provided with the sliding member 140, the spring element 150 and the sixth channel 160 in this embodiment, when the abutment surface 111 of the sleeve 100 is in partial contact with the tissue wall 1310, the through hole 141 in the sliding member 140 can partially overlap with the fifth channel 130 so that both the fourth channel 120 and the fifth channel 130 are opened (see FIG. 27). As the fifth channel 130 is narrowed, the outflow of fluid per unit time can be reduced, so the consumption of the contrast medium can be reduced, especially in the case of multiple injection locations.

At least one sliding member 140 can be provided. It can be understood that since the abutment surface 111 of the sleeve 100 needs to abut against the tissue wall 1310, the abutment surface 111 has a certain area. In order to make the determination of whether the abutment surface111 is in close contact with the tissue wall more accurately, in this embodiment, it is preferred to provide four sliding members 140, and the four sliding members 140 are evenly arranged on the sleeve 100.

Since the sleeve 100 needs to transmit electrical signals to the mapping system, at least one electrode can be provided on the side wall of the main body 110 in this embodiment. The electrode is coupled to the mapping system through a conductive wire, and the mapping system applies excitation signals of different frequencies to the electrode located on the sleeve 100 through the conductive wire, and captures the electric field signals or magnetic field signals generated by the electrode to achieve the purpose of three-dimensional mapping, thereby observing the condition of the sleeve 100.

In another embodiment, the sleeve main body 110 can be made of metal material, so that the main body 110 can be directly coupled to the conductive wire. That is, the electrical signal can be transmitted to the main body 110 through the mapping system.

When the sleeve 100 above is used for treatment of the heart 1000, the guiding sheath 400 for providing an interventional passage is introduced into the left ventricle 1300 through puncture via the femoral artery. The catheter 211 pushes the sleeve 100 through the interior lumen of the guiding sheath 400 into the left ventricle 1300. When fluid is injected into the catheter 211, the fluid flows into the sleeve 100. Based on the outflow of the fluid in the fourth channel 120 and/or the fifth channel 130, it can be determined whether the sleeve 100 is in close contact with the tissue wall 1310, and accordingly it can be determined whether to advance the needle. If the fluid only flows out from the fifth channel 130, it can be determined that the abutment surface 111 is in close contact with the tissue wall 1310, and the fourth channel 120 is blocked, so the needle can be advanced for treatment. Otherwise, further adjustment is needed.

Referring to FIGS. 29 to 34, in some embodiments, an injection device 200 is disclosed, including a delivery assembly 210 and an injection assembly 220.

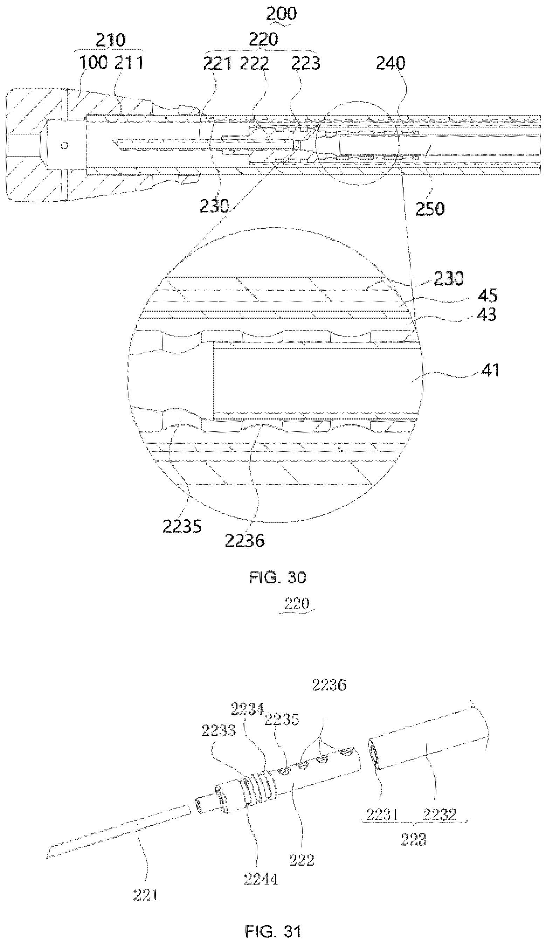

Referring to FIGS. 29 and 30, the delivery assembly 210 includes a catheter 211 and the sleeve 100 mentioned above. The proximal end of the sleeve 100 is connected to the distal end of the catheter 211. The injection assembly 220 is movably received in the catheter 211, for injecting liquid medicine into the lesion tissue.

The above-mentioned injection device 200 is also provided with the sleeve 100 at the distal end of the catheter 211. The sleeve 100 can be the sleeve according to any of the above embodiments. When injecting fluid into the sleeve 100, depending on the outflow of the fluid in the fourth channel 120 or the fifth channel 130 in the sleeve 100, it can be determined whether the sleeve 100 is in close contact with the tissue wall 1310 and whether the injection assembly 220 can advance the needle to inject the liquid medicine for treatment of the myocardial tissue. As long as the fluid does not flow out from the fourth channel 120, it can be determined that the abutment surface 111 is in contact with the tissue wall 1310, the fourth channel 120 is blocked by the tissue wall, and the needle can be advanced for treatment.

In the surgery, it is necessary to input different fluids into the sleeve 100 in order. For example, the contrast fluid is first injected for injection location selection, and then the liquid medicine is injected through the injection assembly 220. In order to facilitate the operation, the catheter 211 and the injection assembly 220 in this embodiment define different channels to inject different fluids.

Specifically, referring to FIG. 30, a gap is defined between the catheter 211 and the injection assembly 220. The gap is configured as a first fluid channel 240. The injection assembly 220 defines a second fluid channel 250 therein. Fluid can flow into the first fluid channel 240 and the second fluid channel 250 respectively. For example, a contrast medium can be injected through the first fluid channel 240 for injection location selection, and then a contrast medium can be injected through the second fluid channel 250 to determine whether the treatment conditions are met. When it is determined that the treatment conditions are met, the liquid medicine can be injected through the second fluid channel 250 for treatment of the myocardial tissue.

It can be understood that since the mapping system needs to apply electrical signals to the sleeve 100, the mapping system needs to be electrically coupled to the sleeve 100, so a conductive wire 230 is connected between the two. The distal end of the conductive wire 230 is connected to the electrode or the main body 110, and the proximal end of the conductive wire 230 is connected to the mapping system. Therefore, the signals from the sleeve 100 can be received by the mapping system through the conductive wire 230 connecting between the sleeve 100 and the mapping system.

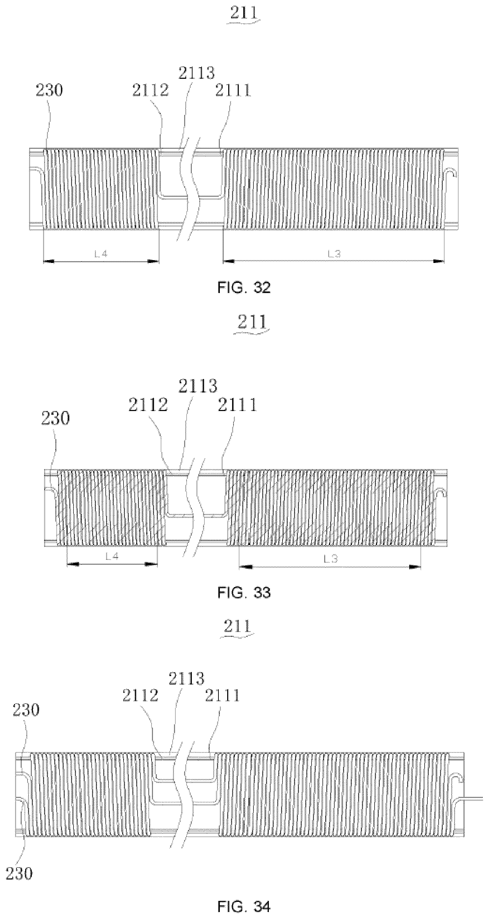

Referring to FIGS. 32 to 34, the conductive wire 230 in this embodiment is buried in the wall of the catheter 211. Specifically, the catheter 211 includes from the inside to the outside: an inner film 2111, a metal braided mesh layer 2112 surrounding the inner film 2111, and a polymer layer 2113 surrounding the metal braided mesh layer 2112. The conductive wire 230 is wound around the metal braided mesh layer 2112. The metal braided mesh layer 2112 is made of stainless steel, and the polymer layer 2113 can be made of PEBAX.

The conductive wire 230 can be made of metal wires such as gold wire, silver wire, copper wire, etc. Copper wire has small resistance, but has poor strength. Silver wire and gold wire have smaller resistance, but has high density and thus heavy weight. Therefore, copper wire is preferably used as the conductive wire in this embodiment.

Specifically, when arranging the copper wire in the catheter 211, the copper wire covers the outer surface of the stainless steel braided mesh layer. In order to prevent the copper wire from directly contacting the metal braided mesh layer 2112 to form electrical conduction, the surface of the copper wire is covered with an insulating material.

The distal end of the copper wire is connected to the sleeve 100, and the proximal end thereof is connected to the mapping system. In order to ensure the conductivity, in this embodiment, both the distal and proximal sections of the copper wire of the conductive wire 230 are wound into coils, and the outer insulation layer and the polymer layer 2113 of the distal and proximal sections of the catheter 211 are scraped off, to improve the conductivity.

Referring to FIG. 33, the catheter 211 needs to be bendable so that it can detect the injection location normally for surgery. For example, when the distal end of the catheter 211 together with the sleeve 100 is introduced into the heart, the catheter 211 needs to be bent so that the abutment surface 111 of the sleeve 100 can abut against the tissue wall 1310. The flexibility of the distal and proximal sections of the catheter 211 is inversely proportional to the number of wound coils of the conductive wire 230. The more wound coils of the conductive wire 230, the higher the hardness, the lower the flexibility of the section of the catheter 211 with the coils. The less wound coils of the conductive wire 230, the lower the hardness, the higher the flexibility of the section of the catheter 211 with the coils. Therefore, in this embodiment, the first length L3 of the exposed proximal section of the conductive wire 230 is preferably in the range of 4.0 mm to 8.0 mm, more preferably 6.0 mm. The first length L4 of the exposed distal section of the conductive wire 230 is preferably in the range of 1.0 mm to 2.0 mm, more preferably 1.5 mm.

The quantity of the conductive wire 230 in this embodiment can be one, two or more. In order to ensure mapping accuracy, the quantity of the conductive wire 230 is two in this embodiment (see FIG. 34). The outer diameter of the conductive wire 230 is preferably in the range of 0.05 mm to 0.2 mm, and more preferably 0.1 m. Since the larger the outer diameter of the wire, the larger the outer diameter of the catheter, resulting in reduced flexibility, bending resistance and bendability of the catheter, the outer diameter of the conductive wire 230 is in the range of 0.05 mm and 0.2 mm, ensuring the flexibility of the catheter 211.

Referring to FIGS. 30 and 31, the injection assembly 220 includes a needle body 221, a needle connector 222 and a needle connection tube 223. The needle connector 222 is used to connect the needle body 221 and the needle connection tube 223, so that the needle body 221 and the needle connection tube 223 can be connected in one piece. Therefore, the liquid medicine such as hydrogel can be supplied to the needle body 221 through the needle connection tube 223.

Specifically, referring to FIG. 30, the needle connection tube 223 includes an inner tube 2231 and an outer tube 2232. There is a gap between the inner tube 2231 and the outer tube 2232. The proximal end of the needle connector 222 is arranged around the inner tube 2231, the outer tube 2232 is arranged around the distal end of the needle connector 222, and the distal end of the needle connector 222 is arranged around the needle body 221, thereby connecting the three into one piece.

The interior of the inner tube 2231 can serve as a fluid channel (equivalent to the first channel in the embodiment as shown in FIG. 10b and FIG. 13), and the gap between the inner tube 2231 and the outer tube 2232 can serve as a fluid channel (equivalent to the second channel 43 mentioned above). It can be understood that the second fluid channel 250 inside the injection assembly 220 includes the first channel 41 and the second channel 43.

Taking the first fluid channel 240 (equivalent to the third channel 45 mentioned above) between the catheter 211 and the injection assembly 220 into account, that is, the catheter 211 and the injection assembly 220 define three fluid channels (the first channel 41, the second channel 43, and the third channel 45), through which different fluids can be delivered as needed. The distal end of the third channel 45 is communicated with the fourth channel 120. The first channel 41 and the second channel 43 meet through the injection needle 42 and can be communicated with the fourth channel 120 through the distal outlet of the injection needle 42.

As can be seen in FIGS. 30 and 31, the side wall of the needle connector 222 has a communication hole 2235. The fluid between the inner tube 2231 and the outer tube 2232 in the radial direction flows into the interior of the inner tube 2231 through the communication hole 2235 after reaching the distal end. That is, the first channel 41 and the second channel 43 are communicated to the distal section of the injection assembly 220, and the fluid is output through the tip of the needle body 221. It can be seen from the figure that in addition to the communication hole 2235, the side wall of the needle connector 222 has a connection hole 2236. Through the connection hole 2236, glue dispensing or spot welding can be performed to strengthen the connection of the needle connector 222 with the inner tube 2231.

In order to enhance the connection stability of the distal end of the needle connection tube 223 and the needle connector 222, the needle connection tube 223 is provided with a plurality of spaced protrusions 2233 on the outer wall of the distal section thereof. The needle connection tube 223 and the needle connector 222 are heat-fused together through the protrusions 2233, and the grooves 2234 between the protrusions 2233 are filled with materials after heat-fusing, thereby increasing the connection force between the needle connection tube 223 and the needle connector 222 and increasing the connection stability.

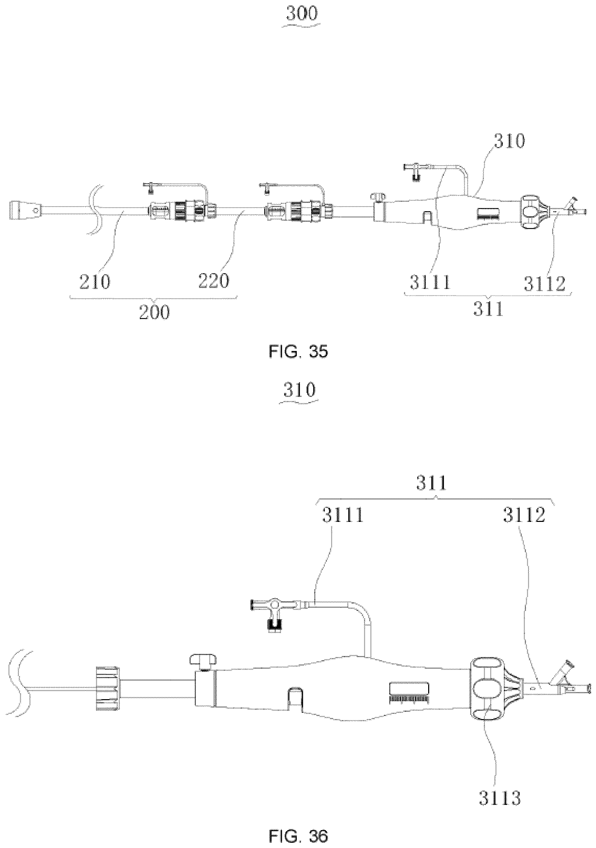

Referring to FIGS. 35 and 36, in some embodiments, an injection system 300 is disclosed, including a handle 310 and the above-mentioned injection device 200.

The handle 310 is connected to the proximal end of the catheter 211. The handle 310 is provided with at least one injection part 311. The injection part 311, the catheter 211, and the injection assembly 220 are communicated with each other, so that fluid can be injected through the injection part 311 of the handle 310, and flow through the catheter 211 and the sleeve 100 sequentially and then flow out through the fourth channel 120 and/or the fifth channel 130 of the sleeve 100, or fluid can flow through the injection device 200 via the injection part 311. Depending on the monitoring results of the mapping system, when the treatment conditions are met, the liquid medicine can be injected into the target tissue through the needle body 221 of the injection assembly 220.

Specifically, referring to FIGS. 35 and 36, in order to inject different fluids into the catheter 211 and the sleeve 100 for different surgical procedures, the injection part 311 in this embodiment includes a first injection part 3111 and a second injection part 3112. The first injection part 3111 and the second injection part 3112 correspond to different injection channels in the catheter 211 and/or the handle 310, so that different liquid medicines can be injected through the first injection part 3111 and the second injection part 3112 for surgery.

Specifically, for example, the first injection part 3111 is communicated to the first fluid channel 240, and the second injection part 3112 is provided in the axial direction of the handle 310 and communicated to the second fluid channel 250. During the treatment of myocardial obstruction, a contrast medium is first injected into the first injection part 3111, and it is determined whether to advance the needle according to the outflow direction of the contrast medium in the sleeve 100. After the needle is pushed out, a contrast medium is first injected into the second injection part 3112 to determine whether the sleeve 100 is in contact with the correct tissue wall, and if the sleeve 100 is positioned at the correct position, hydrogel or other liquid medicines can be injected through the second injection part 3112 and delivered to the target tissue.

It can be seen from the figure that the second injection part 3112 has a three-way structure, with two fluid input ports at the proximal end, which can be communicated to the first channel and the second channel of the second fluid channel 250 respectively, and accordingly contrast medium, hydrogel, or other liquid medicines can be switched as needed.

Referring to FIGS. 37 to 45, the method of using the injection system 300 in this embodiment for hydrogel injection includes the following steps.

In step S11, as shown in FIG. 37, a standard puncture via the femoral artery is performed, and the guiding sheath 400 is introduced into the human body via the femoral artery puncture and reaches the left ventricle 1300 of the heart 1000 through the aortic arch 1100.

In step S12, as shown in FIG. 38, the catheter 211 covered with the bendable sheath 500 is introduced into the human body along the guiding sheath 400 and reaches the left ventricle 1300.

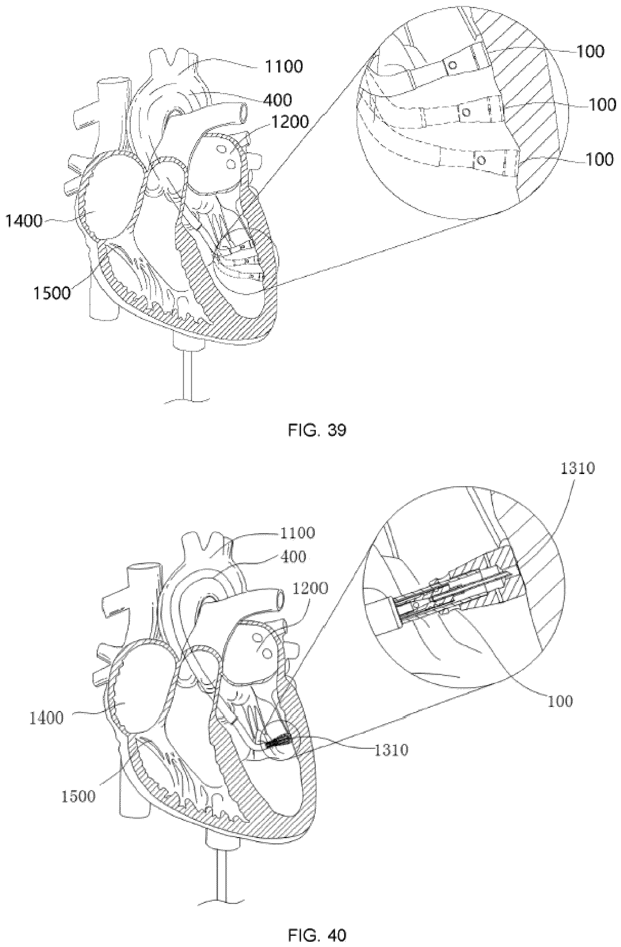

In step S13, the catheter 211 is electrically coupled to the mapping system, after the mapping system completes modeling, the catheter 211 is bent and moved with the assistance of the guiding sheath 400 and the bendable sheath 500, the location of the moving sleeve 100 is observed from the three-dimensional image transmitted from the sleeve 100, and the abutment surface 111 of the sleeve 100 is adjusted to vertically point to the tissue wall 1310 of the lesion tissue, as shown in FIG. 39.

In step S14, under the guidance of DSA (Digital subtraction angiography) and ultrasound images, the abutment surface 111 of the sleeve 100 is adjusted to abut against the tissue wall 1310 of the lesion tissue, as shown in FIG. 40.

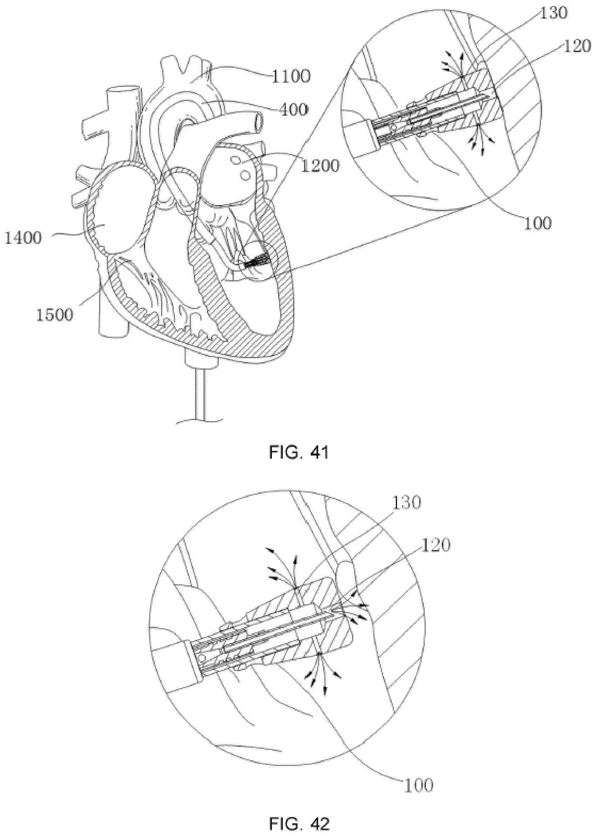

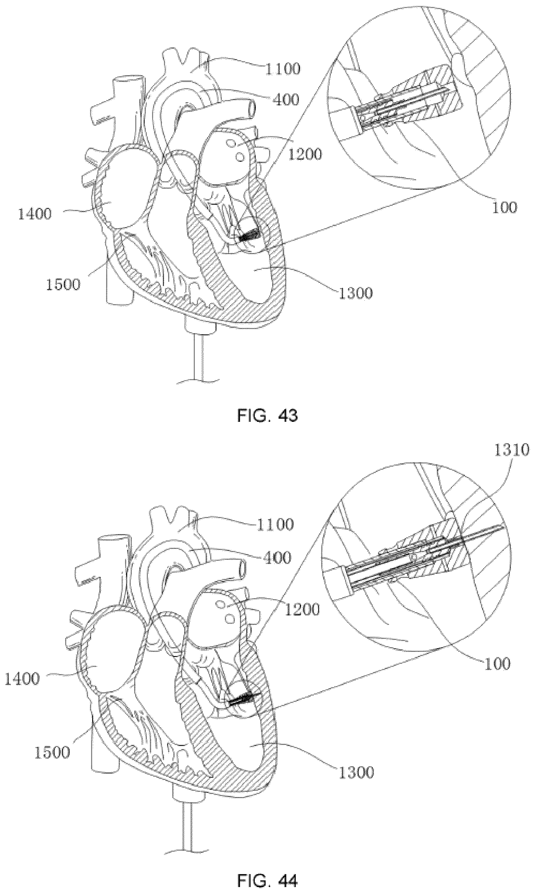

In step S15, a contrast medium is injected into the first injection part 3111 of the handle 310, so that the contrast medium flows through the catheter 211 and the sleeve 100 in sequence, and flows out from the fourth channel 120 and/or the fifth channel 130 of the sleeve. Now, the DSA image is used to determine whether the abutment surface 111 is in close contact with the tissue wall 1310. If the contrast medium flows out from the fourth channel 120 and the fifth channel 130 at the same time, as shown in FIG. 42, it means that the abutment surface 111 of the sleeve 100 is still not in contact with or only in partial contact with the tissue wall 1310 of the lesion tissue. FIG. 43 shows that the abutment surface 111 of the sleeve 100 is only in partial contact with the tissue wall 1310 of the lesion tissue. If the contrast medium only flows out from the fifth channel 130 of the sleeve 100, as shown in FIG. 41, it means that the abutment surface 111 of the sleeve 100 has closely contacted the tissue wall 1310, and the next step can be performed. Otherwise, steps S13 and S14 need to be repeated until the abutment surface 111 of the sleeve 100 is in close contact with the tissue wall 1310.

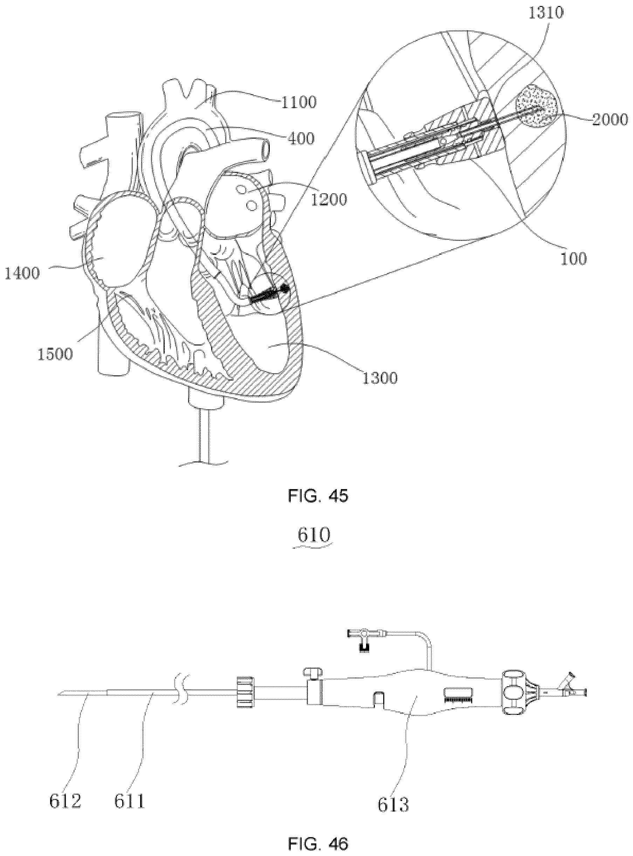

In step S16, as shown in FIGS. 44 and 45, the needle body 221 is driven by the driving member 3113 of the handle 310 to penetrate into the tissue wall 1310. A contrast medium is first injected into the second injection part 3112 of the handle 310. By observing the DSA image, if the contrast medium spreads or flows in a certain direction, it means that the needle body 221 may have penetrated into a groove of a pectinate muscle or into a blood vessel. In this case, the conditions for injecting hydrogel are not met, and the needle needs to be withdrawn and the previous steps are repeated to find the correct tissue wall 1310 and align the abutment surface 111 of the sleeve 100 with the correct tissue wall 1310 until the following conditions are met: under the DSA image, the contrast medium forms a clump and beats with the heart. In this case, the hydrogel 2000 can be injected through the second injection part 3112 into the lesion tissue. The needle body 221 is withdrawn after the injection is completed.

In step S17, the above steps are repeated to find the second injection point, and so on. After injections at all desired injection points, the guiding sheath 400, catheter 211 and sleeve 100 are withdrawn, thereby finishing the injection surgery.

Referring to FIGS. 38 and 46, in some embodiments, an ablation device is disclosed, including a delivery assembly 620 and an ablation assembly 610.

The distal end of the delivery assembly 620 in this embodiment is connected to the sleeve 100 according to any of the above embodiments. The ablation assembly 610 is movably inserted in the delivery assembly 210, and the ablation assembly 610 includes an ablation needle 612. The delivery assembly 620 is configured to be introduced into the heart 1000 through lumens. After the ablation needle 612 moves out from the delivery assembly 620 and the sleeve 100, it enters the myocardial tissue by puncturing the endocardium to ablate the myocardial tissue.

Referring to FIG. 38, the delivery assembly 620 includes a guiding sheath 400 and a bendable sheath 500. The bendable sheath 500 is movably inserted into the guiding sheath 400, and the sleeve 100 is disposed at the distal end of the bendable sheath 500. The ablation needle 612 is movably inserted into the bendable sheath 500. The sleeve 100 is driven by the bendable sheath 500 to be positioned on the tissue wall 1310.

Referring to FIG. 46, the ablation assembly 610 includes an insulating sheath 611, an ablation needle 612 and a second handle 613. The proximal end of the ablation needle 612 is connected to the second handle 613. At least part of the ablation needle 612 is provided with an ablation section, and the ablation section applied with ablation energy generated by an ablation energy generating device can perform ablation on the myocardial tissue. The insulating sheath 611 is movably arranged around the ablation needle 612 and is detachably and rotatably connected to the second handle 613. The distal end of the ablation needle 612 extends out of the insulating sheath 611, and the section of the ablation needle 612 extending out of the insulating sheath 611 performs ablation.

The following describes the ablation process of the ablation device 600 in this embodiment.

In step S21, a standard puncture via the femoral artery is performed, and the guiding sheath 400 is introduced into the human body via the femoral artery puncture and reaches the left ventricle 1300 of the heart 1000 through the aortic arch 1100, as shown in FIG. 37.

In step S22, the sleeve 100 is pushed by the bendable sheath 500 into the human body along the interior lumen of the guiding sheath 400 and reaches the left ventricle 1300, as shown in FIG. 38.

In step S23, the catheter 211 is electrically coupled to the mapping system, after the mapping system completes modeling, the bendable sheath 500 is bent and moved, the location of the moving sleeve 100 is observed from the three-dimensional image transmitted from the sleeve 100, the location is selected with the assistance of the guiding sheath 400 and the bendable sheath 500, the abutment surface 111 of the sleeve 100 is adjusted to vertically point to the tissue wall 1310 of the lesion tissue, as shown in FIG. 39.

In step S24, under the guidance of DSA (Digital subtraction angiography) and ultrasound images, the abutment surface 111 of the sleeve 100 is adjusted to abut against the tissue wall 1310 of the lesion tissue, as shown in FIG. 40.

In step S25, a contrast medium is injected into the catheter 211 so that the contrast medium flows out of the sleeve 100. Now, the DSA image is used to determine whether the abutment surface 111 is in close contact with the tissue wall 1310. If the contrast medium flows out from the fourth channel 120 and the fifth channel 130 at the same time, as shown in FIG. 42, it means that the abutment surface 111 of the sleeve 100 is still not in contact with or only in partial contact with the tissue wall 1310 of the lesion tissue. FIG. 43 shows that the abutment surface 111 of the sleeve 100 is only in partial contact with the tissue wall 1310 of the lesion tissue. If the contrast medium only flows out from the fifth channel 130 of the sleeve 100, as shown in FIG. 41, it means that the abutment surface 111 of the sleeve 100 has closely contacted the tissue wall 1310, and the next step can be performed. Otherwise, steps S13 and S14 need to be repeated until the abutment surface 111 of the sleeve 100 is in close contact with the tissue wall 1310.

In step S26, as shown in FIGS. 44 and 45, the ablation needle 612 is driven by the second handle 613 to penetrate into the tissue wall 1310, and the ablation energy generating device provides ablation energy to the ablation needle 612 to ablate the lesion tissue.

In step S27, the above steps are repeated to find the next ablation point. After ablations at all desired ablation points, the guiding sheath 400, the bendable sheath 500, the sleeve 100 and the ablation needle 612 are withdrawn, thereby finishing the ablation.

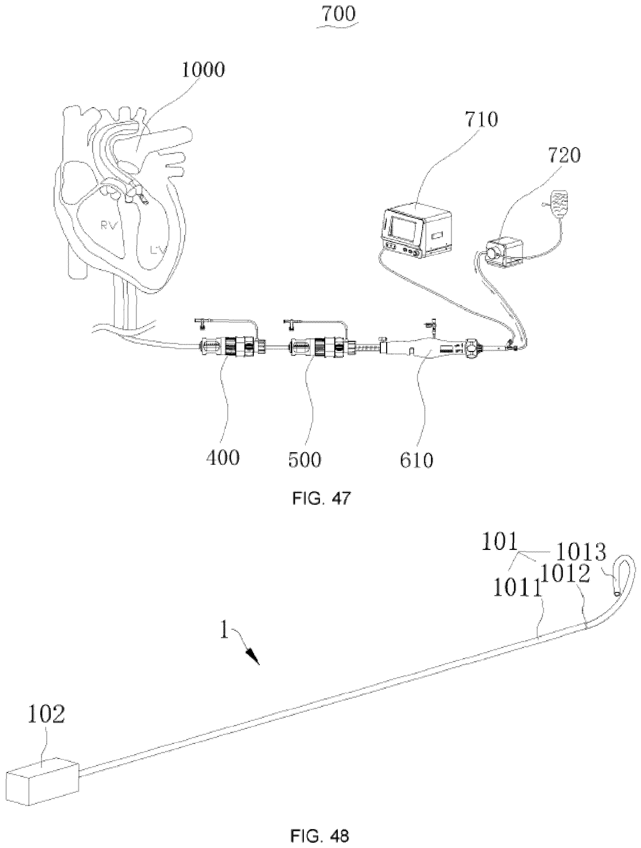

Referring to FIG. 47, in a sixth embodiment, the present disclosure further provides an ablation system 700, which includes an ablation energy generating device 710 and the above-mentioned ablation device 600.

The ablation energy generating device 710 is coupled with the ablation device 600 to provide energy for the ablation device 600.

It can be understood that the temperature of the tissue and blood will increase during the ablation process. In order to protect the human body, the ablation system 700 further includes a perfusion device 720. The perfusion device 720 is used to provide liquid to the ablation device 600, so that the perfusion device 720 can provide liquid to the periphery of the tissue wall 1310, to enlarge the ablation area and cool the tissue and blood within the ablation area. The fluid provided by the perfusion device 720 can be cold saline.

Referring to FIGS. 48 to 54c, some embodiments disclose an adaptive guiding device 1, which includes a hollow flexible catheter 101 of a certain length. The flexible catheter 101 has been pre-shaped, and in its natural state it is bent at least five times relative to its own central axis in different planes, wherein the angle between the two planes of one bend closest to the distal end is greater than 90°, and the angles between the planes of at least four consecutive bends is less than 90°, so that at least part of the flexible catheter can conform to the anatomical shape of the aortic arch.

The angle between the planes of at least four consecutive bends being less than 90° is explained referring to the arc segments below. For example, the first arc segment 10121, the second arc segment 10222, the third arc segment 10123, and the fourth arc segment 10124 each define a plane, and the angle (deflection amplitude) between the planes where adjacent arc segments are located is less than 90°. In the figure, the first arc segment 10121 is located on the plane W1, the second arc segment 1022 is located on the plane W2, and the angle between the plane W1 and plane W2 is less than 90°. The same applies to the other arc segments. Since the arc segments are not coplanar, bending in different planes is achieved.

The angle between the two planes of one bend closest to the distal end is greater than 90°. The flexible catheter 101 can be the guiding sheath 1042 or the guiding sheath 400 in the previous embodiments, and the details below can also be applied to the guiding sheath 1042 or the guiding sheath 400. By providing the guiding device with a flexible catheter 101 which is pre-shaped with a specific shape, the distal end of the flexible catheter 101 in a natural state can conform to the anatomical shape of the aortic arch of the human body, thereby simplifying the guiding operation, improving the reliability of the device, reducing the pressure to human blood vessels and thus reducing the damage to blood vessels, and improving the operation safety of the treatment device.

In this embodiment, in order to provide the adaptive guiding device 1 with strong compliance in human blood vessels, as well as certain pushability and twist resistance, the flexible catheter 101 preferably uses a pre-shaped multi-layer sheath structure. The adaptive guiding device 1 is an adaptive guiding sheath. Pre-shaping refers to the process of placing the flexible catheter in a mold with a specific shape and heating it to a certain temperature to form the flexible catheter into a specific shape. In this embodiment, pre-shaping specifically includes the following steps: placing the multi-layer sheath in the mold the inner cavity of which corresponding to the anatomical shape of the aortic arch, heating the placed multi-layer sheath and the mold together, cooling to room temperature, and de-molding. The heating temperature and time depend on the material and structure of the sheath.

In other embodiments, the flexible catheter 101 can be a metal cut tube, a metal wire braided tube, or a flexible tube made of other polymer materials, which only needs to be pre-shaped so that the catheter has a specific shape adapted to the anatomical shape of the aortic arch.

Further, in this embodiment, the multi-layer sheath structure of the flexible catheter 101 includes at least three layers, including an inner layer (not shown in the figure), a middle layer (not shown in the figure) and an outer layer (not shown in the figure) arranged sequentially from the inside to the outside. The inner layer is a polymer inner film, which can be made of PTFE (polytetrafluoroethylene). The middle layer is a metal braided mesh, which can be stainless steel mesh or tungsten wire mesh. The outer layer is a polymer outer film, and can be optionally made of PEBAX. Depending on the curvature of blood vessels, the hardness of the outer film of the multi-layer sheath at different parts can be varied to adapt to the curvature of different blood vessels, and to improve the pushability and twist resistance of the sheath. For example, the commonly used hardness specifications of PEBAX are 25D, 35D, 55D, 72D, etc. PEBAX with different hardness can be used for different parts of the outer film of the catheter as needed to meet the performance requirements of different parts of the catheter.

Referring to FIGS. 48 to 54c, furthermore, the flexible catheter 101 includes a main section 1011, a trans-aortic arch section 1012 and a distal section 1013 connected in sequence from the proximal end to the distal end, wherein the trans-aortic arch section 1012 has a curved structure simulating the anatomical shape of the aortic arch 3. The main section 1011 is the starting section of the overall flexible catheter 101 and provides support for the overall structure. The trans-aortic arch section 1012 simulates the anatomical shape of the aortic arch 3 and is configured to traverse the aortic arch 3 of the human heart, to improve the compliance with the aortic arch 3 and reduce the pressure to vessels. The distal section 1013 is an output end of the catheter and configured to provide guidance for the treatment device installed in the flexible catheter 101.

For ease understanding and simple description, the side close to the main section 1011 is designated as the proximal end of the flexible catheter 101, and the side close to the distal section 1013 is designated as the distal end of the flexible catheter 101. The treatment device is inserted from the proximal end of the flexible catheter 101 and extends out from the distal end, that is, the lumen inlet of the flexible catheter 101 is located at its proximal end, and the lumen outlet is located at its distal end.

The main section 1011 is usually a straight section or an approximately straight section, and mainly functions to traverse the femoral artery and descending aorta to reach the start of the turning part of the aortic arch 3. Therefore, it is the longest section of the guiding sheath, and the length can be in the range of 540 mm to 840 mm.

In order to avoid twisting or bending of the flexible catheter 101 in curved blood vessels and to increase the twist resistance of the flexible catheter 101, the main section 1011 usually has a high hardness. In this embodiment, the main section 1011 preferably has a PEBAX outer film with a hardness of 72D. The trans-aortic arch section 1012 is connected to the main section 1011 and the distal section 1013 for traversing and conforming to the anatomical shape of the aortic arch 3.

In order to improve the compliance with the aortic arch 3 and reduce the pressure to the blood vessels, the trans-aortic arch section 1012 uses a curved arc segment simulating the shape and curvature of the aortic arch 3. Since the aortic arch 3 is a three-dimensional arch, the more the arc segments that simulate the aortic arch, the better the guiding device can conform to the aortic arch 3. However, the processing difficulty will be increased accordingly. Further, too many arc segments will cause the tube of the trans-aortic arch section 1012 to be stiffer, reducing crossability, and affecting the adaptability.

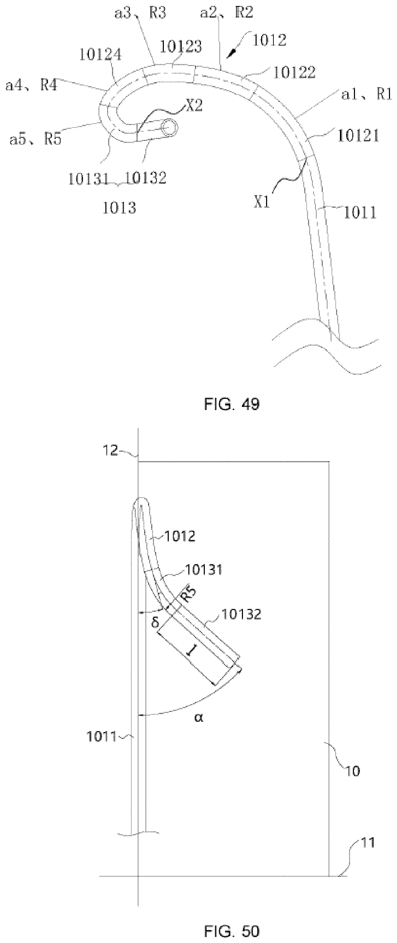

Referring to FIG. 49, further, in this embodiment, the trans-aortic arch section 1012 at least includes a first arc segment 10121, a second arc segment 10222, a third arc segment 10123 and a fourth arc segment 10124 that are sequentially connected and tangent one after another. The first arc segment 10121, second arc segment 10222, third arc segment 10123 and fourth arc segment 10124 are located on different planes. The first arc segment 10121 is connected with and communicated to the main section 1011, and the fourth arc segment 10124 is connected with and communicated to the distal section 1013.

Therefore, the fitness or conformity between the trans-aortic arch section 1012 of the flexible catheter 101 and the aortic arch 3 is higher, the adaptability and crossability are better, and the pressure to blood vessels can be reduced to reduce blood vessel damage.

Further, the angle of the first arc segment 10121 is a1, and the radius of the arc bend of the first arc segment 10121 is R1; the angle of the second arc segment 10222 is a2, and the radius of the arc bend of the second arc segment 10222 is R2; the angle of the third arc segment 10123 is a3, and the radius of the arc bend of the third arc segment 10123 is R3; the angle of the fourth arc segment 10124 is a4, and the radius of the arc bend of the fourth arc segment 10124 is R4.

4.5° ≤ a1 ≤ 30°, 90 mm ≤ R1 ≤ 450 mm; 25° ≤ a2 ≤ 80°, 10 mm ≤ R2 ≤60 mm; 20° ≤ a3 ≤ 70°, 25 mm ≤ R3 ≤ 50 mm; 20° ≤ a4 ≤ 110°, 20 mm ≤ R4 ≤ 50 mm.

The above parameters are obtained by simulation calculation based on the common structure of the aortic arch 3. With the above parameters, the trans-aortic arch section 1012 can better conform to the anatomical shape of the aortic arch 3 of most human hearts, and the pressure to blood vessels can be significantly reduced, in order to reduce blood vessel damage and improve the operation safety of the treatment device.

In addition, in order to provide the trans-aortic arch section 1012 after shaping with a certain hardness and prevent the tube of the trans-aortic arch section 1012 from bending when being pressed by the blood vessel wall, or prevent the trans-aortic arch section 1012 from being stretched when delivering the treatment device, in this embodiment, the hardness of the PEBAX outer film of the trans-aortic arch section 1012 is preferably 55D.

Referring to FIGS. 49 to 52, further, the distal section 1013 at least includes a transition segment 10131 and a straight segment 10132. The transition segment 10131 is arc-shaped, and its two ends are respectively tangent to the straight segment 10132 and the trans-aortic arch section 1012. This can avoid sharply changes in the curvature of the tube of the distal section 1013 and reduce the resistance and difficulty of the treatment device passing through the guiding device.

The at least five bends obtained by the pre-shaping mentioned above can be understood as four of them correspond to the first arc segment 10121, the second arc segment 10222, the third arc segment 10123 and the fourth arc segment 10124, and the fifth bend corresponds to the transition segment 10131 that is pre-shaped into an arc shape.

Further, as shown in FIG. 50, the angle of the transition segment 10131 is a5, and the radius of the arc bend of the transition segment 10131 is R5 ( projection on the first plane 10), where 15° ≤ a5 ≤ 70°, and 25 mm ≤ R5 ≤ 45 mm.

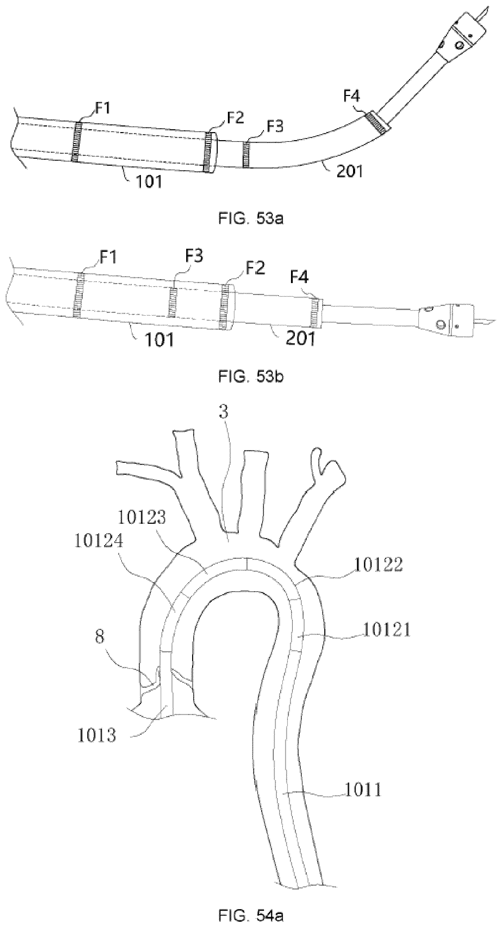

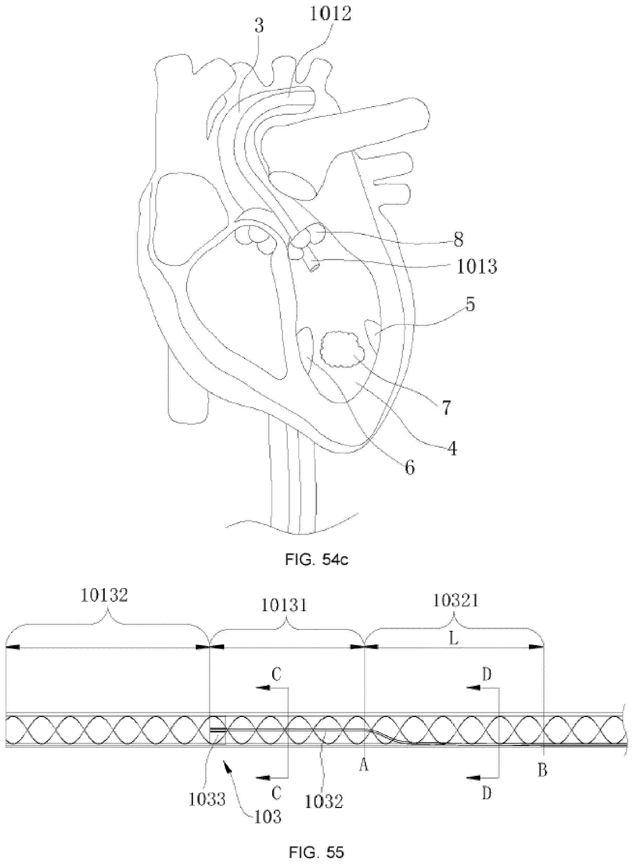

Referring to FIG. 54c, the angle a5 of the transition segment 10131 and the radius R5 of the arc bend thereof affect the orientation of the straight segment 10132. Further, the bending radius of the transition segment 10131 will also affect the bending force. The larger the bending radius, the smaller the rigidity of the transition segment 10131, and the smaller the bending force required when bending. However, the bending radius should not be too large otherwise it would affect the crossability of the tube of the transition segment 10131. Therefore, in this embodiment, based on the above settings, the distal section 1013 in the initial state can point toward the middle position between the anterior papillary muscle 5 and the posterior papillary muscle 6, so that the treatment device that passes through the flexible catheter 101 and enters the heart can have a larger operation and adjustment space and a convenient initial position for adjustment, that is, the delivered treatment device has a wider operation range and coverage, while avoiding affecting the crossability of the tube of the transition segment 10131 and the bending force.

Referring to FIGS. 50 to 52, further, the angles between the central axis of the projection of the straight segment 10132 on the first plane 10, the second plane 11, and the third plane 12, respectively, and the central axis of the main section 1011 are α, β, γ. The first plane 10 is a vertical plane, the second plane 11 is a perpendicular plane perpendicular to the vertical plane, and the third plane 12 is a horizontal plane perpendicular to both the vertical plane and the perpendicular plane, where 35° ≤ α ≤ 85 °, 25° ≤ β ≤ 80°, and 20° ≤ γ ≤ 75°.

It can be seen from the figure that the first plane 10 and the third plane 12 intersect perpendicularly. For ease of understanding, the perpendicular intersection position is roughly the central axis of the main section 1011. The first arc segment 10121 is generally located on the third plane 12. The angles a1 to a4 of the first to fourth arc segments can be understood as the corresponding central angles of projections of the segments on the third plane 12.

As shown in the figure, taking the angle of the first arc segment 10121 as an example, by projecting the first arc segment 10121 on the third plane, the angle a1 can be determined according to its radius R1.

Referring to FIG. 54c, the angles α, β, and γ jointly affect the orientation of the straight segment 10132 at the distal end of the flexible catheter 101. The angle α determines the distance between the distal end of the flexible catheter 101 and the anterior or posterior papillary muscles 5 or 6 in the heart. The smaller the α, the closer the straight segment 10132 of the flexible catheter 101 is to the posterior papillary muscle 6; and the greater the α, the closer the straight segment 10132 of the flexible catheter 101 is to the anterior papillary muscle 5. The angles β and γ determine the distance between the straight segment 10132 of the flexible catheter 101 and the interventricular septum or free wall 9 in the heart. The smaller the β and γ, the closer the straight segment 10132 of the flexible catheter 101 is to the free wall 9; and the greater the β and γ, the closer the straight segment 10132 of the flexible catheter 101 is to the interventricular septum. In this embodiment, by setting α, β and γ in the above ranges which affect the orientation of the end of the straight segment 10132 of the flexible catheter 101, the end of the straight segment 10132 of the flexible catheter 101 can accurately point toward the middle position of the area in front of the papillary muscles, preventing the end of the straight segment 10132 after entering the heart from being initially positioned in other areas except the middle position between the anterior papillary muscle 5 and the posterior papillary muscle 6, so that the straight segment 10132 of the flexible catheter 101 can be located in an appropriate position after entering the heart, and thus the straight segment 10132 of the flexible catheter 101 is easier to point to the target area 7 and quickly lock it.

Referring to FIG. 50 and FIG. 54c, further, the length of the straight segment 10132 is I, where 0 < I < 80mm. In this embodiment, the length of the straight segment 10132 is preferably 40 mm. Within the suitable range, the longer the length of the straight segment 10132, the more accurate the orienting of the treatment device extending out of the straight segment 10132. In this embodiment, the above range allows the treatment device passing through the straight segment 10132 to accurately reach the target area 7.

In order to provide the trans-aortic arch section 1012 after shaping with a certain hardness and prevent the tube of the trans-aortic arch section 1012 from bending when being pressed by the blood vessel wall, or prevent the trans-aortic arch section 1012 from being stretched when delivering the treatment device, in this embodiment, the hardness of the PEBAX outer film of the transition segment 10131 and the straight segment 10132 of the distal section 1013 is preferably 25D.

Further, the angle between the central axis of the projection of the transition segment 10131 on the first plane 10 and the central axis of the main section 1011 is δ, where the first plane 10 is a vertical plane, and 0 < δ ≤ 45°.

The angle δ affects the initial position of the transition segment 10131 of the distal section 1013. Based on the above settings, it can be ensured that when the adaptive guiding device 1 enters the left ventricle 4, the trans-aortic arch section 1012 of the shaped flexible catheter 101 will be located at the middle position of the aortic arch 3, which provides enough space for the flexible catheter 101 to be displaced laterally during the adjustment process, and also greatly reduces the pressure to the blood vessels and aortic valve 8 in the heart.

Referring to FIG. 48, the adaptive guiding device 1 further includes an operating handle 102, which is connected to the main section 1011 and used to drive the flexible catheter 101 to move axially and circumferentially.

Specifically, the operating handle 102 is convenient for the operator to hold and operate, so that the flexible catheter 101 can move axially and circumferentially, and thus the distal section 1013 of the flexible catheter 101 can accurately point to the target area 7, providing accurate guidance for the treatment device. The operating handle 102 can be a conventional handle.

In the technical solution according to this embodiment, the flexible catheter 101 is pre-shaped so that the flexible catheter 101 in a natural state is bent at least five times relative to the central axis in different planes, thereby improving the adaptability of the guiding device to the vascular morphology. The guiding device can better adapt to the complex anatomical morphology of the human aortic arch 3, thereby simplifying the guidance operation, reducing the pressure to human blood vessels, and improving the reliability of the device. In addition, the structure of the guiding device is simplified, and the operation safety of the treatment device is improved. Further, the guiding device in use does not require a large avoidance space, so that the guiding device has a larger operation and adjustment space and a more convenient initial position for adjustment, and the tissue can be prevented from extending into the avoidance area, thereby reducing blood vessel damage and improving the operation safety of the treatment device.

Referring to FIG. 55, in some embodiments, the adaptive guiding device 1 further includes a pull assembly 103 for accurately adjusting the lateral displacement of the distal section 1013 of the flexible catheter 101 to further improve the adaptability to the vascular morphology.

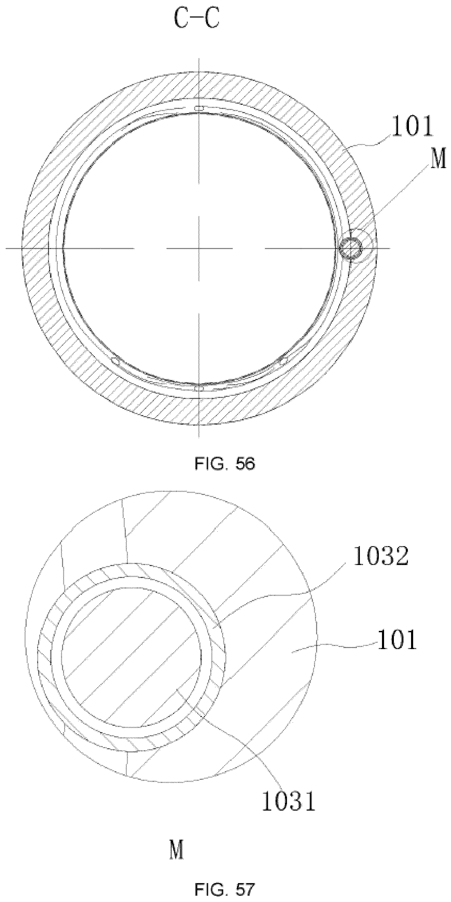

Referring to FIGS. 55 to 58, the pull assembly 103 includes a pull wire 1031, a guiding passageway 1032 and an anchor ring 1033. The guiding passageway 1032 is defined in the wall of the flexible catheter 101, that is, the guiding passageway 1032 is defined between the outer layer and the inner layer of the flexible catheter 101, specifically between the inner layer and the middle layer, or between the middle layer and the outer layer. The guiding passageway 1032 is configured for the pull wire 1031 to pass through. The anchor ring 1033 is fixedly connected to the transition segment 10131 of the distal section 1013, and the pull wire 1031 is fixedly connected to the anchor ring 1033 and the operating handle 102 respectively.

The pull wire 1031 is a flexible filament with a certain length and toughness, and can be selected from stainless steel braided ropes, non-metallic braided ropes (such as commonly used sutures), steel wires, nickel-titanium wires, etc. In this embodiment, stainless steel braided wires are preferred due to good toughness, bending resistance and flexibility.

The guiding passageway 1032 is defined in the wall of the flexible catheter and is used for the pull wire 1031 to pass through and protect the pull wire 1031. The guiding passageway 1032 can be a PI (polyimide) pipe, a PEEK (poly(ether-ether-ketone)) pipe, a flexible stainless steel cut tube, etc.. However, if the material used for the passageway of the pull wire 1031 is too hard, it will increase the resistance to the flexible catheter 101 during bending and lateral displacement. In this embodiment, the guiding passageway preferably uses a PI pipe.

Specifically, the operating handle 102 pulls the anchor ring 1033 through the pull wire 1031 of the pull assembly 103, thereby pulling the distal section 1013 of the flexible catheter 101, so as to control the movement amplitude and direction of the lateral displacement of the distal section 1013 of the flexible catheter 101, to ensure that the distal section 1013 of the flexible catheter 101 is located at the outflow tract of the aortic arch 3, providing a large space for bending and facilitating further clinical operations.

Referring to FIG. 55, further, the anchor ring 1033 is fixed at the transition segment 10131 of the distal section 1013.

That is to say, the transition segment 10131 serves as the to-be-bent segment. The pull wire 1031 is pulled by the operating handle 102, so that the transition segment 10131 can be bent accordingly, thereby driving the straight segment 10132 to displace laterally, so as to control the movement amplitude and direction of the lateral displacement of the distal section 1013 of the flexible catheter 101. Further, by designing the transition segment 10131 as the to-be-bent segment, as the end thereof is connected with the straight segment 10132, only a slight bending to the transition segment 10131 would cause a significant positional change of the end of the straight segment 10132. That is, only a small bending force is required in order to cause a significant positional change of the end of the distal section 1013 of the flexible catheter 101. Therefore, the movement space required by the flexible catheter 101 itself is reduced, and the output end of the distal section 1013 of the flexible catheter 101 can be adjusted more accurately and reliably.

Referring to FIG. 55, further, the anchor ring 1033 is fixedly connected to one end of the transition segment 10131 close to the straight segment 10132.

By designing the main section 1011 as the basis for rotation, the pull torque can be maximized and the pull force required for adjustment of the straight segment 10132 can be reduced.

Furthermore, the anchor ring 1033 can be made of stainless steel. In the case where the anchor ring 1033 is made of stainless steel, it can be fixedly connected to the pull wire 1031 by welding. In some embodiments, a single pull wire 1031 can be arranged in the guiding passageway 1032. By welding, one end of the single pull wire 1031 can be fixed to the anchor ring 1033, and the other end can be fixed to the operating handle 102.

The anchor ring 1033 can be made of other materials, in which case, the pull wire 1031 can be looped and connected to the anchor ring 1033.

In some embodiments, at least two pull wires 1031 are provided, that is, at least a first pull wire and a second pull wire are provided. The first pull wire and the second pull wire form a closed loop of a pull wire group, and one end of the pull wire group is connected to the anchor ring 1033, and the other end is connected to the operating handle 102.

Accordingly, the pull wire group can be pulled by the operating handle 102, so as to pull the distal section 1013 of the flexible catheter 101 to move in different directions.

Further, each pull wire 1031 is provided with a guiding passageway 1032, avoiding multiple pull wires 1031 being arranged in the same guiding passageway 1032 which would cause blockage or interference with each other, maintaining the reliability of adjustment, reducing the diameter of a single guiding passageway 1032 and reducing the profile of the entire flexible catheter 101.

In this embodiment, the pull wire 1031 and the anchor ring 1033 are preferably connected by welding, which can reduce the outer diameter of the whole of the pull wire 1031 and the anchor ring 1033, thereby reducing the resistance during lateral bending, and also reducing the outer diameter of the flexible catheter 101. The material of the anchor ring 1033 is preferably tantalum. Compared with stainless steel, tantalum has a better contrast effect under ultrasound.

Referring to FIG. 55, further, before the flexible catheter 101 is pre-shaped, in the direction from the distal section 1013 toward the main section 1011, the projection of the guiding passageway 1032 perpendicular to the central axis of the flexible catheter 101 gradually deflects around the central axis.

Therefore, compared with the case where the guiding passageway 1032 extends in a straight line parallel to the central axis of the flexible catheter 101 before the flexible catheter 101 is pre-shaped, in this embodiment, after the flexible catheter 101 is pre-shaped, the part of the guiding passageway 1032 from the distal section 1013 to the main section 1011 can deflect around the central axis, and the pull wire 1031 passing through the guiding passageway 1032 will deflect accordingly. Therefore, when the pull wire 1031 is pulled by the operating handle 102, the distal section 1013 can deflect better relative to the main section 1011 with the main section 1011 as the basis for deflection, thereby reducing the deflection of the main section 1011 in the same direction when adjusting the distal section 1013, and reducing the pressure to blood vessels.

Referring to FIGS. 55 to 58, further, the deflection start point of the guiding passageway 1032 at the distal section 1013 is A, and point B is deflected by 90° relative to the point A in the circumferential direction of the flexible catheter 101. A transitional guiding section 10321 is defined between point A and point B. The transitional guiding section 10321 extends along a part or the entire of the trans-aortic arch section 1012, or a part or the entire of the main section 1011.

That is, the terminal point of the deflected part of the guiding passageway 1032 can be set at the trans-aortic arch section 1012 or the main section 1011 in practice according to the desired operation effects.

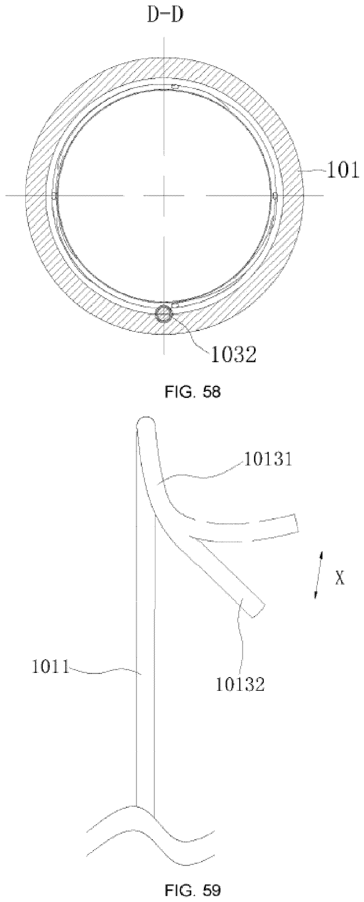

Referring to FIGS. 55 and 59, the length L of the transitional guiding section 10321 affects the deflection of the flexible catheter 101 during lateral bending. If the length of the transitional guiding section 10321 is too short, during lateral bending, a great bending force is required. If the length L of the transitional guiding section 10321 is too long, for example, if the entire pull wire 1031 extends and deflects from the point A to the operating handle 102, the entire flexible catheter 101 would be prone to deflect toward the pull wire 1031 when adjusting the distal section 1013 of the flexible catheter 101, increasing the deflection amplitude of the flexible catheter 101, increasing the pressure to the blood vessels, and easily causing damage to the blood vessels. In the present disclosure, the length L of the transitional guiding section 10321 is in the range of 0 mm < L≤ 250 mm.

Furthermore, due to the high hardness of the PEBAX outer film of the main section 1011, the pull wire 1031 has a small impact on the main section 1011. Therefore, the length of the transitional guiding section 10321 mainly affects the pull force for the trans-aortic arch section 1012 and the deflection amplitude of the trans-aortic arch section 1012 during bending. In this embodiment, the length of the transitional guiding section 10321 is preferably 40 mm, so that the pull force for the trans-aortic arch section 1012 and the deflection amplitude of the trans-aortic arch section 1012 during bending can be appropriate.

Since the transition segment 10131 of the distal section 1013 serves as the to-be-bent segment for lateral displacement, the hardness of the PEBAX outer film is proportional to the bending force of the pull wire 1031. The lower the hardness of the PEBAX outer film, the smaller the bending force of the pull wire 1031 when the distal end of the flexible catheter is displaced.

Therefore, the PEBAX outer film of the transition segment 10131 is preferably 25D, which not only requires a small bending force, but also ensures a certain strength to prevent the tube of the transition segment 10131 from being pressed by the blood vessel wall and bent.

In addition, since the flexible catheter 101 in this embodiment needs to displace laterally in use, it is necessary to provide a fulcrum for the flexible catheter 101 at the aortic arch 3 so that the part of the flexible catheter 101 in front of the fulcrum remains motionless, while in the other part in rear of the fulcrum, the distal section 1013 of the flexible catheter 101 can be displaced laterally by bending, thereby displacing a specified section in a specified direction.

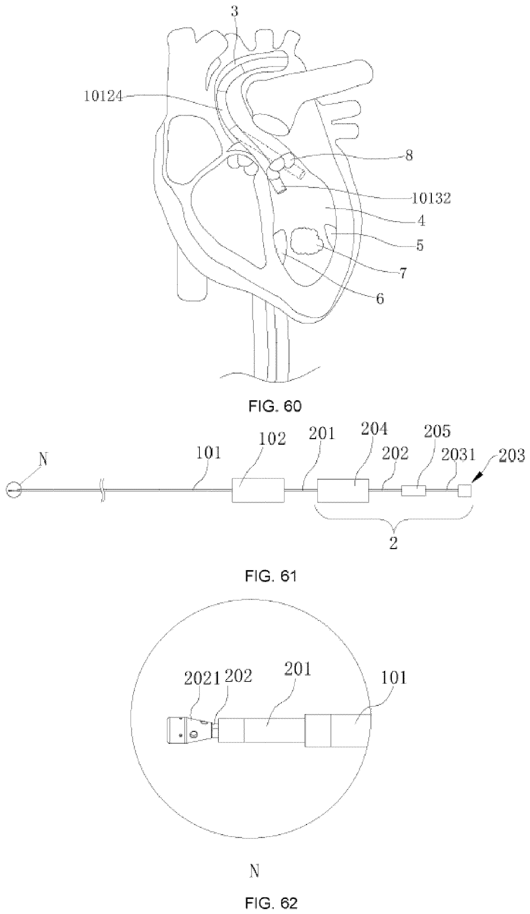

Specifically, as shown in FIGS. 59 and 60, the fourth arc segment 10124 of the trans-aortic arch section 1012 serves as a fulcrum to abut against the outside of the aortic arch 3, so that when the flexible catheter 101 is displaced laterally, the flexible catheter 101 has an appropriate support force, while the first arc segment 10121, the second arc segment 10222, and the third arc segment 10123 are not in contact with the inside of the aortic arch 3, thereby reducing the pressure to blood vessels.

Referring to FIG. 61, in some embodiments, a transcatheter treatment system is disclosed, which includes the adaptive guiding device 1 according to any of the above embodiments, and further includes a treatment device 2 movably installed in the flexible catheter 101. The adaptive guiding device 1 functions to establish a path for intervention from the exterior of the human body into the interior of the human body through the aorta, and the treatment device 2 passes through the aortic arch through the flexible catheter 101. The flexible catheter 101 of the adaptive guiding device 1 serves as the outermost sheath (outer sheath in short) of the entire transcatheter treatment system, and usually cooperates with the guide wire to establish the path from the exterior of the human body into the interior of the human body.

By applying the improved adaptive guiding device 1 into the transcatheter treatment system, the guidance efficiency can be improved and the operation safety risks can be reduced.

Further, the treatment device is selected from at least one of a myocardial injection device, a myocardial ablation device, a valve repair device or a valve replacement device. That is, the adaptive guiding device 1 can be applied in transcatheter endocardium injection systems, transcatheter radiofrequency ablation systems, or heart valve repair systems, to improve guidance efficiency and reduce operation safety risks of the treatment device.

Referring to FIGS. 61 to 63, further, the treatment device 2 includes a bending sheath 201, an inner sheath 202 and a treatment assembly. The bending sheath 201 is movably inserted into the flexible catheter 101 and can be bent in a single direction. The inner sheath 202 is movably inserted into the bending sheath 201. The treatment assembly is inserted into the inner sheath 202. The treatment assembly is selected from at least one of an injection assembly, an ablation assembly, a prosthetic heart valve, an annuloplasty ring, a valve clamping device, a suture, a tissue anchor, or a tissue puncture member. For example, the treatment assembly is an injection assembly 203.

The bending sheath 201 is movably installed in the interior lumen of the flexible catheter 101 of the adaptive guiding device 1 and can be bent unidirectionally. The bending sheath 201 cooperates with the adaptive guiding device 1 to deliver the treatment assembly to the predetermined treatment site. The inner sheath 202 is movably installed in the interior lumen of the bending sheath 201 to prevent the treatment assembly from scratching the inner wall of the bending sheath 201 and to protect and ensure the movement of the treatment assembly.

Referring to FIG. 61, FIG. 62, FIG. 65 and FIG. 68, optionally, the distal end of the inner sheath 202 is fixedly connected to a limiting element. The limiting element can be a metal sleeve 2021. The end face of the metal sleeve 2021 is a plane with a certain area, in order to avoid damaging or puncturing the free wall 9 of the left ventricle 4 when it is in contact with the free wall 9. The structure of the metal sleeve 2021 can use the sleeve 100 or the head 32 of the previous embodiments.

The following describes a transcatheter treatment system of the present disclosure, taking the injection assembly 203 as the treatment assembly for example.

Referring to FIGS. 61, 63 to 65, and 67 to 70, a transcatheter endocardium injection system is shown for injecting hydrogel, cells and other therapeutic agents to diseased ischemic myocardial tissue through a catheter to improve heart function, and can be applied in heart failure treatment. The transcatheter endocardium injection system includes an adaptive guiding device 1 and a treatment device 2. The adaptive guiding device 1 serves as an outer sheath, and the treatment device 2 is inserted into the adaptive guiding device 1. The treatment device 2 includes an injection assembly 203. The injection assembly 203 includes an injection channel 2031 and a needle 2032 connected to and communicated with the distal end of the injection channel 2031. The needle 2032 is used to penetrate into the myocardial free wall 9 and inject therapeutic agents such as contrast medium, drugs, or hydrogels. The end plane of the metal sleeve 2021 at the distal end of the injection channel 2031 defines a through hole for the needle 2032 to pass through. The injection channel 2031 includes an inner channel (not shown in the figure) for drug injection and an outer channel for contrast medium injection. The contact of the metal sleeve 2021 with the free wall 9 can be judged by the diffusion of the contrast medium on the free wall 9.

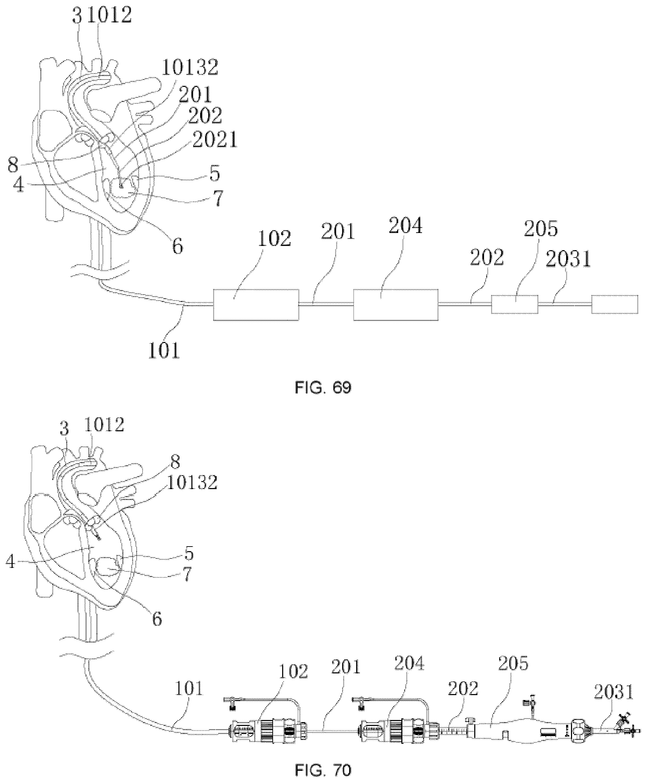

Specifically, the treatment device 2 includes a bending sheath 201 inserted in the flexible catheter 101, an inner sheath 202 inserted in the bending sheath 201, and an injection assembly 203 inserted in the inner sheath 202. Under the main guidance of the outer sheath, with the bending sheath 201 as the intermediate support and auxiliary adjustment basis, the injection assembly 203 can pass through the inner sheath 202 and then inject therapeutic drugs or other agents into the target area 7 (myocardial tissue). FIG. 70 is a further exemplary structural schematic view of the transcatheter endocardium injection system.

Referring to FIGS. 61, 63, 67-70, in order to facilitate the adjustment of the bending sheath 201 and the inner sheath 202, a first adjustment handle 204 and a second adjustment handle 205 are provided corresponding to the bending sheath 201 and the inner sheath 202 respectively. Both the first adjustment handle 204 and the second adjustment handle 205 can be conventional handles.

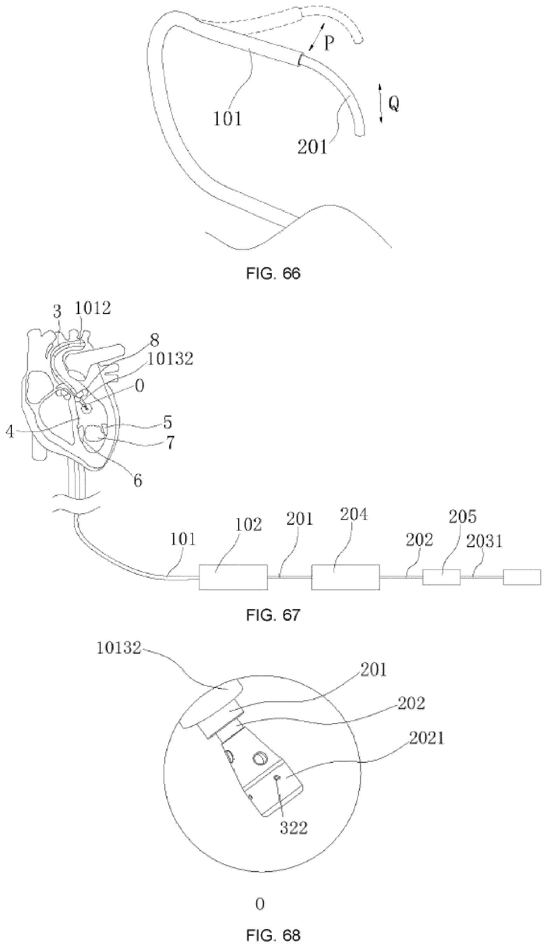

Referring to FIG. 66, further, the bend direction of the bending sheath 201 is different from the bend direction of the distal section 1013 of the flexible catheter 101 of the adaptive guiding device 1. By setting the bend direction of the bending sheath 201 to be different from the bend direction of the distal section 1013, the adjustment range of the orientation of the inner sheath 202 passing through the bending sheath 201 and thus the orientation of the treatment assembly passing through the inner sheath 202 can be enlarged. Therefore, the adjustment flexibility of the output end of the transcatheter treatment system can be improved, which is conducive to guiding the treatment assembly passing through the inner sheath 202 to the target area 7 more accurately, achieving precise guidance.

Referring to FIG. 66, further, the bend direction of the bending sheath 201 is substantially perpendicular to the bend direction of the distal section 1013 of the flexible catheter 101 of the adaptive guiding device 1.

As a preferred embodiment, the bending sheath 201 is arranged in the direction perpendicular to the bend direction of the distal section 1013, in order to improve the adjustment flexibility of the output end of the transcatheter treatment system and guide the treatment assembly passing through the inner sheath 202 to the target area 7 more accurately, achieving precise guidance.

For example, in use of the endocardium injection system of the present disclosure, the distal section 1013 of the outer sheath (i.e., the flexible catheter 101 of the adaptive guiding device 1) is displaced laterally, and the bend direction of the middle sheath (i.e., the bending sheath 201) that is inserted into the lumen of the outer sheath is the vertical direction perpendicular to the lateral displacement. The different bend directions of the two sheaths cooperating with each other further ensure that the injection assembly is delivered to the desired site for drug injection, so as to achieve good therapeutic effects.

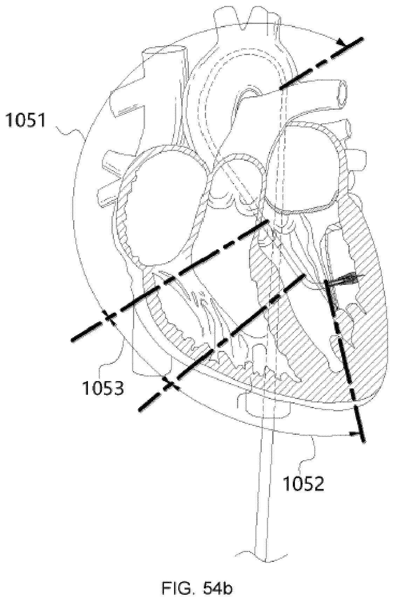

Referring to FIG. 54b, an embodiment of the present disclosure provides a guide tube assembly for a transcatheter treatment system. The guide tube assembly can adapt to complex in-vivo environments and establish an interventional path for the delivery of treatment device such as the inner sheath and the treatment assembly into the human body. The guide tube assembly has opposing distal and proximal ends. The distal part of the guide tube assembly has a curved extension including:
a first curved section 1051, which is configured to have a bend angle by pre-shaping and / or bending; and
a second curved section 1052, which is located at the distal end of the first curved section 1051 and configured to have a bend angle by bending.

The first curved section 1051 and the second curved section 1052 are located in different planes, and are provided by one or two tubes respectively which are movably inserted one in the other.

Taking the intervention into the left ventricle through the aorta as an example, the first curved section 1051 can adaptively pass through and be placed in the aortic arch, and be supported on the outside of the turning part of the aortic arch. In the case where the first curved section 1051 is pre-shaped, it has a profile similar to the aortic arch in the natural state. In the case of bending, the first curved section 1051 can have other shapes in the natural state and be bent into the desired shape in the human body. Alternatively, pre-shaping and bending can be used in combination. Especially for the section at the distal end of the first curved section 1051, if active bending can be performed, it will be more convenient to obtain the desired orientation, so that the section at the distal end can cross the aortic valve conveniently.

Depending on the needs of treatment (such as injection, ablation, etc.), especially for multi-point treatment, the second curved section is preferably configured to be actively bent, to facilitate the completion of multi-point treatment in a single intervention.

The first curved section 1051 and the second curved section 1052 can be provided by the same tube. For example, the bending sheath 201 can be pre-shaped at a corresponding section to form the first curved section 1051, and can be bent at the distal end thereof to form the second curved section 1052. Alternatively, the bending sheath 201 can have two or more bendable points. For example, anchor rings can be respectively arranged at preset force application points, and pull wires can be used to perform bending.

The first curved section 1051 and the second curved section 1052 can be alternatively provided by two tubes respectively. For example, the flexible catheter 101 can be pre-shaped and bent to form the first curved section 1051. The first curved section 1051 can correspond to the first to fourth arc segments and the transition segment of the flexible catheter 101.The specific parameters can refer to R1 to R5, a1 to a5, etc. as mentioned in other embodiments. For example, the angle of the first arc segment is a1, and the radius of the arc bend of the first arc segment is R1; the angle of the second arc segment is a2, and the radius of the arc bend of the second arc segment is R2; the angle of the third arc segment is a3, and the radius of the arc bend of the third arc segment is R3; the angle of the fourth arc segment is a4, and the radius of the arc bend of the fourth arc segment is R4; wherein 4.5° ≤ a1 ≤ 30°, 90 mm ≤ R1 ≤ 450 mm; 25° ≤ a2 ≤ 80°, 10 mm ≤ R2 ≤ 60 mm; 20° ≤ a3 ≤ 70°, 25 mm ≤ R3 ≤ 50 mm; 20° ≤ a4 ≤ 110°, 20 mm ≤ R4 ≤ 50 mm. The angle of the transition segment is a5, and the radius of the arc bend of the transition segment is R5, wherein 15° ≤ a5 ≤ 70° and 25 mm ≤ R5 ≤ 45 mm.

The bendable section of the bending sheath 201 movably inserted into the flexible catheter 101 serves as the second curved section 1052. Since the bending sheath 201 and the flexible catheter 101 are movably matched with each other, the division of the first curved section and the second curved section 1052 can refer to the specific arrangement during treatment, wherein at least part of the bending sheath 201 at the distal end thereof extends out of the flexible catheter 101, and the distal section of the bending sheath 201 is bent to form the second curved section 1052 in order to target or change the injection site. Although the part of the bending sheath 201 inside the flexible catheter 101 is attributed to the first curved section in the sense of division, it does not need to be pre-shaped and bent, instead, it is only passively and adaptively inserted into the flexible catheter 101. Therefore, the part of the bending sheath 201 inside the flexible catheter 101 will not be considered as part of the first curved section.

In order to accurately cross the valve, the first curved section and the second curved section can be connected to each other through an extension section. The extension section generally extends in a straight line. The extension section is configured to cross the aortic valve. The extension section between the first curved section and the second curved section can be provided by the flexible catheter 101, that is, the most distal section of the flexible catheter 101 has a straight segment. Alternatively, the extension section between the first curved section and the second curved section can be provided by the bending sheath 201, that is, the bending sheath 201 has a straight segment on the proximal side of the bendable section which can extend out of the flexible catheter 101.

The proximal side of the first curved section can be the main section 1011 mentioned above as the starting part of the entire flexible catheter 101 and the support basis. The main section 1011 does not need to be pre-shaped or bent. As a spatial reference, it can be approximately regarded as extending roughly in a straight line (which can be designated as a reference axis, corresponding to the central axis of the main section 1011) in the natural state. In a desired configuration, the distal end of the first curved section is located on the outflow side of the aortic valve and points to the center of the aortic valve.

The distance between the proximal end of the first curved section and the distal end of the first curved section in three-dimensional space refers to, for example, the distance between the proximal end X1 of the first curved section and the distal end X2 of the first curved section in three-dimensional space as shown in FIG. 49.

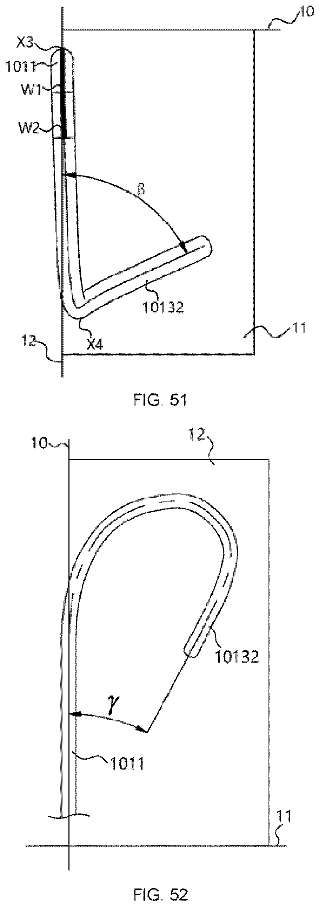

In a plane perpendicular to the reference axis, the distance between the portion of the first curved section that is farthest from the reference axis and the reference axis reflects the overall span of the first curved section. The overall span refers to, for example, the distance between the position X3 where the reference axis is located and the portion X4 that is farthest from the reference axis as shown in FIG. 51.

Before the first curved section is pre-shaped or if the first curved section is straightened, the angle between the orientation of the distal end of the first curved section and the orientation of the proximal end of the first curved section is 180 degrees. To form the first curved section, the orientation of the distal end gradually changes so that the angle between the orientation of the distal end and the orientation of the proximal end gradually reduces. Further, since the first curved section has a non-planar three-dimensional configuration, the orientation of the distal end of the first curved section and the orientation of the proximal end of the first curved section are not coplanar in the three-dimensional space.

Since the second curved section is configured to be actively bent, its spatial orienting is more flexible to adapt to different directions in the left ventricle. In order to facilitate the monitoring of the surgical process, the distal section of the guide tube assembly can be configured with one or more imaging marks. For example, the anchor ring at the bending force application point can be directly used as the imaging mark, or additional imaging marks can be configured.

In order to facilitate the bending operation, imaging marks can be configured at the respective distal ends (generally also the bending force application points) of the first curved section and the second curved section. Furthermore, imaging marks can be configured at the proximal end of the second curved section and on the straight segment between the first curved section and the second curved section. For example, imaging marks can be configured at both the distal end and the proximal end of the straight segment. In the case where the sections are connected to each other, there is no need to configure the imaging mark at the connection.

To adapt to different treatment sites, the length of the distal section of the guide tube assembly, particularly the distance between the first curved section and the second curved section, is variable. For example, a guide tube assembly for a transcatheter treatment system in one embodiment has opposite distal and proximal ends, and the distal section of the guide tube assembly includes the following sections arranged in sequence from the proximal end to the distal end:
a first curved section, which is configured to have a bend angle by bending;
an extension section, the length of which is adjustable; and
a second curved section, which is configured to have a bend angle by bending.

At least part of each of the first curved section and the second curved section is bendable. The bending can be implemented using existing technologies. For example, an anchor ring can be embedded at the bending force application point, and force can be applied to a pull wire connected to the anchor ring for bending.

In this embodiment, the first curved section and the second curved section are respectively provided by different tubes, wherein the first curved section is provided by a first tube, and the second curved section is provided by a second tube that slidably passes through the first tube. The extension section includes a straight segment of the most distal section of the first tube, and a straightly extending segment of a section of the second tube which can extend out of the first tube, which is adjacent to the straight segment.

The extension section extends in a straight line. Since it is between the first curved section and the second curved section, it can be provided by one or two of the first tube and the second tube. During bending, or taking the practical anatomical configuration in the human body into account, the first curved section and the second curved section are in different planes, and the first curved section itself is not a planar section. The first curved section itself can not only be bent, but can also be pre-shaped, and its pre-shaping features can refer to the various embodiments of the flexible catheter 101 mentioned above.

The length of the extension section is in the range of 0 to 120 mm, for example, in the range of 20 to 120 mm, for another example, in the range of 20 to 80 mm, for further example, in the range of 40 to 80 mm. In the first tube, the most distal section is a straight segment, which is also at least part or even the entire of the extension section, and the length of the straight segment is in the range of 0 to 80 mm, for example, in the range of 40 to 80 mm.

The straightly extending segment of the section of the second tube which can extend out of the first tube, which is adjacent to the straight segment, can also be regarded as at least a part of the extension section, and the length of this segment is in the range of 0 to 80 mm, for example, in the range of 40 to 80 mm.

The first tube and the second tube are slidably matched with each other, so that the distance between the first curved section and the second curved section can be flexibly and significantly changed to adapt to the complex interventional path or for switching of multiple treatment sites.

For flexible orienting, the first tube and the second tube have different bend directions. For each tube, its bend direction is generally determined by its own structure, especially the circumferential position of the bending force application point and the change in the local strength of the tube itself. For example, the first tube and the second tube respectively have force application points for bending and pulling, and the force application points of the two are offset from each other in the circumferential direction, so as to obtain different bend directions.

The first tube and the second tube are not only slidably matched in the axial direction, but also rotatably matched, and at least can be rotated to have different bend directions. For example, the first tube and the second tube have perpendicular bend directions.

Taking trans-aortic access as an example, the distal section of the first tube needs to pass through the aortic arch, and the length of the corresponding first curved section is in the range of 160 to 300 mm, for example, in the range of 200 to 260 mm. After entering the left ventricle through the extension section, the section of the second tube extending out of the first tube is mainly used for bending and changing the spatial direction to adapt to different treatment sites. The length of the corresponding second curved section is in the range of 20 to 80 mm, for example, in the range of 40 to 80 mm.

The first curved section and the second curved section are respectively bent entirely or partially. For example, the bent portions of the first curved section and the second curved section are adjacent to their respective distal sections. As mentioned above, the first curved section is bent by pre-shaping, and has a bendable transition segment adjacent to the distal section thereof.

To guide the operation in real time and accurately determine the positions or relative postures of the tubes in the human body, the guide tube assembly is configured with imaging marks at the bending force application points. For example, the material of the anchor ring itself can be displayed on the imaging device, and the distal ends of the first curved section and the second curved section respectively serve as bending force application points and are configured with imaging marks.

In order to further determine the relative positions and postures, the distal sections of the first tube and the second tube are each configured with two imaging marks. The distance between the two imaging marks is in the range of 20 to 120 mm, for example, in the range of 40 to 80 mm, or approximately 40 mm.

With reference to FIGS. 53a and 53b, taking the above-mentioned flexible catheter 101 and bending sheath 201 as the first tube and the second tube respectively for example, the flexible catheter 101 is provided with imaging marks F1 and F2, and F2 is located on the distal side of F1.

F1 is located at the bending force application point of the first tube, corresponding to the connection between the first curved section and the extension section (also the connection between the transition segment and the straight segment); F2 is located at the most distal end of the first tube.

The bending sheath 201 is provided with imaging marks F3 and F4, and F4 is located at the distal side of F3. F3 and F4 correspond to two ends of the second curved section respectively, and F4 is located at the bending force application point of the second tube.

The distal end of the second curved section can be adjacent to or serve as the distal end of the second tube. The distance between the distal end of the second curved section F4 and the distal end of the second tube is in the range of 0 to 20 mm. The length of the extension section is adjustable. That is to say, referring to the layout of the imaging marks, when the second tube extends out of the distal end of the first tube, the distance between F1 and F4 is adjustable. The distance between F1 and F4 is in the range of 40 to 200 mm, for example, in the range of 60 to 120 mm, for another example, 80 mm.

The variation in the length of the extension section can further refer to FIGS. 53a to 53b. The extension section includes the straight segment between F1 and F2 at the most distal section of the first tube, and the length of this segment is fixed.

When F3 is gradually exposed to the distal end of F2, the section between F2 and F3 (i.e., the straightly extending segment of the section of the second tube which can extend out of the first tube, which is adjacent to the straight segment) is also regarded as part of the extension section. As F3 moves distally further, the length between F2 and F3 changes, realizing the length change of the extension section, so that different requirements can be flexibly met.

According to the imaging marks on the flexible catheter 101, the position of the distal end of the first curved section and the position of the most distal end of the flexible catheter 101 can be determined. According to the imaging marks on the bending sheath 201, the position of the distal end of the second curved section can be determined, and it can also be determined whether the second curved section completely extends out of the most distal end of the flexible catheter 101 by referring to the imaging mark at the most distal end of the flexible catheter 101. The bending should not be performed until at least most of the second curved section extends out of the flexible catheter 101. Otherwise, the bending will be interfered by the flexible catheter 101, affecting the bending effect. In combination with the above guide tube assembly, an embodiment of the present disclosure also provides a transcatheter treatment system, including:
a guide tube assembly;
an inner sheath movably inserted into the guide tube assembly; and
a treatment assembly movably installed in the inner sheath and being extendable out of the distal end of the inner sheath.

For ease of operation, the transcatheter treatment system further includes:
a plurality of handles, which are respectively arranged at the proximal ends of the treatment assembly, the inner sheath and the guide tube assembly; and
an auxiliary support including a guide rod and a plurality of clamping members that can move along the guide rod and be positioned on the guide rod. The handles are respectively installed on corresponding clamping members, and the relative positions thereof are adjusted through the movement of the clamping members.

The proximal ends of the first tube and the second tube of the guide tube assembly are each equipped with a handle corresponding to a respective clamping member on the auxiliary support. The relative position of the imaging marks on the distal sections of the first tube and the second tube are adjusted by the corresponding clamping members on the auxiliary support, and can be verified through the position of the clamping members, or vice versa.

The specific arrangement of the handles can refer the embodiment as shown in FIG. 1. For example, the guide tube assembly includes a first tube and a second tube that is slidably inserted into the first tube. The first tube has a first, bendable curved section. The second tube has a second, bendable curved section.

The proximal end of the first tube is connected to the guide handle 1044; the proximal end of the second tube is connected to the bending handle 1064; the proximal ends of the inner sheath and the treatment assembly are connected to the operating handle 20; wherein the guide handle, the bending handle, and the operating handle are arranged in sequence from the distal end to the proximal end, and the distances therebetween are adjustable.

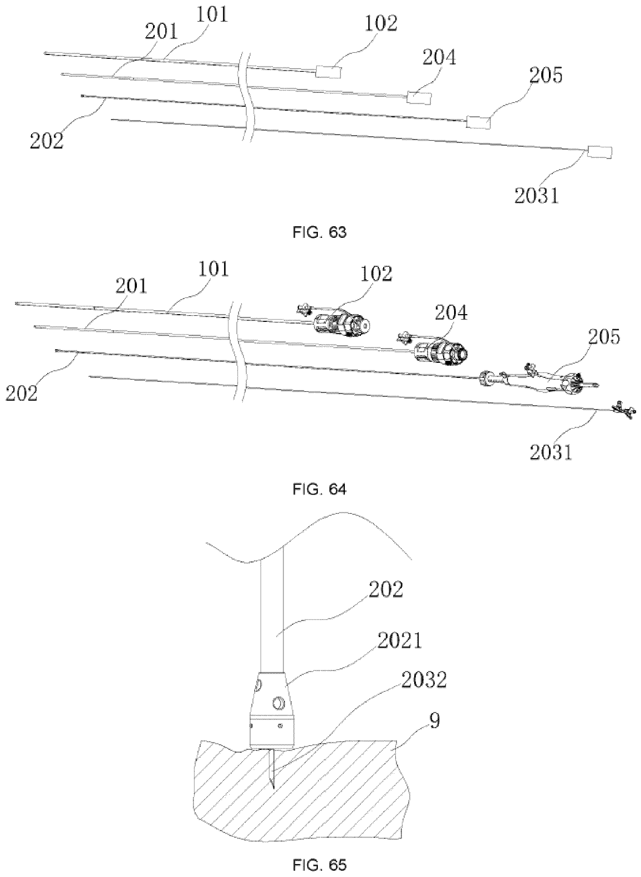

The first tube and the second tube can refer to the previous embodiments, for example:
the guide handle 1044 connected to the guiding sheath 1042; the bending handle 1064 connected to the bendable sheath 1062; and the operating handle 20 connected to the sheath 30 as shown in FIG. 1 and FIG. 2;
the second handle 613 of the ablation assembly 610 as shown in FIG. 46, and the handle 310 connected to the catheter 211 as shown in FIG. 35;
the operating handle 102 connected to the flexible catheter 101, the first adjustment handle 204 connected to the bending sheath 201, and the second adjustment handle 205 connected to the inner sheath 202 as shown in FIG. 64.

The movement of the three clamping members along the guide rod is transmitted to the corresponding distal ends through the corresponding handles. Taking the flexible catheter 101 and the bending sheath 201 as an example, the movement of the clamping members can reflect the position change of the corresponding distal ends.

In order to facilitate the operation, any two of the handles have a synchronous motion state after locking, and an independent motion state after unlocking. Taking the two most proximal handles (the bending handle and the operating handle) as an example, the synchronous motion refers to that the bending handle and the operating handle can move as a whole relative to the first tube, which facilitates operations such as quick positioning over long distances. However, when fine adjustment is required, the bending handle and the operating handle are controlled independently in most cases.

In order to facilitate operation, a driving wheel can be configured for the operating handle. The driving wheel drives the operating handle through a transmission mechanism to change the distance from the bending handle, so as to achieve fine adjustment. The driving wheel can be arranged on the auxiliary support to drive the clamping member, or be arranged on the bending handle to drive the operating handle.

The switching between synchronous motion state and independent motion state can be realized through a locking mechanism. The locking mechanism can be provided by the transmission mechanism through linkage or de-linkage, or can be configured additionally.

The locking mechanism can act between the clamping member and the guide rod. By controlling the clamping member to move or not, the linkage state of the handles is controlled. For the bending handle and the operating handle, the locking mechanism can also act between the two. The treatment assembly can be an ablation assembly including an ablation needle, or an injection assembly. In case of injection assembly, the transcatheter treatment system further includes an implantable material delivered through the injection assembly, and the implantable material is hydrogel.

The hydrogel system can be a sodium alginate-calcium alginate system; or a sodium alginate-alkaline earth metal salt system (the alkaline earth metal salt is an alkaline earth metal salt containing magnesium, calcium, strontium, and barium, and the alkaline earth metal salt is at least one of sulfate, citrate, gluconate, lactate, carbonate, phosphate, and alginate. Specifically, the alkaline earth metal salt can be a soluble calcium salt, that is, a sodium alginate-soluble calcium salt system is used).

The transcatheter treatment system of the present disclosure involves a long interventional path in use. For example, the interventional path extends from the femoral artery to the heart through the aortic arch. The interventional path is generally more than 1 meter, in addition, the device is partially extended outside the human body, and thus the implantable material needs to be delivered by 1 to 2 meters in a lumen with a smaller diameter. For example, the first tube and the second tube of the guide tube assembly are both more than 1 meter, such as 1 to 2 meters. Therefore, the selection of the gel system has an impact on the surgical effect. In the prior art, when the storage modulus of the gel is nonuniform, the part of the gel with a larger storage modulus will block the needle, causing the push pressure to increase rapidly in a short period of time, which is not conducive to long-distance injection and/or implantation of the hydrogel in the catheter. On one hand, the off-site preparation of hydrogels may involve cross contamination. On the other hand, on-site preparation of hydrogels takes a long time and delays the surgery or experimental time. Specific treatment sites have high demands on the properties of myocardial injection gel compositions, and it is difficult to balance physical, chemical, and biological properties to meet practical clinical needs.

The present disclosure not only provides a suitable hydrogel system, but also provides improved process parameters. The hydrogel of the present disclosure can be prepared on site. By adjusting the proportion of components, the gelation time of the hydrogel is ≤ 1 h, for example ≤ 5 min, for another example, ≤ 1 min.

The injection timing is controlled so that the gel elasticity (storage modulus G', generally in the range of 2880 to 4250 pa) is 60% 85% of the maximum storage modulus (generally in the range of 4400 to 5000 pa) after injection into the organism and crosslinking reaction. In addition, considering the characteristics of the delivery method in catheter intervention, in order to obtain good injectable performance, the present disclosure discloses a suitable gel viscosity range, that is, the loss modulus G" is in the range of 500 ± 300 pa, and the storage modulus G' > G".

The myocardial repair is based on Laplace's law. In order to achieve cardiac remodeling, it is difficult to determine the range of the amount of gel implanted at a single point and the amount of gel implanted each time. The present disclosure provides an improved solution. For example, for a single injection site, the gel volume injected at a single point is 300 µl. In case of multiple injection sites in a single interventional treatment, the gel volume injected each time is in the range of 2 to 3 ml. Considering the characteristics of the delivery path through the femoral artery-aortic arch 3-left ventricle 4, and referring to the disclosure related to FIGS. 50 to 52, an embodiment of the present disclosure further provides a transcatheter treatment system, including:
an adaptive guiding device including a hollow flexible catheter, the flexible catheter including a main section, a trans-aortic arch section and a distal section that are connected and communicated from proximal to distal, the trans-aortic arch section being pre-shaped into a plurality of arc segments distributed in different planes, the distal section at least including a transition segment and a straight segment, the transition segment being arc-shaped and its two ends being tangent to the straight segment and the trans-aortic arch section respectively, the transition segment being configured to be bendable, and the angles between the central axis of the projection of the straight segment on the first plane, the second plane and the third plane, respectively, and the central axis of the main section being α, β, and γ respectively, where the first plane is a vertical plane, the second plane is a perpendicular plane perpendicular to the vertical plane, and the third plane is a horizontal plane perpendicular to both the vertical plane and the perpendicular plane, and 35° ≤ α ≤ 85 °, 25° ≤ β ≤ 80°, 20° ≤ γ ≤ 75°;
a bending sheath being movably inserted in the flexible catheter and being bendable in a single direction different from the bend direction of the distal section; and
a treatment assembly being arranged in the bending sheath.

In addition, it may further include an inner sheath that is movably inserted into the bending sheath, and the treatment assembly is movably arranged in the inner sheath.

Through the combination of the pre-shaping and the bendable characteristic of the flexible catheter, it is easier and faster to center (align) the distal end of the flexible catheter with the aortic valve. Especially when the distal end of the flexible catheter does not cross the valve, the orienting accuracy of the distal end of the flexible catheter has a great influence on the needle orienting in the subsequent process.

In order to improve the operating efficiency, the bending force-bearing position of the flexible catheter is on the transition segment, that is, the anchor ring, for example, is positioned adjacent to the connection of the transition segment with the straight segment 10132, so that it can affect the orienting of the distal end of the straight segment 10132 to a greater extent. Furthermore, since the injection site generally avoids two papillary muscles, the bend direction of the bending sheath 201 has an inherent synergistic relationship with the pre-shaped configuration and adjustment direction of the flexible catheter 101.

The transcatheter treatment system for injection can refer to the aforementioned interventional injection system 1000A. The treatment assembly, inner sheath and the configured handle can use the aforementioned interventional injection device 100A, corresponding to the injection member 40, the sheath 30, and the operating handle 20 respectively, so the specific structural features will not be described in detail. The guide tube assembly of the transcatheter treatment system can correspond to the bendable guiding sheath 1042 and the bendable sheath 1062.

The transcatheter treatment system for injection can refer to the above-mentioned injection device 200. The treatment assembly, inner sheath and configured handle can use the above-mentioned injection assembly 220, catheter 211 and handle 310, and the specific structural features will not be described in detail. The guide tube assembly of the transcatheter treatment system can correspond to the guiding sheath 400 and the bendable sheath 500.

The transcatheter treatment system for ablation can refer to the ablation assembly 610 mentioned above.

In the case where the treatment assembly is an injection assembly, the following takes the delivery path through the femoral artery - aortic arch 3 - left ventricle 4 as an example to illustrate the method of using the transcatheter treatment system according to this embodiment, and also provide a method for transcatheter treatment, including: providing the transcatheter treatment system according to any of the above embodiments; delivering the distal part of the transcatheter treatment system into the left ventricle via the aortic arch; determining the current treatment location on the endocardium of the left ventricle and performing corresponding operations. Specifically, the method includes the following steps.

In step S1, as shown in FIG. 67, after puncture of the femoral artery, the adaptive guiding device 1 cooperates with the conventional guide wire to reach the left ventricle 4 through the aortic arch 3 via the femoral artery. Now, the flexible catheter 101 of the adaptive guiding device 1 is in the initial state, wherein the distal end of the tube of the adaptive guiding device 1 is centered at a certain distance below the aortic valve 8. Then the treatment device 2 is delivered into the left ventricle 4 along the flexible catheter 101 of the adaptive guiding device 1 until the metal sleeve 2021 at the distal end of the inner sheath 202 is exposed.

After the flexible catheter 101 reaches the predetermined position first, and then the treatment device 2 is delivered, which is conducive to taking advantage of the pre-shaping of the flexible catheter 101 and avoiding the impact on the pre-shaping if the flexible catheter 101 is delivered synchronously with the treatment device 2. Alternatively, the distal end of the flexible catheter 101 does not cross the valve, that is, it stays above the aortic valve 8 and does not extend into the left ventricle.

In step S2, as shown in FIGS. 67 and 69, the distal output end of the flexible catheter 101 is adjusted to point between the anterior and posterior papillary muscles 6. Now, the bending sheath 201 of the treatment device 2 is pushed forward so that the distal end of the bending sheath 201 is at a certain distance from the distal output end of the flexible catheter 101, and then the distal end of the bending sheath 201 is bent so that the metal sleeve 2021 at the distal end of the inner sheath 202 faces the target area 7 as perpendicularly as possible. Then, the inner sheath 202 is pushed further forward until the metal sleeve 2021 contacts the free wall 9 of the target area 7.

In step S3, as shown in FIG. 65, the needle 2032 at the distal end of the injection channel 2031 penetrates into the free wall 9. Now, the contrast medium can be injected into the outer channel of the injection channel 2031 and the diffusion of the contrast medium is observed. For example, if the contrast medium diffuses out from the circumferentially distributed through holes of the metal sleeve 2021, it can be determined that the needle 2032 has firmly penetrated into the treatment site. Now, the therapeutic agent such as hydrogel can be injected into the inner channel to fill the free wall 9 with the drug so as to achieve drug injection.

Alternatively, for example, the fifth channel 130 in FIG. 24 can also be used for determining the positioning of the distal end of the metal sleeve 2021. Before the needle 2032 penetrates into the free wall 9, whether the distal surface of the metal sleeve 2021 is in close contact, or in partial contact, or not in contact with the tissue wall of the myocardial tissue can be judged by the flow direction of the contrast medium. FIG. 68 also shows that the side wall of the metal sleeve 2021 has injection orifices 322 equivalent to the fifth channel.

In step S4, if injection at multiple treatment sites is required, the needle 2032 is withdrawn so that the needle 2032 is retracted into the metal sleeve 2021; and the inner sheath 202 is withdrawn so that the metal sleeve 2021 contacts the distal end of the bending sheath 201. The bent configuration of the flexible catheter 101 is kept unchanged, the bending sheath 201 is rotated, and steps S1 to S3 are repeated so as to achieve hydrogel injection at other sites on the same level as the first site. Optionally, the extension of the flexible catheter 101 is increased or decreased, and the bending sheath 201 is rotated so as to select a higher or lower site with respect to the papillary muscle than the first site, and then steps S1 to S3 are repeated to achieve hydrogel injection.

In step S5, after completing the drug injection at all treatment sites, the needle 2032 is withdrawn, the injection channel 2031 is retracted, the bending sheath 201 is released, and the flexible catheter 101 is released. Finally, the treatment device 2 and the flexible catheter 101 are withdrawn.

For multi-point injection, in one embodiment, a method for controlling a transcatheter endocardium injection system is provided. The transcatheter endocardium injection system includes a flexible catheter 101, a bending sheath 201 and a needle 2032 slidably arranged sequentially from the outside to the inside, wherein the proximal end of the flexible catheter 101 is controlled by the operating handle 102, the proximal end of the bending sheath 201 is controlled by the first adjustment handle 204, and the proximal end of the needle 2032 is controlled by the second adjustment handle 205. The flexible catheter 101 and the bending sheath 201, driven by the corresponding handles, can be rotated and bent respectively.

The second adjustment handle 205 can be optionally connected to the inner sheath 202, which is located outside the needle 2032 and provided with a metal sleeve 2021 connected to the distal end of the inner sheath 202. In this case, the transcatheter endocardium injection system can use the relevant components in the above embodiments.

Referring to FIG. 71 and FIG. 72, the target area 7 is in the left ventricle LV. The flexible catheter 101 itself can be bent (for example, the flexible catheter 101 can be bent into the flexible catheter 101' as shown in FIG. 72), and can be driven by the operating handle 102 to rotate around its own longitudinal axis. Similarly, the bending sheath 201 itself can be bent (for example, the bending sheath 201 can be bent into the bending sheath 201' as shown in FIG. 72), and can be driven by the first adjustment handle 204 to rotate around its own longitudinal axis. In order to simplify the adjustment, the flexible catheter 101 after being adjusted in place can be kept stationary, and only the orientation and the distal/proximal position of the bending sheath 201 need to be adjusted. This allows for quick adjustment of the orientation and the position of the injection needle in specific cases of multi-point injection, thereby improving the surgical efficiency.

Referring to FIGS. 73 to 75, in the following embodiment, an operation method of the transcatheter treatment system is explained taking injection locations in a multiplerow-and-multiple-column arrangement as an example, that is, a position adjustment method of the needle for continuous injections is explained. The arrangement of injection locations in the figures is only an illustration, and can be adjusted in practice according to the area and location of the lesion, wherein the injection locations are the target area 7 and located on the left ventricular wall 206. In FIGS. 73 to 75, the inner sheath 202 and the metal sleeve 2021 are not shown, and during changing the injection location, the process of withdrawing the needle 2032 before changing the injection location and the process of advancing the needle 2032 after changing the injection location are not shown.

In injection, a method for controlling the transcatheter endocardium injection system according to this embodiment includes:
performing injection on a current injection location;
obtaining a spatial offset between the next injection location and the current injection location; and
performing injection.

The spatial offset is realized by using at least one of the following methods so as to adjust the needle position until it corresponds to the next injection location. For example, the the needle position is adjusted by adjusting the flexible catheter 10 and/or adjusting the bending sheath 201, and specifically, by rotating, bending, moving distally or proximally as a whole. The various methods can be combined, and the adjustment magnitude can be controlled based on the modeling in combination with the real-time imaging device. The change method of injection location in different situations is explained by referring to the following exemplary operations. The same applies to other situations.

Referring to FIG. 73, the flexible catheter remains unchanged and the needle 2032 can be moved from the injection location X1 to the injection location X2 by simply rotating the bending sheath 201 around its longitudinal axis.

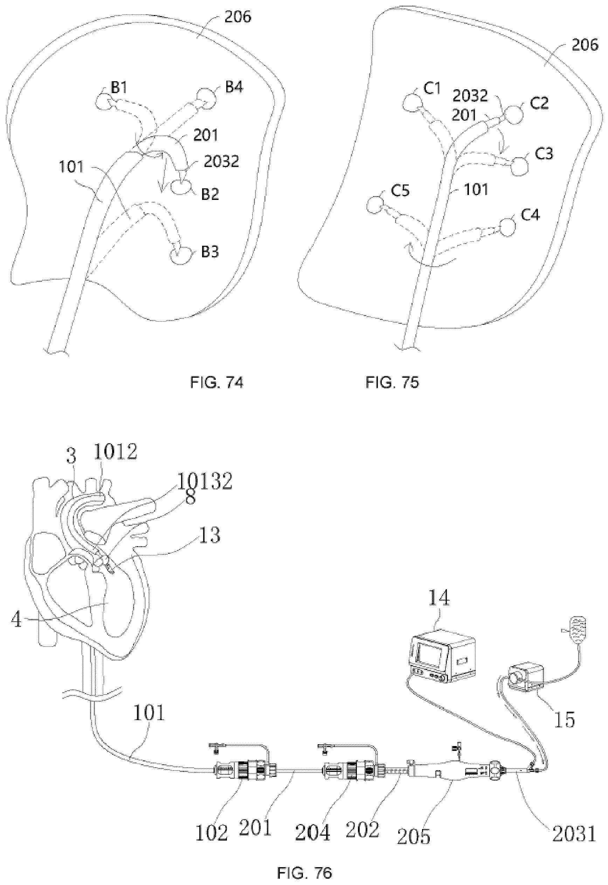

Referring to FIG. 74, the flexible catheter 101 remains unchanged, and the bending sheath 201 is rotated around its longitudinal axis, so that the needle 2032 moves from the injection location B1 to the injection location B2. The bending sheath 201 is bent to move the needle 2032 from the injection location B2 to the injection location B4.

Referring to FIG. 75, the flexible catheter 101 remains unchanged and the bending sheath 201 is rotated around its longitudinal axis to move the needle 2032 from the injection location C1 to the injection location C2 or C3. The bending sheath 201 remains bent and is retreated along the interventional path, so that the needle 2032 moves from the injection location C3 to the injection location C4. The bending sheath 201 is rotated around its longitudinal axis, so that the needle 2032 moves from the injection location C5 to the injection location C6.

In case of multiple injection locations, the bending sheath 201 is preferably rotated and bent to reduce axial movement along the interventional path, so as to reduce corresponding potential surgical risks.

It can be understood that the adaptive guiding device 1 of the present disclosure can be applied in different fields, such as transcatheter radiofrequency ablation systems, heart valve repair systems, etc., to establish lumens from the exterior of the human body to the interior of the human body for interventional devices as needed. The exemplary embodiments are not intended to limit the scope of the present disclosure.

For example, referring to FIG. 76, in the case where the adaptive guiding device 1 is applied to, for example, a transcatheter radiofrequency ablation system, an ablation assembly is used as a treatment assembly. The ablation assembly includes an ablation needle 13, an ablation energy generating device 14 connected to and providing ablation energy for the ablation needle 13, and a perfusion device 15 for providing electrolyte solution. Other structures of the transcatheter radiofrequency ablation system can refer to the above embodiments where the treatment assembly is an injection assembly, that is, the ablation assembly includes a bending sheath, an inner sheath and an ablation needle. The ablation assembly is movably inserted in the adaptive guiding device 1. After the ablation needle passes through the inner sheath, it enters the myocardial tissue by puncturing the endocardium, and then the energy generating device provides energy for the ablation needle to perform ablation on the myocardial tissue of the interventricular septum, thereby treating hypertrophic cardiomyopathy (HCM) through minimally invasive intervention.

It can also be understood that in the case where the adaptive guiding device 1 is applied in, for example, a transcatheter valve repair system, and an anchor is used as the treatment assembly, the anchor can be implanted in the ventricular wall through a catheter, and the anchor is connected with e-PTFE sutures, wherein one end of the suture is connected to the valve leaflet. The suture is used as an artificial chordae to realize the chordae tendineae repair of the valve. Alternatively, multiple anchors connected to each other through sutures can be implanted on the ventricular wall in sequence, and then the sutures are tightened to achieve ventricular volume reduction or valvuloplasty, thereby treating valvular regurgitation disease through minimally invasive intervention.

In summary, the adaptive guiding device 1 and the transcatheter treatment system including the adaptive guiding device 1 provided by the present disclosure can better adapt to the complex anatomical morphology of the aortic arch 3, and the structure of the guiding device is simplified, improving the operation safety of the treatment device. Further, the guiding device in use does not require a large avoidance space, so that the guiding device has a larger operation and adjustment space and a more convenient initial position for adjustment, and the tissue can be prevented from extending into the avoidance area, thereby reducing blood vessel damage and improving the operation safety of the treatment device.

The technical solutions of the present disclosure are not limited to the technical solutions disclosed in the above embodiments, but further include technical solutions by combining the above technical features in any suitable manner. It should be noted that the above embodiments are only preferred embodiments of the present disclosure and do not limit the present disclosure in any form. While the disclosure has been particularly shown and described in conjunction with the above preferred embodiments, these embodiments are not intended to limit the present disclosure. Those skilled in the art can make various modifications and variations to the technical solutions of the present disclosure motivated by the methods and techniques disclosed above, or obtain equivalent embodiments without departing from the scope of the disclosure. Thus, any simple amendments, equivalent changes and modifications made to the above embodiments under the teaching of the present disclosure without departing from the spirit of the present disclosure still fall within the scope of the present disclosure.

## Claims

1. A guide tube assembly for a transcatheter treatment system, having a distal end and an opposing proximal end, a distal section of the guide tube assembly having a curved extension comprising:
a first curved section which is configured to have a bend angle by pre-shaping and/or bending; and
a second curved section which is located at a distal end of the first curved section and configured to have a bend angle by bending;
wherein the first curved section and the second curved section are located in different planes and provided by one or two tubes respectively which are movably inserted one in the other.

2. The guide tube assembly for a transcatheter treatment system of claim 1, wherein the first curved section is provided by a hollow flexible catheter, and the second curved section is provided by a bending sheath;
the flexible catheter comprises a main section, a trans-aortic arch section and a distal section that are connected and communicated in sequence from a proximal end to a distal end, the distal section at least comprises a transition segment and a straight segment, wherein the trans-aortic arch section and the transition segment constitute the first curved section; and
the bending sheath is bendable in a single direction which is different from a bend direction of the distal section of the flexible catheter, and a bendable section of the bending sheath constitutes the second curved section.

3. The guide tube assembly for a transcatheter treatment system of claim 2, wherein the trans-aortic arch section is pre-shaped into a plurality of arc segments distributed in different planes, and the transition segment of the distal section is arc-shaped and two ends of the transition segment are respectively tangent to the straight segment and the trans-aortic arch section.

4. The guide tube assembly for a transcatheter treatment system of claim 3, wherein angles between a central axis of a projection of the straight segment on a first plane, a second plane, and a third plane, respectively, and a central axis of a projection of the main section are α, β, and γ respectively, wherein the first plane is a vertical plane, the second plane is perpendicular plane perpendicular to the vertical plane, and the third plane is a horizontal plane perpendicular to both the vertical plane and the perpendicular plane, where 35° ≤ α ≤ 85°, 25° ≤ β ≤ 80°, 20° ≤ γ ≤ 75°.

5. The guide tube assembly for a transcatheter treatment system of claim 4, wherein the trans-aortic arch section has a curved structure simulating an aortic arch, and comprises a first arc segment, a second arc segment, a third arc segment and a fourth arc segment connected in sequence and tangent to each other, and the first arc segment, the second arc segment, the third arc segment and the fourth arc segment are distributed in different planes, wherein the first arc segment is connected and communicated with the main section, and the fourth arc segment is connected and communicated with the distal section.

6. The guide tube assembly for a transcatheter treatment system of claim 5, wherein the first arc segment has an angle of a1, and a radius of an arc bend of the first arc segment is R1; the second arc segment has an angle of a2, and a radius of an arc bend of the second arc segment is R2; the third arc segment has an angle of a3, and a radius of an arc bend of the third arc segment is R3; the fourth arc segment has an angle of a4, and a radius of an arc bend of the fourth arc segment is R4;
wherein 4.5° ≤ a1 ≤ 30°, 90 mm ≤ R1 ≤ 450 mm; 25° ≤ a2 ≤ 80°, 10 mm ≤ R2 ≤ 60 mm; 20° ≤ a3 ≤ 70°, 25 mm ≤ R3 ≤ 50 mm; 20° ≤ a4 ≤ 110 °, 20 mm ≤ R4 ≤ 50 mm.

7. The guide tube assembly for a transcatheter treatment system of claim 3, wherein the transition segment has an angle of a5, and a radius of an arc bend of the transition segment is R5, wherein 15° ≤ a5 ≤ 70°, 25 mm ≤ R5 ≤ 45 mm.

8. The guide tube assembly for a transcatheter treatment system of claim 4, wherein an angle between a central axis of a projection of the transition segment on the first plane and the central axis of the main section is δ, where the first plane is the vertical plane, 0 < δ ≤ 45°.

9. The guide tube assembly for a transcatheter treatment system of claim 2, wherein the straight segment has a length of I, where 0 < I < 80 mm.

10. The guide tube assembly for a transcatheter treatment system of claim 2, wherein the transition segment is configured to be bent by a pull assembly, and the pull assembly comprises a pull wire, a guiding passageway and an anchor ring, wherein the guiding passageway is defined in a wall of the flexible catheter for the pull wire to pass through, the anchor ring is fixedly connected to the transition segment of the distal section, and the pull wire is connected to the anchor ring.

11. The guide tube assembly for a transcatheter treatment system of claim 10, wherein the anchor ring is adjacent to a connection of the transition segment and the straight segment; and the anchor ring also serves as an imaging mark, and a distal end of the straight segment is also configured with an imaging mark.

12. The guide tube assembly for a transcatheter treatment system of claim 10, wherein a start point of the guiding passageway at the distal section is A, point B is deflected by 90° relative to the point A in a circumferential direction of the flexible catheter, and a transitional guiding section is formed between point A and point B, wherein the transitional guiding section extends along a part or an entire of the trans-aortic arch section, or a part or an entire the main section.

13. The guide tube assembly for a transcatheter treatment system of claim 12, wherein the transitional guiding section has a length of L, where 0 mm < L ≤ 250 mm.

14. A guide tube assembly for a transcatheter treatment system, having a distal end and an opposing proximal end, a distal section of the guide tube assembly comprises:
a first curved section, which is configured to have a bend angle by bending;
an extension section, which has an adjustable length; and
a second curved section, which is configured to have a bend angle by bending;
wherein the first curved section, the extension section and the second curved section are arranged in sequence from the proximal end to the distal end.

15. The guide tube assembly for a transcatheter treatment system of claim 14, wherein the extension section extends in a straight line.

16. The guide tube assembly for a transcatheter treatment system of claim 14, wherein the first curved section and the second curved section are located in different planes.

17. The guide tube assembly for a transcatheter treatment system of claim 14, wherein the first curved section itself is a non-planar section.

18. The guide tube assembly for a transcatheter treatment system of claim 14, wherein the length of the extension section is in a range of 0 to 120 mm.

19. The guide tube assembly for a transcatheter treatment system of claim 14, wherein the first curved section and the second curved section are respectively provided by different tubes, wherein,
the first curved section is provided by a first tube;
the second curved section is provided by a second tube slidably inserted in the first tube; and
the extension section comprises a straight segment of a most distal section of the first tube, and a straightly extending segment of a section of the second tube which is extendable out of the first tube, which is adjacent to the straight segment.

20. The guide tube assembly for a transcatheter treatment system of claim 19, wherein the straight segment of the most distal section of the first tube has a length in a range of 0 to 80 mm.

21. The guide tube assembly for a transcatheter treatment system of claim 19, wherein the straightly extending segment of the section of the second tube which is extendable out of the first tube, which is adjacent to the straight segment, has a length in a range of 0 to 80 mm.

22. The guide tube assembly for a transcatheter treatment system of claim 19, wherein the first tube and the second tube have different bend directions.

23. The guide tube assembly for a transcatheter treatment system of claim 22, wherein the first tube and the second tube are rotatably matched and at least rotatable to have different bend directions.

24. The guide tube assembly for a transcatheter treatment system of claim 23, wherein the first tube and the second tube have perpendicular bend directions.

25. The guide tube assembly for a transcatheter treatment system of claim 23, wherein the first tube and the second tube each have a force application point for bending and pulling, and the force application points of the first tube and the second tube are offset from each other in a circumferential direction, thereby obtaining different bend directions.

26. The guide tube assembly for a transcatheter treatment system of claim 14, wherein the second curved section has a length in a range of 20 to 80 mm, and the first curved section has a length in a range of 160 to 300 mm.

27. The guide tube assembly for a transcatheter treatment system of claim 14, wherein the first curved section and the second curved section are respectively configured to be bent entirely or partially.

28. The guide tube assembly for a transcatheter treatment system of claim 14, wherein bent portions of the first curved section and the second curved section are respectively adjacent to distal ends of corresponding tubes.

29. The guide tube assembly for a transcatheter treatment system of claim 14, wherein the first curved section is pre-shaped and has a bendable transition segment adjacent to a distal section of the first curved section.

30. The guide tube assembly for a transcatheter treatment system of claim 14, wherein a force application point for bending of the guide tube assembly is configured with an imaging mark.

31. The guide tube assembly for a transcatheter treatment system of claim 14, wherein distal ends of the first curved section and the second curved section respectively serve as force application points for bending.

32. The guide tube assembly for a transcatheter treatment system of claim 14, wherein the guide tube assembly comprises a first tube and a second tube slidably inserted in the first tube, wherein the first curved section is provided by the first tube, the second curved section is provided by the second tube, distal sections of the tubes are each configured with at least two imaging marks, and the imaging marks on a same tube are axially spaced from each other.

33. The guide tube assembly for a transcatheter treatment system of claim 32, wherein each tube is configured with two imaging marks, and a distance between the two imaging marks is in a range of 20 to 120 mm.

34. The guide tube assembly for a transcatheter treatment system of claim 32, wherein the first tube is configured with a first imaging mark and a second imaging mark, and the second imaging mark is located at a distal side of the first imaging mark;
the first imaging mark is located at a force application point for bending of the first tube, corresponding to a connection of the first curved section and the extension section; and the second imaging mark is located at a most distal end of the first tube;
the second tube is configured with a third imaging mark and a fourth imaging mark, and the fourth imaging mark is located at a distal side of the third imaging mark; and
the third imaging mark and the fourth imaging mark correspond to two ends of the second curved section, and the fourth imaging mark is located at a force application point for bending of the second tube.

35. The guide tube assembly for a transcatheter treatment system of claim 32, wherein a distance between a distal end of the second curved section and a distal end of the second tube is in a range of 0 to 20 mm.

36. The guide tube assembly for a transcatheter treatment system of claim 34, wherein when the second tube extends out of the distal end of the first tube, a distance between the first imaging mark and the fourth imaging mark is adjustable, and the distance between the first imaging mark and the fourth imaging mark is in a range of 40 to 200 mm.

37. A transcatheter treatment system, comprising:
a guide tube assembly of any one of claims 1 to 36;
an inner sheath movably inserted in the guide tube assembly; and
a treatment assembly movably installed in the inner sheath and being extendable out of a distal end of the inner sheath.

38. The transcatheter treatment system of claim 37, further comprising:
handles respectively arranged at proximal ends of the treatment assembly, the inner sheath and the guide tube assembly; and
an auxiliary support comprising a guide rod and a plurality of clamping members that are movable along the guide rod and positionable on the guide rod, wherein the handles are respectively installed on corresponding clamping members and relative positions of the handles are adjusted by movement of the clamping members.

39. The transcatheter treatment system of claim 38, wherein the guide tube assembly comprises a first tube and a second tube slidably inserted into the first tube, and proximal ends of the first tube and the second tube are respectively configured with handles corresponding to respective clamping members on the auxiliary support; and
the first tube and the second tube are respectively configured with imaging marks, and relative positions of the imaging marks on the first tube and the second tube are adjusted through the respective clamping members on the auxiliary support.

40. The transcatheter treatment system of claim 37, wherein the treatment assembly is an ablation assembly comprising an ablation needle; or the treatment assembly is an injection assembly for supplying hydrogel.

41. The transcatheter treatment system of claim 40, wherein the hydrogel has a storage modulus in a range of 2880 to 4250 Pa and a loss modulus in a range of 200 to 800 Pa when supplied in the injection assembly.

42. The transcatheter treatment system of claim 37, wherein the treatment assembly is an injection assembly, the injection assembly comprises a needle body, a needle connector and a needle connection tube, the needle connector connects the needle body and needle connection tube, and the needle connection tube comprises an inner tube and an outer tube, wherein an interior of the inner tube is a first channel, a gap is defined between the inner tube and the outer tube and serves as a second channel, and a third channel is defined between a catheter and the injection assembly; and
a distal end of the inner sheath is provided with a sleeve for abutting a target tissue region, the sleeve comprises a main body, the main body defines a fourth channel passing through the main body in an axial direction, and a side wall of the main body defines at least one fifth channel which is communicated with the fourth channel.

43. The transcatheter treatment system of claim 42, wherein the main body comprises a distal section, and the distal section is provided with an abutment surface for abutting a myocardial tissue; and the sleeve further comprises at least one sliding member, at least a part of the sliding member protrudes from the abutment surface, and the sliding member is configured to selectively open or close the fifth channel.

44. The transcatheter treatment system of claim 43, wherein the sliding member has a first position for opening the fifth channel and a second position for closing the fifth channel, and the sleeve further comprises at least one spring element acting on the sliding member to move the sliding member toward the second position.

45. The transcatheter treatment system of claim 44, wherein the sleeve further comprises at least one sixth channel communicated with the fifth channel, and the sliding member and the spring element are arranged in the sixth channel.

46. The transcatheter treatment system of claim 45, wherein a distal end of the sixth channel defines an opening on the abutment surface, and a proximal end of the sixth channel is closed or provided with a blocking member, wherein a distal end of the sliding member protrudes from the abutment surface through the opening, and the sliding member is configured to selectively open or close the fifth channel according to a position of the sliding member in the sixth channel.

47. The transcatheter treatment system of claim 45, wherein a proximal end of the sliding member is connected to the spring element, and a proximal end of the spring element abuts a proximal end of the sixth channel.

48. The transcatheter treatment system of claim 45, wherein the sliding member defines a through hole, and when the spring element is in a first state, the sliding member is in the first position, and the through hole communicates with the fifth channel; and
when the spring element is in a second state, the sliding member is in the second position, and the sliding member blocks the fifth channel.

49. The transcatheter treatment system of claim 45, wherein an outer wall of the main body has a balance opening communicated to a proximal end of the sixth channel.

50. The transcatheter treatment system of claim 42, wherein the main body comprises a proximal section, an outer diameter of the proximal section gradually increases from a proximal end to a distal end, and a radial dimension of a part of the fourth channel corresponding to the proximal section is a constant value or gradually decreases.

51. The transcatheter treatment system of claim 50, wherein the main body further comprises a middle section extending from the proximal section to the distal section, the middle section defines the fifth channel, and a radial dimension of a part of the middle section adjacent to the proximal section is greater than a radial dimension of a part of the middle section adjacent to the distal section.

52. The transcatheter treatment system of claim 51, wherein a radial dimension of the distal section is a constant value, or a radial dimension of a part of the distal section adjacent to the middle section is greater than a radial dimension of a part of the distal section away from the middle section.

53. The transcatheter treatment system of claim 42, wherein at least one electrode is provided on a side wall of the main body, or the main body is made of metal.

54. The transcatheter treatment system of claim 42, wherein an operating handle is connected to proximal ends of the inner sheath and the injection assembly, and the operating handle comprises a housing and a driving assembly; wherein the driving assembly comprises a driving knob that is rotatably connected to the housing and a driving rod that is movably inserted into the housing and threaded to the driving knob; wherein
the proximal end of the inner sheath is fixedly connected to the housing;
the proximal end of the injection assembly is fixedly connected to the driving rod; and
the driving rod is configured to move axially by rotation of the driving knob relative to the housing so as to drive the injection assembly to move and thus drive the needle body to penetrate into the target tissue region.

55. The transcatheter treatment system of claim 54, wherein a damping structure is provided between the driving knob and the housing.

56. The transcatheter treatment system of claim 55, wherein the damping structure comprises a damping ring and a rib in an interference fit with the damping ring, and one of the damping ring and the rib is provided on the housing, and the other is provided on the driving knob.

57. The transcatheter treatment system of claim 56, wherein a part of the driving knob extends into an interior of the housing and is provided with a circumferential groove, and the damping ring is at least partially received in the circumferential groove; and the rib protrudes inward on an inner wall of the housing, and the rib press the damping ring.

58. The transcatheter treatment system of claim 54, wherein the operating handle further comprises an injection interface seat located outside the housing, a proximal end of the driving rod is fixedly connected to the injection interface seat, and a proximal end of the injection assembly is fixedly connected to the injection interface seat.

59. The transcatheter treatment system of claim 54, wherein the driving rod defines a connection through-hole extending in an axial direction, a first protection tube is arranged in the connection through-hole and extends out of the connection through-hole, and the injection assembly is inserted into the first protection tube and the connection through-hole.

60. The transcatheter treatment system of claim 54, wherein the operating handle further comprises a hemostatic valve assembly fixedly installed in the housing, and a distal end of the hemostatic valve assembly is fixedly connected to the proximal end of the inner sheath, wherein the hemostatic valve assembly comprises a sealing gasket, a second protection tube passes through the sealing gasket, the second protection tube is connected to the sealing gasket in a tight seal, and the injection assembly passes through the second protection tube.

61. The transcatheter treatment system of claim 54, further comprising a feedback mechanism, the feedback mechanism comprising:
a middle piece fixedly connected to the housing of the operating handle, an outer wall of the middle piece being provided with a first abutment portion;
a sleeving tube movably arranged around the middle piece, an inner wall of the sleeving tube is provided with a second abutment portion, wherein in an initial state, a proximal end of the sleeving tube contacts a distal end of the housing, and the second abutment portion is located on a proximal side of the first abutment portion; and
a spring element, one end of the spring element abuts the first abutment portion, and an other end of the spring element abuts the second abutment portion;
wherein when the distal end of the inner sheath abuts the target tissue region, the middle piece is forced to press the spring element and the middle piece moves proximally relative to the sleeving tube, generating a gap between the proximal end of the sleeving tube and the distal end of the housing.

62. The transcatheter treatment system of claim 54, wherein the guide tube assembly comprises a first tube and a second tube slidably inserted in the first tube, the first curved section is provided by the first tube, and the second curved section is provided by the second tube; wherein
the first tube is a bendable guiding sheath, and a proximal end of the guiding sheath is connected with a guide handle for controlling the guiding sheath; and
the second tube is a bendable sheath, and a proximal end of the bendable sheath is connected with a bending handle for controlling the bendable sheath;
wherein the transcatheter treatment system further comprises an adjusting device, the adjusting device comprises:
a connecting piece, fixedly connected to a proximal end of the bending handle;
a guide tube, inserted into the connecting piece and rotatably connected to the connecting piece, the inner sheath being movably inserted into the guide tube;
a middle piece, movably arranged around the guide tube and fixedly connected to the housing; and
an adjusting piece, movably provided on the middle piece and configured to directly or indirectly lock or unlock the guide tube in a radial direction of the middle piece to prevent or allow axial movement of the middle piece relative to the guide tube, thereby preventing or allowing axial movement of the operating handle relative to the bending handle.

63. A transcatheter treatment system, having a distal end and an opposing proximal end and comprising:
a guide tube assembly, comprising a first tube and a second tube slidably inserted in the first tube, the first tube having a first, bendable curved section, and the second tube having a second, bendable curved section;
a guide handle, a proximal end of the first tube being connected to the guide handle;
a bending handle, a proximal end of the second tube being connected to the bending handle;
an inner sheath, movably inserted into the guide tube assembly;
a treatment assembly, movably installed in the inner sheath and being extendable out of a distal end of the inner sheath; and
an operating handle, proximal ends of the inner sheath and the treatment assembly being both connected to the operating handle;
wherein the guide handle, the bending handle and the operating handle are arranged in sequence from a distal end to a proximal end, and distances therebetween are adjustable.

64. The transcatheter treatment system of claim 63, wherein the guide handle, the bending handle and the operating handle have a synchronous motion state after at least two of them are locked with each other, and an independent motion state after unlocking.

65. The transcatheter treatment system of claim 64, wherein the transcatheter treatment system further comprises an auxiliary support comprising a guide rod and a plurality of clamping members that are movable along the guide rod and positionable on the guide rod, and wherein at least one of the guide handle, the bending handle and the operating handle is installed on a corresponding clamping member, and a position of the at least one of the guide handle, the bending handle and the operating handle is adjusted by movement of the corresponding clamping member.

66. The transcatheter treatment system of claim 65, wherein the transcatheter treatment system is further provided with a locking mechanism for maintaining the synchronous motion state after locking or for unlocking and switching to the independent motion state; and
the locking mechanism acts between the clamping member and the guide rod, or between two adjacent ones of the guide handle, the bending handle and the operating handle.

67. The transcatheter treatment system of claim 65, wherein in the transcatheter treatment system, a driving wheel is configured for the operating handle, and the driving wheel is configured to drive the operating handle through a transmission mechanism to change a distance from the bending handle.

68. A transcatheter treatment method, comprising:
providing the transcatheter treatment system of any one of claims 37 to 67;
delivering a distal part of the transcatheter treatment system into a left ventricle via an aortic arch; and
determining a current treatment location on a endocardium of the left ventricle and performing operations.
